# EUROPEAN PATENT APPLICATION

(11) **EP 2 366 389 A1**
(43) Date of publication of application: **21.09.2011**
(21) Application number: 11000034.6
(22) Date of filing: 07.04.2003
(51) Int. Cl.: A61K 31/215, A61K 31/4196

(54) **Severe sepsis preventive therapeutic agent**

(30) Priority: 08.04.2002 JP 2002105204
(62) Divisional of application: 03745702.5
(71) Applicant: Takeda Pharmaceutical Company Limited, Chuo-ku Osaka shi Osaka 541-0005 (JP)
(72) Inventor: Il, Masayuki, Minoo-shi, Osaka 562-0005 (JP); Izawa, Yuji, Muko-shi, Kyoto 617-0002 (JP); Kitazaki, Tomoyuki, Kita-ku, Kobe-shi, Hyogo 651-1221 (JP); Kubo, Kazuki, Amagasaki-shi, Hyogo 661-0043 (JP)
(74) Representative: König, Gregor Sebastian

(57) **Abstract**

The present invention provides an agent for the prophylaxis or treatment of severe sepsis, which contains a compound represented by the formula (I): 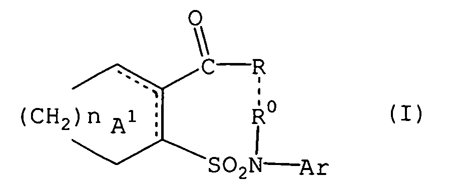 or, the formula (II): 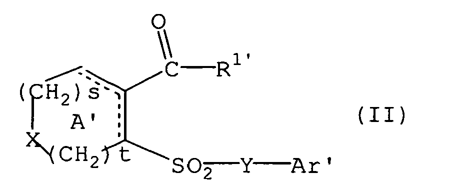 , or a salt thereof or a prodrug thereof, a TLR signal inhibitor containing a non-peptide compound and an agent for the prophylaxis or treatment of organ dysfunction and the like, which contains a TLR signal inhibitory substance.

## Description

### Technical Field

The present invention relates to a novel use of a cycloalkene derivative, which has an inducible nitric oxide (NO) synthetase-derived nitric oxide production-inhibiting effect and/or an inhibitory effect on the production of inflammatory cytokines such as TNF-α, IL-1, IL-6 and the like as an agent for the prophylaxis or treatment of sepsis, particularly severe sepsis. The present invention also relates to a TLR signal inhibitory action of a non-peptide compound including the cycloalkene derivatives and a novel use of the compound as an agent for the prophylaxis or treatment of various diseases based on the action thereof.

### Background Art

Nitric oxide (NO) is known to have various important in vivo physiological activities in mammals. NO is produced principally from L-arginine by NO synthetase (NOS) and currently is known to exist as three genetic isoforms, namely, neuronal NOS, endothelial NOS and inducible NOS (iNOS) [Cell, Vol.70, p. 705-707 (1992)].

Of these, iNOS is induced in macrophages and neutrophile by various cytokines and bacterial lipopolysaccharides (LPS) to continuously produce a large amount of NO. Therefore, iNOS is considered to have not only the pharmacological effects described above but also cell- and tissue-damaging effects at the site of the production [Immunol. Today, Vol.13, p.157-160 (1992)]. NO produced in cells and tissues expressing iNOS is known to be involved in various diseases and pathologies. Accordingly, a substance that inhibits the NO production by iNOS inducible cells is considered to be effective as an agent for the prophylaxis or treatment of various diseases.

On the other hand, cytokines such as TNF-α, IL-1 and IL-6 are secreted from various cells such as monocyte, macrophage, lymphocyte, neutrophile, fibroblast and vascular endothelial cells, and involved widely in inflammation-related biological defense and immune mechanisms [The Cytokine Handbook, 2nd ed., Academic Press Limited (1994), Advances Immunol., Vol.62, p.257-304 (1996)], and thus are referred to as inflammatory cytokines. However, these cytokines, once produced excessively or produced in a wrong site or at a wrong time, exhibit undesirable biological effects, and are proven to be involved in various diseases such as cachexia due to protozoa, bacteria, fungi, viruses and cancers, allergic diseases, chronic rheumatoid arthritis, abscess, graft rejection, anemia, arteriosclerosis, autoimmune disease, diabetes, central nervous system diseases, inflammatory bowel diseases, cardiac failure, hepatitis, hepatocirrhosis, nephritis, osteoporosis, psoriasis, septic shock and the like. In addition, substances which have inhibitory effects or antagonistic effects on the production of these cytokines were reported to be expected to serve as the therapeutic agents for the diseases listed above [Eur. J. Immunol., Vol. 18, p.951-956 (1991), Immunol., Vol.83, p.262-267 (1994), Proc. Natl. Acad. Sci., Vol.93, p.3967-3971 (1997), J. Immunol., Vol. 147, p.1530-1536 (1991), Immunol. Today, Vol.12, p.404-410 (1991)].

WO 99/46242 describes that (i) a compound represented by the formula: 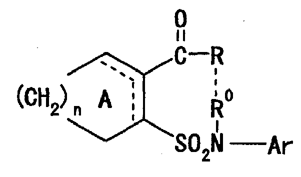 wherein R represents an aliphatic hydrocarbon group optionally having substituents, an aromatic hydrocarbon group optionally having substituents, a heterocyclic group optionally having substituents, a group represented by the formula: -OR¹ (wherein R¹ represents a hydrogen atom or an aliphatic hydrocarbon group optionally having substituents) or a group represented by the formula: 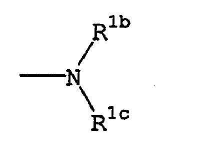 (wherein R^{1b} represents a hydrogen atom or an aliphatic hydrocarbon group optionally having substituents, and R^{1c} is, the same as or different from R^{1b}, a hydrogen atom or an aliphatic hydrocarbon group optionally having substituents), R⁰ represents a hydrogen atom or an aliphatic hydrocarbon group, or R¹ and R⁰ represent a bond with each other, ring A is a cycloalkene substituted by 1 to 4 substituents selected from (1) an aliphatic hydrocarbon group optionally having substituents, (2) an aromatic hydrocarbon group optionally having substituents, (3) a group represented by the formula: -OR¹¹ (wherein R¹¹ represents a hydrogen atom or an aliphatic hydrocarbon group optionally having substituents) and (4) a halogen atom,
Ar represents an aromatic hydrocarbon group optionally having substituents,
a group represented by the formula: 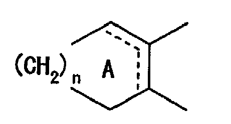 represents a group represented by the formula: 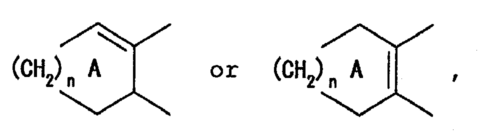 and n represents an integer of 1 to 4, and
(ii) a compound represented by the formula: 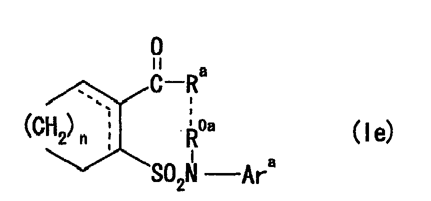 wherein R^{a} represents an aliphatic hydrocarbon group optionally having substituents, an aromatic hydrocarbon group optionally having substituents, a heterocyclic group optionally having substituents, a group represented by the formula: -OR^{1a} (wherein R^{1a} represents a hydrogen atom or an aliphatic hydrocarbon group optionally having substituents) or a group represented by the formula: 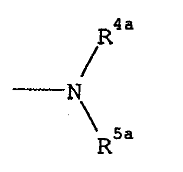 (wherein R^{4a} and R^{5a} are the same or different and each represents a hydrogen atom or an aliphatic hydrocarbon group optionally having substituents),
R^{0a} represents a hydrogen atom or an aliphatic hydrocarbon group, or R^{a} and R^{0a} represent a bond with each other,
Ar^{a} represents an aromatic hydrocarbon group optionally having substituents,
a group represented by the formula: 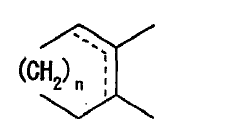 represents a group represented by the formula: 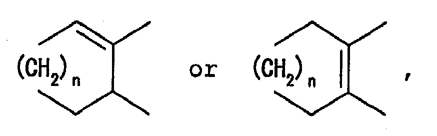 and n represents an integer of 1 to 4, a salt thereof and a prodrug thereof; and
WO01/10826 describes that a compound represented by the formula: 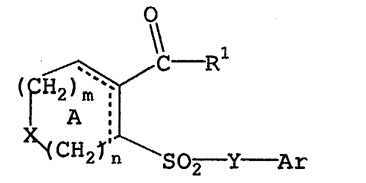 wherein R¹ represents an aliphatic hydrocarbon group optionally having substituents, an aromatic hydrocarbon group optionally having substituents, a heterocyclic group optionally having substituents, a group represented by the formula: -OR^{1a} (wherein R^{1a} represents a hydrogen atom or an aliphatic hydrocarbon group optionally having substituents) or a group represented by the formula: 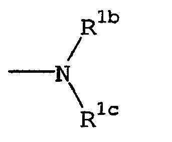 wherein R^{1b} and R^{1c} are the same or different and each represents a hydrogen atom or an aliphatic hydrocarbon group optionally having substituents,
X represents a methylene group, NH, a sulfur atom or an oxygen atom,
Y represents a methylene group optionally having substituents or NH optionally having substituents,
ring A represents a 5 to 8-membered ring optionally having 1 to 4 substituents selected from the group consisting of (1) an aliphatic hydrocarbon group optionally having substituents, (2) an aromatic hydrocarbon group optionally having substituents, (3) a group represented by the formula: -OR² (wherein R² represents a hydrogen atom or an aliphatic hydrocarbon group optionally having substituents) and (4) a halogen atom,
Ar represents an aromatic hydrocarbon group optionally having substituents, a group represented by the formula: 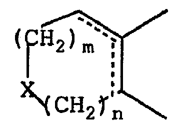 represents a group represented by the formula: 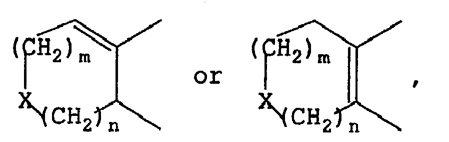 m represents an integer of 0 to 2,
n represents an integer of 1 to 3, and
the total of m and n is not more than 4; provided that when X is a methylene group, Y represents a methylene group optionally having substituents, a salt thereof and a prodrug thereof have a nitric oxide (NO) production-inhibiting effect and an inhibitory effect on the production of inflammatory cytokines, such as TNF-α, IL-1, IL-6 and the like, and are useful as an agent for the prophylaxis or treatment of diseases including cardiac diseases, autoimmune diseases, inflammatory diseases, central nervous system diseases, infectious diseases, sepsis, septic shock and the like. However, it has not been suggested if these compounds show a treatment effect on sepsis after a considerable time from the onset, particularly, after the onset of severe sepsis associated with organ failure, hypoperfusion, hypotension and the like. Furthermore, a detail of the action mechanism of these compounds has not been clarified.

In recent years, accumulation of information relating to inflammatory mediators, such as NO and cytokine, has been ongoing and the role of complicated networks in the biological phenomena has been elucidated. It has been clarified that various mediators are playing key roles in the response to invasion as well. It has come to be known that inflammatory mediators are also involved in the occurrence of organ failure associated with septic shock and sepsis, which has been conventionally considered to be caused by microorganisms and toxins thereof, and the understanding of sepsis, septic shock and organ failure has been dramatically changing. Sepsis is defined to be a systemic inflammatory response syndrome caused by infectious diseases (Chest, Vol. 101, pages 1644-1655 (1992)), and the essential disease state is considered to be caused by excessive production of inflammatory mediators such as NO and cytokine. In fact, there are many reports on the effectiveness of an antiinflammatory mediator therapy on individual inflammatory mediators in initial stages in animal models. In connection with substances that suppress the above-mentioned inflammatory mediators, moreover, many clinical tests targeting sepsis (severe sepsis) patients associated with organ failure, hypoperfusion, hypotension and the like have been ongoing.

As mentioned above, while the effectiveness of a substance that suppresses an inflammatory mediator on sepsis has been shown at an animal model level, clinical tests of an antiinflammatory mediator therapy for severe sepsis patients in US and Europe have not shown any expected effects so far (British Medical Bulletin, Vol. 55, pages 212-225 (1999)). One of the reasons therefor is considered to be the fact that efficacy evaluation in an animal model was performed by administration before the onset of sepsis but, in clinical tests, a drug was administered after the onset of sepsis, particularly, sepsis associated with organ failure, hypoperfusion, hypotension and the like (severe sepsis). It has been also considered difficult for a drug that suppresses each one of complexly intertwined inflammatory mediators to show high effectiveness.

In the meantime, living organisms have a natural immune system as a defensive mechanism against invasion of microorganism infection and the like. The natural immune system distinguishes the molecule structure (pathogen-associated molecular patterns, PAMPs) unique to a pathogenic microorganism, and induces biological defense responses (Nature Reviews Immunology, Vol. 1, pages 135-145 (2001)). The molecule that recognizes PAMPs is called a pattern recognition receptor, which is distributed on the cell surface and blood flow of a host, and as a result of activation of the natural immunity, secretion of inflammatory cytokine from immunocompetent cells, activation of complement system, phagocytosis and the like occur to induce infection protective responses. In recent years, Toll-like receptor (TLR) family has been found as a membrane protein receptor that plays an important role in the recognition of PAMPs and is drawing attention. Until today, 10 kinds have been registered in the database and named as TLR1 - TLR10. Each TLR distinguishes PAMPs represented by a cell wall component of pathogenic microorganisms and induces immune reactions of a host. For example, it is considered that TLR4 transmits signals of lipopolysaccharide (LPS), which is a Gram-negative bacterial cell wall-constituting component, (Nature Immunology, Vol. 2, pages 675-680 (2001)) and TLR2 transmits signals of peptide glycan, which is a bacterial cell wall-constituting component, zymosan from a yeast and the like, into a host cell from the outside of the cell, and TLR9 is essential for the recognition of bacterial DNA.

However, while PAMPs that each TLR recognizes have been identified, a real role of TLR in various diseases and disease states has not been elucidated. This is because identification of PAMPs that TLR recognizes all depended on the use of gene-deleted mice, and the effect in various disease models using selective inhibitors of TLR signals has not been investigated yet.

### Disclosure of the Invention

Thus, an object of the present invention is to provide a pharmaceutical agent effective not only for the prophylaxis of sepsis and the treatment of mild sepsis, but also for the prophylaxis or treatment of severe sepsis. In addition, another object of the present invention is to find a TLR selective inhibitor and provide a drug for the prophylaxis or treatment of various diseases such as organ dysfunction.

In view of the above-mentioned situation, the present inventors have proceeded with the research and study of a drug for the prophylaxis or treatment of severe sepsis, which suppresses production of NO and/or cytokine, and which is effective even by the administration after the onset of sepsis, and intensively studied. As a result, they have found that the above-mentioned cycloalkene compound is also effective for the prophylaxis or treatment of severe sepsis. The present inventors have also found that non-peptide compounds including these cycloalkene compounds suppress production of NO and/or cytokine by inhibiting TLR signals, and therefore, are effective for the prophylaxis or treatment of various diseases caused by changes in TLR signals, particularly organ dysfunction and the like. Further studies made by the present inventors based on said finding resulted in the completion of the present invention.

Accordingly, the present invention relates to
[1] an agent for the prophylaxis or treatment of severe sepsis, which comprises a cycloalkene compound as an active ingredient,
[2] an agent for the prophylaxis or treatment of severe sepsis, which comprises a compound represented by the formula (I): 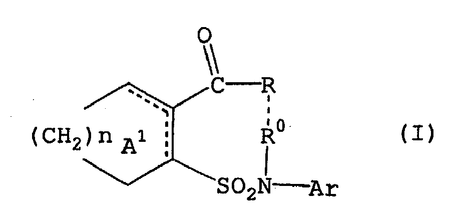 wherein
   R represents an aliphatic hydrocarbon group optionally
   having substituents, an aromatic hydrocarbon group optionally having substituents, a heterocyclic group optionally having substituents, a group represented by the formula: -OR¹ wherein R¹ represents a hydrogen atom or an aliphatic hydrocarbon group optionally having substituents, or a group represented by the formula: 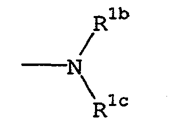 wherein
   R^{1b} and R^{1c}
   are the same or different and each represents a hydrogen atom or an aliphatic hydrocarbon group optionally having substituents,
   R⁰ represents a hydrogen atom or an aliphatic hydrocarbon
   group, or R and R⁰ in combination form a bond,
   ring A¹ represents a cycloalkene optionally substituted by 1 to 4 substituents selected from the group consisting of
   (1) an aliphatic hydrocarbon group optionally having substituents,
   (2) an aromatic hydrocarbon group optionally having substituents,
   (3) a group represented by the formula: -OR¹¹ wherein R¹¹ represents a hydrogen atom or an aliphatic hydrocarbon group optionally having substituents and (4) a halogen atom,
   Ar represents an aromatic hydrocarbon group optionally
   having substituents,
   a group represented by the formula: 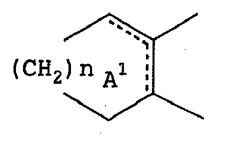 represents a group represented by the formula: 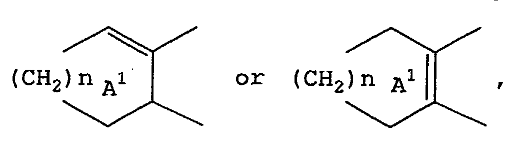 and
   n represents an integer of 1 to 4,
   or a salt thereof or a prodrug thereof, or a compound represented by the formula (II): 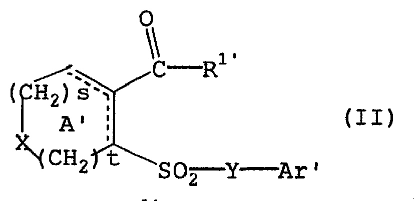 wherein R^{1'} represents an aliphatic hydrocarbon group optionally having substituents, an aromatic hydrocarbon group optionally having substituents, a heterocyclic group optionally having substituents, a group represented by the formula: -OR^{1a'} wherein R^{1a'} represents a hydrogen atom or an aliphatic hydrocarbon group optionally having substituents, or a group represented by the formula: 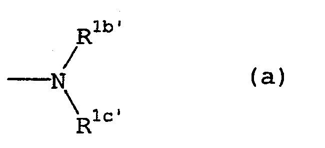 wherein R^{1b'} and R^{1c'} are the same or different and each represents a hydrogen atom or an aliphatic hydrocarbon group optionally having substituents,
   X represents a methylene group, NH, a sulfur atom or an oxygen atom,
   Y represents a methylene group optionally having substituents or NH optionally having substituents,
   ring A' represents a 5- to 8-membered ring optionally having 1 to 4 substituents selected from the group consisting of (1) an aliphatic hydrocarbon group optionally having substituents, (2) an aromatic hydrocarbon group optionally having substituents, (3) a group represented by the formula: -OR^{2'} wherein R^{2'} represents a hydrogen atom or an aliphatic hydrocarbon group optionally having substituents and (4) a halogen atom, Ar' represents an aromatic hydrocarbon group optionally having substituents,
   a group represented by the formula: 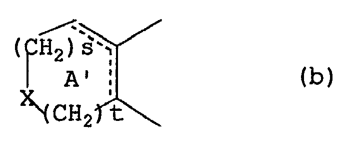 represents a group represented by the formula: 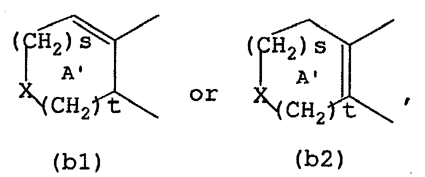 s represents an integer of 0 to 2,
   t represents an integer of 1 to 3, and
   the total of s and t is not more than 4;
   provided that when X is a methylene group, Y represents a methylene group optionally having substituents, or a salt thereof or a prodrug thereof,
[3] the agent of the above-mentioned [2], wherein the formula (I) is the formula (Ia): 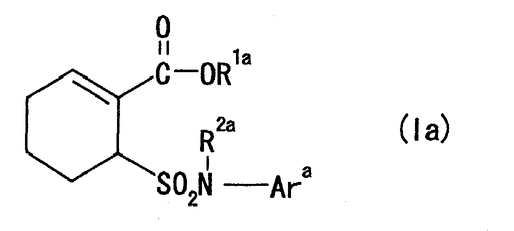 wherein R^{1a} represents a C₁₋₆ alkyl, R^{2a} represents a hydrogen atom or a C₁₋₆ alkyl and Ar^{a} represents a phenyl group substituted by 1 or 2 halogen atoms, and the formula (II) is the formula (IIa): 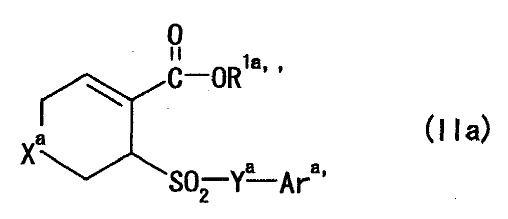 wherein R^{1a"} represents a C₁₋₆ alkyl, X^{a} represents a methylene group or an oxygen atom, Y^{a} represents a methylene group or - NH- and Ar^{a'} represents a phenyl group optionally having 1 or 2 substituents selected from a halogen atom and a C₁₋₆ alkoxy group.
[4] the agent of the above-mentioned [2], further comprising at least one kind of drug selected from the group consisting of antibacterial agent, antifungal agent, non-steroidal antiinflammatory drug, steroid and anticoagulant,
[5] a method for the prophylaxis or treatment of severe sepsis, which comprises administration of an effective amount of a compound represented by the formula (I) or the formula (II) or a salt thereof or a prodrug thereof described in the above-mentioned [2] to a mammal,
[6] use of a compound represented by the formula (I) or the formula (II) or a salt thereof or a prodrug thereof described in the above-mentioned [2] for the production of an agent for the prophylaxis or treatment of severe sepsis,
[7] a TLR signal inhibitor comprising a non-peptide compound as an active ingredient,
[8] the agent of the above-mentioned [7], wherein the non-peptide compound is a non-peptide compound having a molecular weight of not more than about 1000,
[9] the agent of the above-mentioned [7], wherein the non-peptide compound is a compound represented by the formula (I): 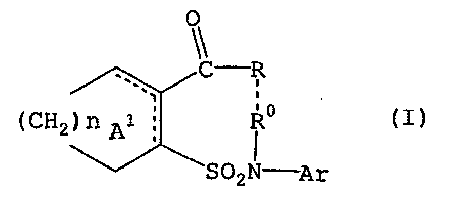 wherein R represents an aliphatic hydrocarbon group optionally having substituents, an aromatic hydrocarbon group optionally having substituents, a heterocyclic group optionally having substituents, a group represented by the formula: -OR¹ wherein
   R¹ represents a hydrogen atom or an aliphatic hydrocarbon group optionally having substituents, or a group represented by the formula: 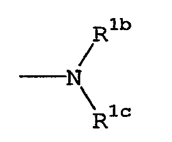 wherein R^{1b} and R^{1c} are the same or different and each represents a hydrogen atom or an aliphatic hydrocarbon group optionally having substituents, R⁰ represents a hydrogen atom or an aliphatic hydrocarbon group, or R and R⁰ in combination form a bond, ring A¹ represents a cycloalkene optionally substituted by 1 to 4 substituents selected from the group consisting of (1) an aliphatic hydrocarbon group optionally having substituents, (2) an aromatic hydrocarbon group optionally having substituents, (3) a group represented by the formula: -OR¹¹ wherein R¹¹ represents a hydrogen atom or an aliphatic hydrocarbon group optionally having substituents and (4) a halogen atom,
   Ar represents an aromatic hydrocarbon group optionally having substituents,
   a group represented by the formula: 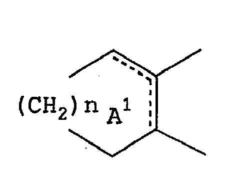 represents a group represented by the formula: 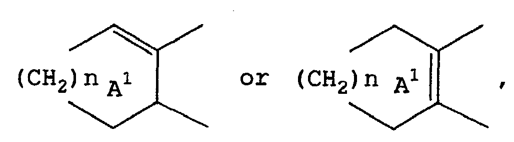 and
   n represents an integer of 1 to 4, or a salt thereof or a prodrug thereof, or, a compound represented by the formula (II): 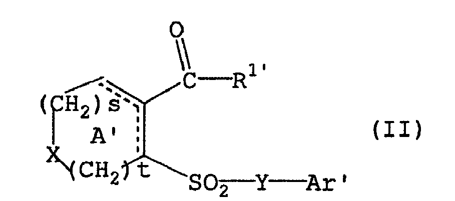 wherein R^{1'} represents an aliphatic hydrocarbon group optionally having substituents, an aromatic hydrocarbon group optionally having substituents, a heterocyclic group optionally having substituents, a group represented by the formula: -OR^{1a'} wherein R^{1a'} represents a hydrogen atom or an aliphatic hydrocarbon group optionally having substituents, or a group represented by the formula: 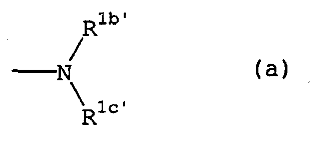 wherein R^{1b'} and R^{1c'} are the same or different and each represents a hydrogen atom or an aliphatic hydrocarbon group optionally having substituents,
   X represents a methylene group, NH, sulfur atom or oxygen atom,
   Y represents a methylene group optionally having substituents or NH optionally having substituents,
   ring A' represents a 5 to 8-membered ring optionally having 1 to 4 substituents selected from the group consisting of (1) an
   aliphatic hydrocarbon group optionally having substituents, (2) an aromatic hydrocarbon group optionally having substituents, (3) a group represented by the formula: -OR^{2'} wherein R^{2'} represents a hydrogen atom or an aliphatic hydrocarbon group optionally having substituents and (4) a halogen atom, Ar' represents an aromatic hydrocarbon group optionally having substituents,
   a group represented by the formula: 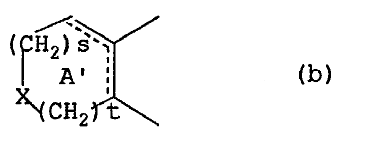 represents a group represented by the formula: 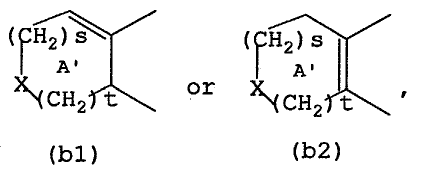 s represents an integer of 0 to 2,
   t represents an integer of 1 to 3,
   the total of s and t is not more than 4;
   provided that when X is a methylene group, Y represents a methylene group optionally having substituents, or a salt thereof or a prodrug thereof,
[10] the agent of the above-mentioned [7], wherein TLR is TLR4,
[11] an agent for the prophylaxis or treatment of a disease caused by a change in a TLR signal, which comprises the agent of the above-mentioned [7],
[12] the agent of the above-mentioned [11], wherein the disease caused by the change in the TLR signal is organ dysfunction,
[13] the agent of the above-mentioned [12], wherein the organ is an organ of central nervous system, circulatory system, respiratory system, bone and joint system, digestive system or renal and urinary system,
[14] a method for the inhibition of TLR signal, which comprises administration of an effective amount of a non-peptide compound to a mammal,
[15] a method for the prophylaxis or treatment of a disease caused by a change in a TLR signal, which comprises administration of an effective amount of a non-peptide compound to a mammal,
[16] use of a non-peptide compound for the production of a TLR signal inhibitor,
[17] use of a non-peptide compound for the production of an agent for the prophylaxis or treatment of a disease caused by a change in a TLR signal,
[18] an agent for the prophylaxis or treatment of organ dysfunction, which comprises a TLR signal inhibitory substance,
[19] the agent of the above-mentioned [18], wherein the organ is an organ of central nervous system, circulatory system, respiratory system, bone and joint system, digestive system or renal and urinary system,
[20] a method for the prophylaxis or treatment of severe sepsis or organ dysfunction, which comprises inhibition of TLR signal.

### Brief Description of the Drawings

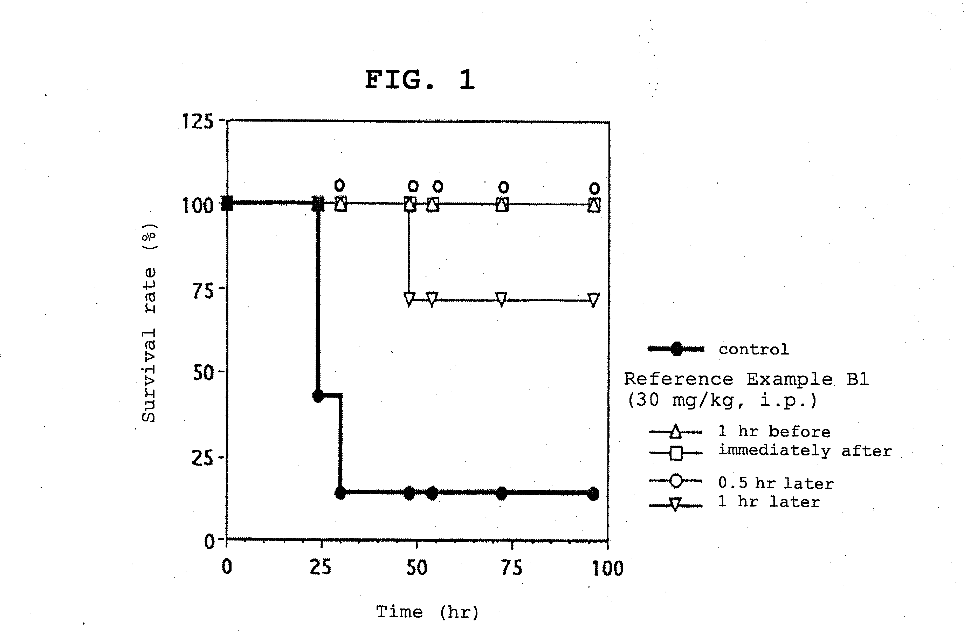

Fig. 1 shows changes in survival rate with the lapse of time of LPS-inoculated mice administered with a test substance (Reference Example B1) at various times, wherein the transverse axis shows the time after LPS inoculation and the vertical axis shows the survival rate (Survival (%)) of the mice, • shows the result of non-administration of a test substance, Δ shows the result of administration of a test substance at 1 hr before LPS inoculation, □ shows the result of administration of a test substance immediately after LPS inoculation, O shows the result of administration of a test substance at 0.5 hr after LPS inoculation, and V shows the result of administration of a test substance at 1 hr after LPS inoculation.

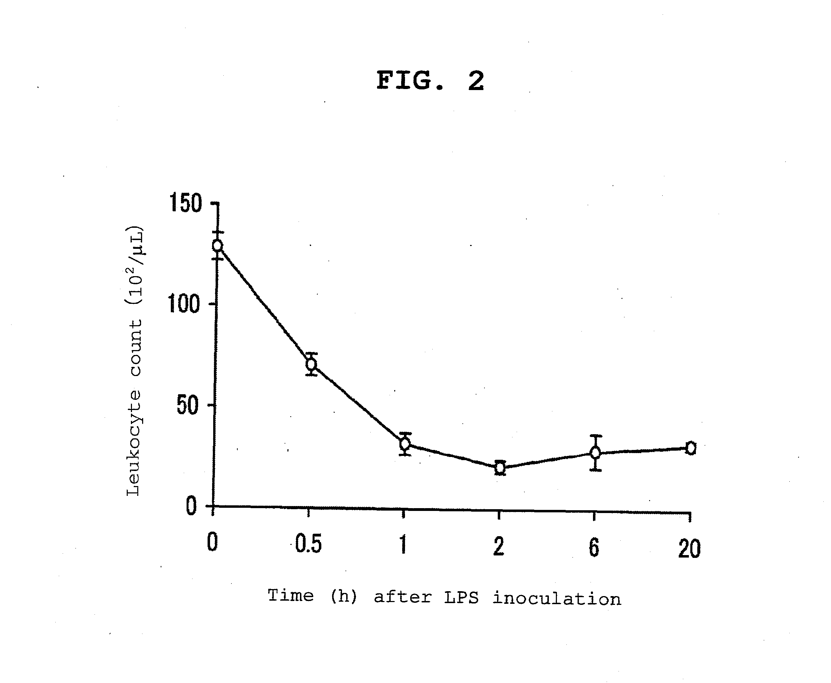

Fig. 2 shows changes in the leukocyte count with the lapse of time in the mice after LPS inoculation, wherein the transverse axis shows the time after LPS inoculation and the vertical axis shows leukocyte count (× 10²/µL).

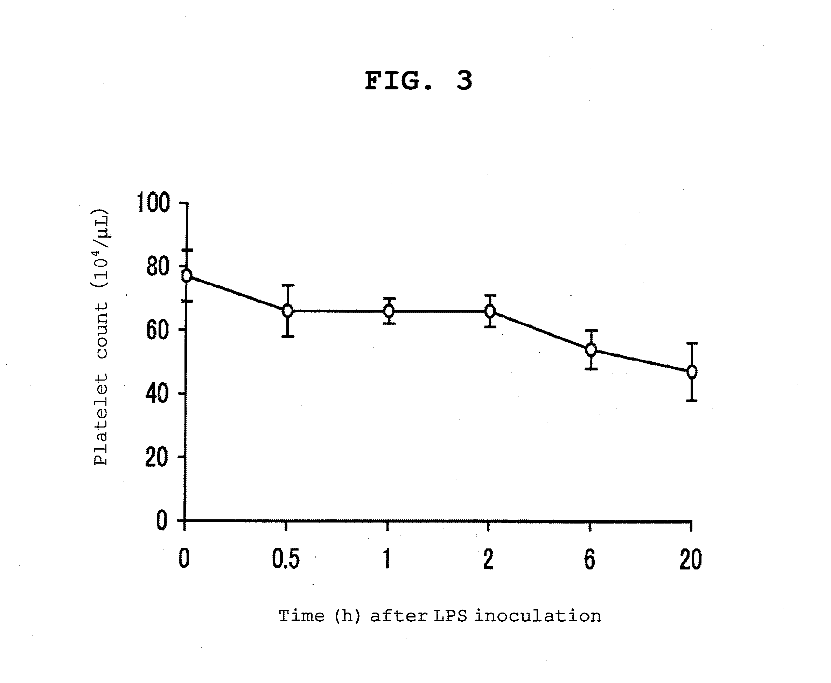

Fig. 3 shows changes in platelet count with the lapse of time in the mice after LPS inoculation, wherein the transverse axis shows the time after LPS inoculation and the vertical axis shows platelet count (× 10⁴/µL).

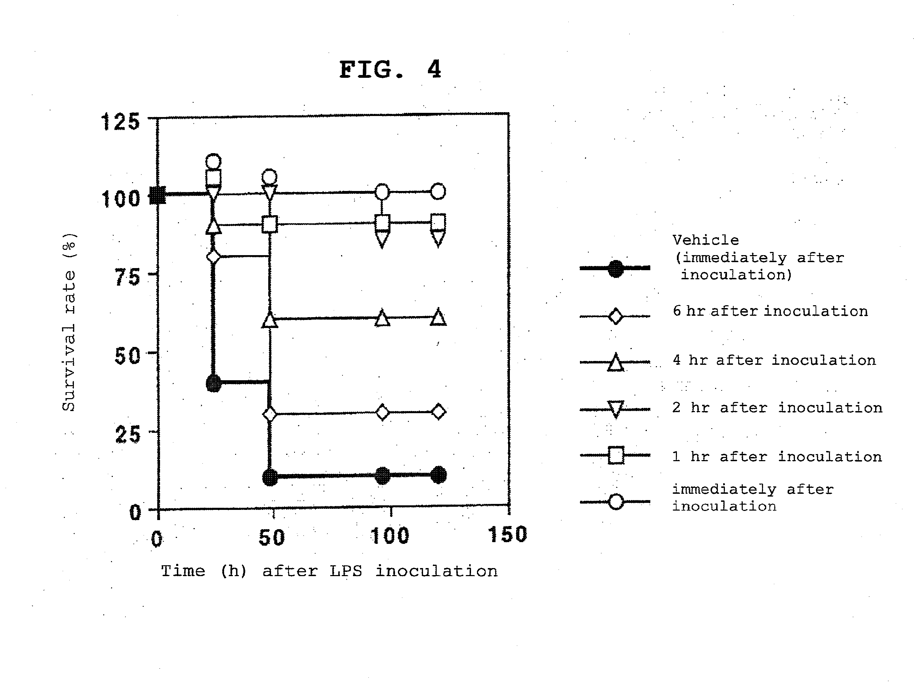

Fig. 4 shows changes in survival rate with the lapse of time of LPS-inoculated mice administered with a test substance (Reference Example B66) at various times, wherein the transverse axis shows the time after LPS inoculation and the vertical axis shows a survival rate (Survival (%)) of the mice, • shows the result of administration of an emulsion without a test substance immediately after LPS inoculation, O shows the result of administration of a test substance immediately after LPS inoculation, □ shows the result of administration of a test substance at 1 hr after LPS inoculation, V shows the result of administration of a test substance at 2 hr after LPS inoculation, Δ shows the result of administration of a test substance at 4 hr after LPS inoculation, and 0 shows the result of administration of a test substance at 6 hr after LPS inoculation.

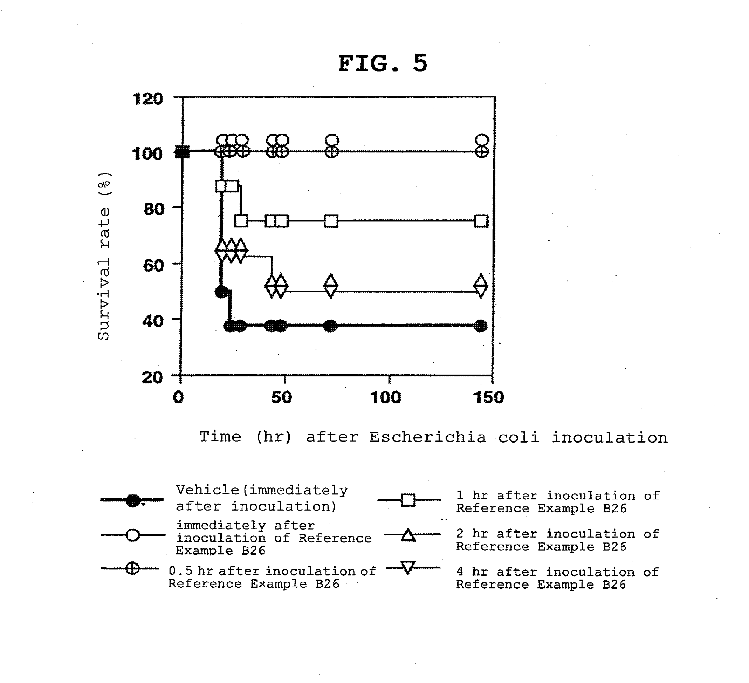

Fig. 5 shows changes in survival rate with the lapse of time of galactosamine-loaded mouse Escherichia coli inoculation model administered with a test substance (Reference Example B26) at various times, wherein the transverse axis shows the time after Escherichia coli inoculation and the vertical axis shows a survival rate (Survival (%)) of the mice, • shows the result of administration of a solvent without a test substance immediately after Escherichia coli inoculation, O shows the result of administration of a test substance immediately after Escherichia coli inoculation, ⊕ shows the result of administration of a test substance at 0.5 hr after Escherichia coli inoculation, □ shows the result of administration of a test substance at 1 hr after Escherichia coli inoculation, Δ shows the result of administration of a test substance at 2 hr after LPS inoculation, and V shows the result of administration of a test substance at 4 hr after LPS inoculation.

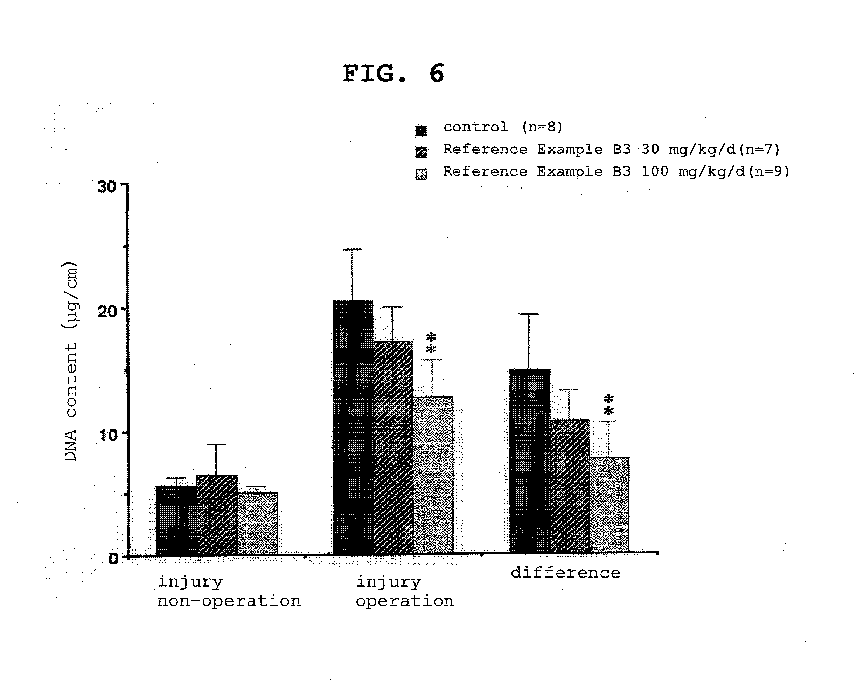

Fig. 6 shows the effect of a test substance (Reference Example B3) on vascular thickening of rat due to balloon injury, wherein the vertical axis shows DNA content (µg/cm), Normal shows rats free of balloon injury operation, Injured shows balloon injury operation rats, Difference shows difference in these two, and columns are for control, the compound of Reference Example B3, 30 mg/kg/d and 100 mg/kg/d, from the left. (**: p<0.01)

### Best Mode for Carrying Out the Invention

Severe sepsis, which is the target disease of an agent for the prophylaxis or treatment of severe sepsis, which comprises a cycloalkene compound, of the present invention, is high in the level of severeness as compared to other systemic inflammatory response syndromes caused by infection, satisfies at least two of the following items of, for example, body temperature: not less than 38°C or less than 36°C, cardiac rate: not less than 90 bpm/min, respiration rate: not less than 20 times/min, and leukocyte count: not less than 12000 leukocytes/mm³ or less than 4000 leukocytes/mm³, shows a disease state of hypotension (systolic blood pressure of not more than 90 mmHg), half consciousness, cryptic speech and action, urine per 1 hr of less than 0.5 mL/kg, platelets of less than 80000 platelets/mm³ and the like, and typically accompanies symptoms such as organ failure, hypoperfusion, hypotension and the like.

The cycloalkene compound (hereinafter to be simply referred to as the cycloalkene compound) to be used for an agent for the prophylaxis or treatment of severe sepsis of the present invention is free of any particular limitation as long as it can show effect for the prophylaxis and/or treatment of severe sepsis. As the cycloalkene compounds, the above-mentioned compounds represented by the formula (I) and the formula (II), salts thereof and prodrugs thereof are preferable.

The above-mentioned compounds are explained in detail in the following.

In the specification, R represents an aliphatic hydrocarbon group optionally having substituents, an aromatic hydrocarbon group optionally having substituents, a heterocyclic group optionally having substituents, a group represented by the formula: -OR¹ (wherein R¹ represents a hydrogen atom or an aliphatic hydrocarbon group optionally having substituents) or a group represented by the formula: 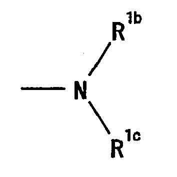 wherein R^{1b} and R^{1c} are the same or different and each represents a hydrogen atom or an aliphatic hydrocarbon group optionally having substituents, or R and R⁰ in combination form a bond, with particular preference given to the group represented by the formula: -OR¹ (wherein R¹ is as defined above).

When R and R⁰ in combination form a bond, the compound represented by the formula (I) can be represented by the formula: 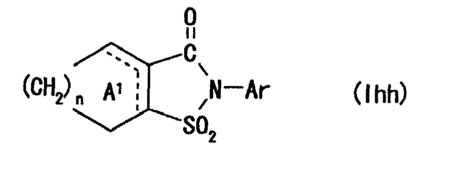 wherein each symbol is as defined above, and specifically can be represented by the formula: 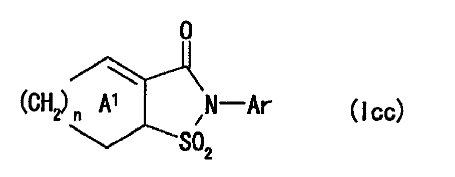 wherein each symbol is as defined above, or 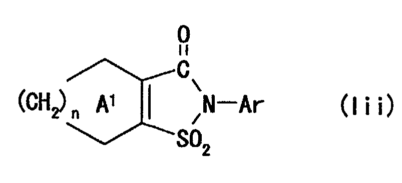 wherein each symbol is as defined above.

When R is a group represented by the formula: -OR¹ (wherein R¹ is as defined above), the compound represented by the formula (I) can be represented by the formula: 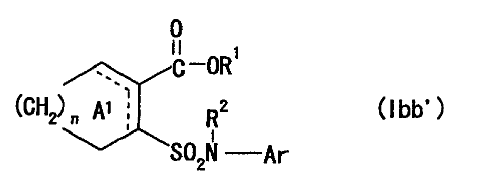 wherein R² is a hydrogen atom or an aliphatic hydrocarbon group, and other symbols are as defined above, and specifically can be represented by the formula: 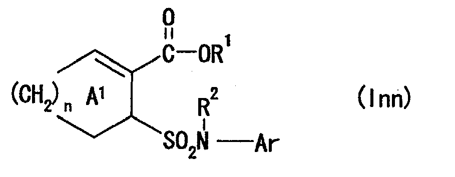 wherein each symbol is as defined above, or the formula: 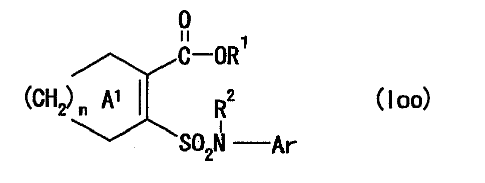 wherein each symbol is as defined above.

As the compound represented by the formula (I), a compound represented by the formula (Icc) or the formula (Inn) is preferable.

As the "aliphatic hydrocarbon group" of the "aliphatic hydrocarbon group optionally having substituents" represented by R, R¹, R¹¹, R^{1b} and R^{1c} and the "aliphatic hydrocarbon group" represented by R⁰ and R², for example, an alkyl group, a cycloalkyl group, a cycloalkylalkyl group, an alkenyl group, an alkynyl group, etc. are preferable.

As the alkyl group, for example, a linear or branched alkyl group having 1 to 20 carbon atoms (e.g., a methyl group, an ethyl group, an n-propyl group, an isopropyl group, an n-butyl group, an isobutyl group, a sec-butyl group, a tert-butyl group, a pentyl group, a hexyl group, a heptyl group, an octyl group, a nonyl group, a decyl group, a dodecyl group, etc.) and the like are preferable, and particularly, for example, a lower alkyl group having 1 to 6 carbon atoms (e.g., a methyl group, an ethyl group, an n-propyl group, an isopropyl group, an n-butyl group, an isobutyl group, a sec-butyl group, a tert-butyl group, etc.) and the like are preferable.

As the cycloalkyl group, for example, a cycloalkyl group having 3 to 10 carbon atoms (e.g., a cyclopropyl group, a cyclobutyl group, a cyclopentyl group, a cyclohexyl group, a cycloheptyl group, a cyclooctyl group, etc.) and the like are preferable, and particularly, for example, a cycloalkyl group having 3 to 6 carbon atoms (e.g., a cyclopropyl group, a cyclobutyl group, a cyclopentyl group, a cyclohexyl group, etc.) and the like are preferable.

As the cycloalkylalkyl group, for example, a cycloalkylalkyl group having 4 to 12 carbon atoms (e.g., a cyclopropylmethyl group, a cyclopentylmethyl group, a cyclohexylmethyl group, a cycloheptylmethyl group, etc.) and the like are preferable, and particularly, for example, a cycloalkylalkyl group having 4 to 8 (particularly 4 to 7) carbon atoms (e.g., a cyclopropylmethyl group, a cyclopentylmethyl group, a cyclohexylmethyl group, etc.) and the like are preferable.

As the alkenyl group, for example, a lower alkenyl group having 3 to 6 carbon atoms (e.g., a propenyl group, a butenyl group, a pentenyl group, etc.) and the like are preferable, and particularly, for example, a lower alkenyl group having 3 or 4 carbon atoms (e.g., a propenyl group, a butenyl group, etc.) and the like are preferable.

As the alkynyl group, for example, a lower alkynyl group having 3 to 6 carbon atoms (e.g., a propynyl group, a butynyl group, a pentynyl group, etc.) and the like are preferable, and particularly, for example, a lower alkynyl group having 3 or 4 carbon atoms (e.g., a propynyl group, a butynyl group, etc.) and the like are preferable.

As the "substituents" of the above-mentioned "aliphatic hydrocarbon group optionally having substituents", for example, a heterocyclic group, an oxo group, a hydroxy group, a C₁₋₆ alkoxy group, a C₃₋₁₀ (particularly C₃₋₆) cycloalkyloxy group, a C₆₋₁₀ aryloxy group, a C₇₋₁₉ (particularly C₇₋₁₂) aralkyloxy group, a heterocyclic-oxy group, a C₁₋₆ alkylthio group (sulfur atom may be oxidized), a C₃₋₁₀ (particularly C₃₋₆) cycloalkylthio group (sulfur atom may be oxidized), a C₆₋₁₀ arylthio group (sulfur atom may be oxidized), a C₇₋₁₉ (particularly C₇₋₁₂) aralkylthio group (sulfur atom may be oxidized), a heterocyclic-thio group, a heterocyclic-sulfinyl group, a heterocyclic-sulfonyl group, a nitro group, a halogen atom, a cyano group, a carboxyl group, a C₁₋₁₀ (particularly C₁₋₆) alkoxy-carbonyl group, a C₃₋₆ cycloalkyloxy-carbonyl group, a C₆₋₁₀ aryloxy-carbonyl group, a C₇₋₁₉ (particularly C₇₋₁₂) aralkyloxy-carbonyl group, a heterocyclic-oxy-carbonyl group, a C₆₋₁₀ aryl-carbonyl group, C₁₋₆ alkanoyl group, C₃₋₅ alkenoyl group, a C₆₋₁₀ aryl-carbonyloxy group, a C₂₋₆ alkanoyloxy group, a C₃₋₅ alkenoyloxy group, a carbamoyl group optionally having substituents, a thiocarbamoyl group optionally having substituents, a carbamoyloxy group optionally having substituents, a C₁₋₆ alkanoylamino group, a C₆₋₁₀ arylcarbonylamino group, a C₁₋₁₀ (particularly C₁₋₆) alkoxy-carboxamido group, a C₆₋₁₀ aryloxy-carboxamido group, a C₇₋₁₉ (particularly C₇₋₁₂) aralkyloxy-carboxamido group, a C₁₋₁₀ (particularly C₁₋₆) alkoxy-carbonyloxy group, a C₆₋₁₀ aryloxy-carbonyloxy group, a C₇₋₁₉ (particularly C₇₋₁₂) aralkyloxy-carbonyloxy group, a C₃₋₁₀ (particularly C₃₋₆) cycloalkyloxy-carbonyloxy group, a ureido group optionally having substituents, a C₆₋₁₀ aryl group optionally having substituents, etc. are used.

These substituents are substituted at substitutable positions in the above-mentioned "aliphatic hydrocarbon group", wherein the substituents are not limited to a single substituent but may be the same or different plural (preferably 2 to 4) substituents.

As the "C₁₋₆ alkoxy group", for example, a methoxy group, an ethoxy group, an n-propoxy group, an isopropoxy group, an n-butoxy group, a tert-butoxy group, an n-pentyloxy group, an n-hexyloxy group, etc. are used, as the "C₃₋₁₀ cycloalkyloxy group", for example, a cyclopropyloxy group, a cyclohexyloxy group, etc. are used, as the "C₆₋₁₀ aryloxy group", for example, a phenoxy group, a naphthyloxy group, etc. are used, as the "C₇₋₁₉ aralkyloxy group", for example, a benzyloxy group, a 1-phenylethyloxy group, a 2-phenylethyloxy group, a benzhydryloxy group, a 1-naphthylmethyloxy group, etc. are used, as the "C₁₋₆ alkylthio group (sulfur atom may be oxidized)", for example, a methylthio group, an ethylthio group, an n-propylthio group, an n-butylthio group, a methylsulfinyl group, a methylsulfonyl group, etc. are used, as the "C₃₋₁₀ cycloalkylthio group (sulfur atom may be oxidized)", for example, a cyclopropylthio group, a cyclohexylthio group, a cyclopentylsulfinyl group, a cyclohexylsulfonyl group, etc. are used, as the "C₆₋₁₀ arylthio group (sulfur atom may be oxidized)", for example, a phenylthio group, a naphthylthio group, a phenylsulfinyl group, a phenylsulfonyl group, etc. are used, as the "C₇₋₁₉ aralkylthio group (sulfur atom may be oxidized)", for example, a benzylthio group, a phenylethylthio group, a benzhydrylthio group, a benzylsulfinyl group, a benzylsulfonyl group, etc. are used, as the "halogen atom", a fluorine atom, a chlorine atom, a bromine atom and an iodine atom are used, as the "C₁₋₁₀ alkoxy-carbonyl group", for example, a methoxycarbonyl group, an ethoxycarbonyl group, an n-propoxycarbonyl group, an isopropoxycarbonyl group, an n-butoxycarbonyl group, an isobutoxycarbonyl group, a tert-butoxycarbonyl group, etc. are used, as the "C₃₋₆ cycloalkyloxy-carbonyl group", for example, a cyclopropyloxycarbonyl group, a cyclopentyloxycarbonyl group, a cyclohexyloxycarbonyl group, etc. are used, as the "C₆₋₁₀ aryloxy-carbonyl group", for example, a phenoxycarbonyl group, a naphthyloxycarbonyl group, etc. are used, as the "C₇₋₁₉ aralkyloxy-carbonyl group", for example, a benzyloxycarbonyl group, a benzhydryloxycarbonyl group, a 2-phenethyloxycarbonyl group, etc. are used, as the "C₆₋₁₀ aryl-carbonyl group", for example, a benzoyl group, a naphthoyl group, etc. are used, as the "C₁₋₆ alkanoyl group", for example, a formyl group, an acetyl group, a propionyl group, a butyryl group, a valeryl group, a pivaloyl group, etc. are used, as the "C₃₋₅ alkenoyl group", for example, an acryloyl group, a crotonoyl group, etc. are used, as the "C₆₋₁₀ aryl-carbonyloxy group", for example, a benzoyloxy group, a naphthoyloxy group, etc. are used, as the "C₂₋₆ alkanoyloxy group", for example, an acetoxy group, a propionyloxy group, a butyryloxy group, a valeryloxy group, a pivaloyloxy group, etc. are used, and as the "C₃₋₅ alkenoyloxy group", for example, an acryloyloxy group, a crotonoyloxy group, etc. are used.

As the "carbamoyl group optionally having substituents", for example, a carbamoyl group or a cyclic-amino (e.g., pyrrolidinyl, piperidinyl, piperazinyl, morpholinyl, etc.) carbonyl group, which may be substituted by 1 or 2 substituents selected from a C₁₋₄ alkyl (e.g., methyl, ethyl, etc.), a phenyl, a C₁₋₇ acyl (e.g., acetyl, propionyl, benzoyl, etc.), a C₁₋₄ alkoxy-phenyl (e.g., methoxyphenyl, etc.), etc., and the like are used, and specifically, for example, a carbamoyl group, an N-methylcarbamoyl group, an N-ethylcarbamoyl group, an N,N-dimethylcarbamoyl group, an N,N-diethylcarbamoyl group, an N-phenylcarbamoyl group, an N-acetylcarbamoyl group, an N-benzoylcarbamoyl group, an N-(p-methoxyphenyl)carbamoyl group, a 1-pyrrolidinylcarbonyl group, a piperidinocarbonyl group, a 1-piperazinylcarbonyl group, a morpholinocarbonyl group, etc. are used. As the "thiocarbamoyl group optionally having substituents", for example, a thiocarbamoyl group which may be substituted by 1 or 2 substituents selected from C₁₋₄ alkyl (e.g., methyl, ethyl, etc.), phenyl, etc. are used, and specifically, for example, a thiocarbamoyl group, an N-methylthiocarbamoyl group, an N-phenylthiocarbamoyl group, etc. are used. As the "carbamoyloxy group optionally having substituents", for example, a carbamoyloxy group which may be substituted by 1 or 2 substituents selected from C₁₋₄ alkyl (e.g., methyl, ethyl, etc.), phenyl, etc. are used, and specifically, for example, a carbamoyloxy group, an N-methylcarbamoyloxy group, an N,N-dimethylcarbamoyloxy group, an N-ethylcarbamoyloxy group, an N-phenylcarbamoyloxy group, etc. are used.

As the "C₁₋₆ alkanoylamino group", for example, an acetamido group, a propionamido group, a butyramido group, a valeramido group, a pivalamido group, etc. are used, as the "C₆₋₁₀ aryl-carbonylamino group", for example, a benzamido group, a naphthamido group, a phthalimido group, etc. are used, as the "C₁₋₁₀ alkoxy-carboxamido group", for example, a methoxycarboxamido (CH₃OCONH-) group, an ethoxycarboxamido group, a tert-butoxycarboxamido group, etc. are used, as the "C₆₋₁₀ aryloxy-carboxamido group", for example, a phenoxycarboxamido (C₆H₅OCONH-) group, etc. are used, as the "C₇₋₁₉ aralkyloxy-carboxamido group", for example, a benzyloxycarboxamido (C₆H₅CH₂OCONH-) group, a benzhydryloxycarboxamido group, etc. are used, as the "C₁₋₁₀ alkoxy-carbonyloxy group", for example, a methoxycarbonyloxy group, an ethoxycarbonyloxy group, an n-propoxycarbonyloxy group, an isopropoxycarbonyloxy group, an n-butoxycarbonyloxy group, a tert-butoxycarbonyloxy group, an n-pentyloxycarbonyloxy group, an n-hexyloxycarbonyloxy group, etc. are used, as the "C₆₋₁₀ aryloxy-carbonyloxy group", for example, a phenoxycarbonyloxy group, a naphthyloxycarbonyloxy group, etc. are used, as the "C₇₋₁₉ aralkyloxy-carbonyloxy group", for example, a benzyloxycarbonyloxy group, a 1-phenylethyloxycarbonyloxy group, a 2-phenylethyloxycarbonyloxy group, a benzhydryloxycarbonyloxy group, etc. are used, and as the "C₃₋₁₀ cycloalkyloxy-carbonyloxy group", for example, a cyclopropyloxycarbonyloxy group, a cyclohexyloxycarbonyloxy group, etc. are used.

As the "ureido group optionally having substituents", for example, a ureido group optionally substituted by 1 to 3 (preferably 1 or 2) substituents selected from a C₁₋₄ alkyl group (e.g., a methyl group, an ethyl group, etc.), a phenyl group, etc. are used, and, for example, a ureido group, a 1-methylureido group, a 3-methylureido group, a 3,3-dimethylureido group, a 1,3-dimethylureido group, a 3-phenylureido group, etc. are used.

When a heterocyclic group, a heterocyclic-oxy group, a heterocyclic-thio group, a heterocyclic-sulfinyl group, a heterocyclic-sulfonyl group or a heterocyclic-oxy-carbonyl group is used as the "substituents" of the "aliphatic hydrocarbon group optionally having substituents", the heterocyclic group represents a group formed by excluding one hydrogen atom that binds to the heterocycle. It represents, for example, a 5- to 8-membered ring (preferably 5- or 6-membered ring) group containing 1 to a few, preferably 1 to 4 hetero atoms such as a nitrogen atom (optionally oxidized), an oxygen atom, a sulfur atom, etc., or its condensed cyclic group. As these heterocyclic groups, for example, a pyrrolyl group, a pyrazolyl group, an imidazolyl group, a 1,2,3-triazolyl group, a 1,2,4-triazolyl group, a tetrazolyl group, a furyl group, a thienyl group, an oxazolyl group, an isoxazolyl group, a 1,2,3-oxadiazolyl group, a 1,2,4-oxadiazolyl group, a 1,2,5-oxadiazolyl group, a 1,3,4-oxadiazolyl group, a thiazolyl group, an isothiazolyl group, a 1,2,3-thiadiazolyl group, a 1,2,4-thiadiazolyl group, a 1,2,5-thiadiazolyl group, a 1,3,4-thiadiazolyl group, a pyridyl group, a pyridazinyl group, a pyrimidinyl group, a pyrazinyl group, an indolyl group, a pyranyl group, a thiopyranyl group, a dioxinyl group, a dioxolyl group, a quinolyl group, a pyrido[2,3-d]pyrimidyl group, a 1,5-, 1,6-, 1,7-, 1,8-, 2,6-or 2,7-naphthyridyl group, a thieno[2,3-d]pyridyl group, a benzopyranyl group, a tetrahydrofuryl group, a tetrahydropyranyl group, a dioxolanyl group, a dioxanyl group, etc. are used.

These heterocyclic groups may be substituted at substitutable positions by 1 to 3 substituents selected from a C₁₋₄ alkyl (e.g., methyl, ethyl, etc.), a hydroxy, an oxo, a C₁₋₄ alkoxy (e.g., methoxy, ethoxy, etc.), and the like.

As the "C₆₋₁₀ aryl group" of the "C₆₋₁₀ aryl group optionally having substituents", for example, a phenyl group, a naphthyl group, etc. are used. The C₆₋₁₀ aryl group may be substituted at a substitutable position by a substituent selected from those exemplified as the "substituent" (except for a C₆₋₁₀ aryl group optionally having substituents) of the "aliphatic hydrocarbon group optionally having substituents" described above. Such substituent is not limited to a single substituent, but the same or different, more than one (preferably 2 to 4) substituents may be used.

In the "aliphatic hydrocarbon group optionally having substituents", the substituent together with the aliphatic hydrocarbon group may form an optionally substituted fused ring group, and as such fused ring group, an indanyl group, a 1,2,3,4-tetrahydronaphthyl group, etc. are used. This fused ring group may be substituted at a substitutable position by a substituent selected from those exemplified as the "substituent" of the "aliphatic hydrocarbon group optionally having substituents" described above. Such substituent is substituted at a substitutable position of the fused ring group, wherein the substituent is not limited to a single substituent, but the same or different, more than one (preferably 2 to 4) substituents may be used.

As preferable examples of the above-mentioned "aliphatic hydrocarbon group optionally having substituents" for R, R¹, R¹¹, R^{1b} and R^{1c}, for example, a lower alkyl group having 1 to 6 carbon atoms (e.g., a methyl group, an ethyl group, an n-propyl group, an isopropyl group, an n-butyl group, an isobutyl group, a tert-butoxycarbonylmethyl group, a hydroxyethyl group etc.) optionally having substituents, etc., are used. Of these, a methyl group, an ethyl group, an n-propyl group, an isopropyl group, an n-butyl group, an isobutyl group, etc. are preferable. For example, a methyl group, an ethyl group, an n-propyl group and the like are more preferable, and particularly, an ethyl group, etc. are preferable.

As the "aromatic hydrocarbon group" of the "aromatic hydrocarbon group optionally having substituents" represented by R, an aromatic hydrocarbon group having 6 to 14 carbon atoms (e.g., a phenyl group, a naphthyl group, an anthryl group, an indenyl group etc.) and the like are preferable, and particularly for example, an aryl group having 6 to 10 carbon atoms (e.g., phenyl, naphthyl groups etc.) and the like are preferable and, of these, a phenyl group and the like are particularly preferable.

As the "substituent" of the "aromatic hydrocarbon group optionally having substituents" represented by R, for example, a halogen atom (fluorine atom, chlorine atom, bromine atom, iodine atom), a lower (C₁₋₄)alkyl group (e.g., a methyl group, an ethyl group, a propyl group, a butyl group etc.), a lower (C₁₋₄)alkoxy group (e.g., a methoxy group, an ethoxy group, a propoxy group, a butoxy group etc.), a lower (C₃₋₄)alkoxy-carbonyl group (e.g., a methoxycarbonyl group, an ethoxycarbonyl group, a propoxycarbonyl group, a butoxycarbonyl group etc.), a carboxyl group, a nitro group, a cyano group, a hydroxyl group, an acylamino group (e.g., an alkanoylamino group having 1 to 4 carbon atoms such as an acetylamino group, a propionylamino group, a butyrylamino group and the like, etc.), a cycloalkyl group having 3 to 6 carbon atoms (e.g., a cyclopropyl group, a cyclopentyl group etc.), an aryl group having 6 to 10 carbon atoms (e.g., a phenyl group, a naphthyl group, an indenyl group etc.), a halogeno-lower (C₁₋₄)alkyl group (e.g., a trifluoromethyl group, a trifluoroethyl group etc.), a halogeno-lower (C₁₋₄) alkoxy group (e.g., a trifluoromethoxy group, a 1,1,2,2-tetrafluoroethoxy group, a 2,2,3,3,3-pentafluoropropoxy group etc.), a lower (C₁₋₄)alkylthio group (e.g., a methylthio group, an ethylthio group, a propylthio group etc.), a lower (C₁₋₄) alkanesulfonyl group (e.g., a methanesulfonyl group, an ethanesulfonyl group, a propanesulfonyl group etc.), a lower (C₁₋₄)alkanoyl group (e.g., a formyl group, an acetyl group, a propionyl group etc.), a 5-membered aromatic heterocyclic group (e.g., a 1,2,3-triazolyl group, a 1,2,4-triazolyl group, a tetrazolyl group, a thiazolyl group, an isothiazolyl group, an oxazolyl group, an isoxazolyl group, a thiadiazolyl group, a thienyl group, a furyl group etc.), a carbamoyl group, a lower (C₁₋₄) alkyl-carbamoyl group (e.g., a methylcarbamoyl group, a dimethylcarbamoyl group, a propylcarbamoyl group etc.), a lower (C₁₋₄) alkoxy-carbonyl-lower (C₁₋₄) alkyl-carbamoyl group (e.g., a butoxycarbonylmethylcarbamoyl group, an ethoxycarbonylmethylcarbamoyl group etc.), a 1,3-diacylguanidino-lower (C₁₋₄) alkyl group (e.g., 1,3-diacetylguanidinomethyl, 1,3-bis-(tert-butoxycarbonyl)guanidinomethyl etc.) and the like are used, and a halogen atom (fluorine, chlorine, bromine, iodine atoms), a lower (C₁₋₄) alkyl group (e.g., a methyl group, an ethyl group, a propyl group, a butyl group etc.) and the like are preferably used, and a fluorine atom, a chlorine atom and a methyl group are more preferably used.

These substituents are substituted at substitutable positions of the aromatic hydrocarbon group, and the number of the substituents is preferably 1 to 5, more preferably 1 to 3, most preferably 1 or 2. When two or more of such substituents are present, they may be the same or different.

The "heterocyclic group" of the "heterocyclic group optionally having substituents" represented by R is, for example, a 5 to 8-membered ring (particularly 5 or 6-membered ring) group containing 1 to several, preferably 1 to 4, hetero atoms such as nitrogen atom (optionally oxidized), oxygen atom, sulfur atom and the like, and a fused ring group thereof. As such heterocyclic group, for example, pyrrolyl group, pyrazolyl group, imidazolyl group, 1,2,3-triazolyl group, 1,2,4-triazolyl group, tetrazolyl group, furyl group, thienyl group, oxazolyl group, isoxazolyl group, 1,2,3-oxadiazolyl group, 1,2,4-oxadiazolyl group, 1,2,5-oxadiazolyl group, 1,3,4-oxadiazolyl group, thiazolyl group, isothiazolyl group, 1,2,3-thiadiazolyl group, 1,2,4-thiadiazolyl group, 1,2,5-thiadiazolyl group, 1,3,4-thiadiazolyl group, pyridyl group, pyridazinyl group, pyrimidinyl group, pyrazinyl group, indolyl group, pyranyl group, thiopyranyl group, dioxinyl group, dioxolyl group, quinolyl group, pyrido[2,3-d]pyrimidyl group, 1,5-, 1,6-, 1,7-, 1,8-, 2,6- or 2,7-naphthyridinyl group, thieno[2,3-d]pyridyl group, benzopyranyl group, tetrahydrofuryl group, tetrahydropyranyl group, dioxolanyl group, dioxanyl group and the like are used.

These heterocyclic groups are optionally substituted by 1 to 3 substituents selected from C₁₋₄ alkyl (e.g., methyl, ethyl etc.), hydroxy, oxo, C₁₋₄ alkoxy (e.g., methoxy, ethoxy etc.) and the like at substitutable positions.

As the "aromatic hydrocarbon group" of the "aromatic hydrocarbon group optionally having substituents" represented by Ar, an aromatic hydrocarbon group having 6 to 14 carbon atoms (e.g., a phenyl group, a naphthyl group, an anthryl group, an indenyl group etc.) and the like are preferable, and particularly for example, an aryl group having 6 to 10 carbon atoms (e.g., phenyl, naphthyl groups etc.) and the like are preferable and, of these, a phenyl group and the like are particularly preferable.

As the "substituent" of the "aromatic hydrocarbon group optionally having substituents" represented by Ar and Ar^{a}, for example, a halogen atom (fluorine, chlorine, bromine, iodine atoms), a lower (C₁₋₄) alkyl group (e.g., a methyl group, an ethyl group, a propyl group, an isopropyl group, a butyl group etc.), a lower (C₁₋₄) alkoxy group (e.g., a methoxy group, an ethoxy group, a propoxy group, a butoxy group etc.), a lower (C₁₋₄) alkoxy-carbonyl group (e.g., a methoxycarbonyl group, an ethoxycarbonyl group, a propoxycarbonyl group, a butoxycarbonyl group etc.), a carboxyl group, a nitro group, a cyano group, a hydroxyl group, an acylamino group (e.g., an alkanoylamino group having 1 to 4 carbon atoms such as an acetylamino group, a propionylamino group, a butyrylamino group and the like, etc.), a cycloalkyl group having 3 to 6 carbon atoms (e.g., a cyclopropyl group, a cyclopentyl group etc.), an aryl group having 6 to 10 carbon atoms (e.g., a phenyl group, a naphthyl group, an indenyl group etc.), a halogeno-lower (C₁₋₄) alkyl group (e.g., a trifluoromethyl group, a trifluoroethyl group etc.), a halogeno-lower (C₁₋₄) alkoxy group (e.g., a trifluoromethoxy group, a 1,1,2,2-tetrafluoroethoxy group, a 2,2,3,3,3-pentafluoropropoxy group etc.), a lower (C₁₋₄) alkylthio group (e.g., a methylthio group, an ethylthio group, a propylthio group etc.), a lower (C₁₋₄) alkanesulfonyl group (e.g., a methanesulfonyl group, an ethanesulfonyl group, a propanesulfonyl group etc.), a lower (C₁₋₄) alkanoyl group (e.g., a formyl group, an acetyl group, a propionyl group etc.), a 5-membered aromatic heterocyclic group (e.g., a 1,2,3-triazolyl group, a 1,2,4-triazolyl group, a tetrazolyl group, a thiazolyl group, an isothiazolyl group, an oxazolyl group, an isoxazolyl group, a thiadiazolyl group, a thienyl group, a furyl group etc.), a carbamoyl group, a lower (C₁₋₄) alkyl-carbamoyl group (e.g., a methylcarbamoyl group, a dimethylcarbamoyl group, a propionylcarbamoyl group etc.), a lower (C₁₋₄) alkoxy-carbonyl-lower (C₃₋₄) alkyl-carbamoyl group (e.g., a butoxycarbonylmethylcarbamoyl group, a tert-butoxycarbonylmethylcarbamoyl group, an ethoxycarbonylmethylcarbamoyl group etc.), a 1,3-diacylguanidino-lower (C₁₋₄) alkyl group (e.g., 1,3-diacetylguanidinomethyl, 1,3-bis-(tert-butoxycarbonyl)guanidinomethyl etc.) and the like are used, and a halogen atom (fluorine, chlorine, bromine, iodine atoms), a lower (C₁₋₄) alkyl group (e.g., a methyl group, an ethyl group, a propyl group, a butyl group etc.) and the like are preferably used, and a fluorine atom, a chlorine atom and a methyl group are more preferably used.

These substituents are substituted at substitutable positions of the aromatic hydrocarbon group, and the number of the substituents is preferably 1 to 5, more preferably 1 to 3, most preferably 1 or 2. When two or more of such substituents are present, they may be the same or different.

Typically, as Ar, for example, a phenyl group, a halogenophenyl group, a lower (C₁₋₄) alkylphenyl group, a lower (C₁₋₄) alkoxyphenyl group, a lower (C₁₋₄) alkoxy-carbonylphenyl group, a carboxylphenyl group, a nitrophenyl group, a cyanophenyl group, a halogeno-lower (C₁₋₄) alkylphenyl group, a halogeno-lower (C₁₋₄) alkoxyphenyl group, a lower (C₁₋₄) alkanoyl phenyl group, a 5-membered aromatic heterocycle-substituted phenyl group, a lower (C₁₋₄) alkoxy-carbonyl-lower (C₁₋₄) alkyl-carbamoylphenyl group, 1,3-diacylguanidino-lower (C₁₋₉) alkylphenyl group, a halogen- and lower (C₁₋₄) alkyl-substituted phenyl group, a halogen- and lower (C₁₋₄) alkoxycarbonyl-substituted phenyl group, a halogen- and cyano-substituted phenyl group, a halogen- and 5-membered aromatic heterocycle-substituted phenyl group, a halogen- and lower (C₁₋₄) alkoxy-carbonyl-lower (C₁₋₄) alkyl-carbamoyl-substituted phenyl group and the like are used.

As Ar, a phenyl group optionally having substituents is preferable. Of these, a halogenophenyl group, a lower (C₁-₄) alkylphenyl group, a halogen- and lower (C₁₋₄) alkoxycarbonyl-substituted phenyl group, a halogen- and lower (Cₗ-₄) alkyl-substituted phenyl group and the like are preferably used.

As Ar, a group represented by the formula: 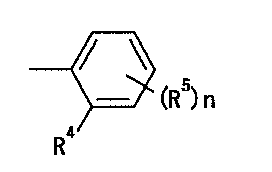 wherein R⁴ and R⁵ are the same or different and each represents a halogen atom or a lower (C₁₋₄) alkyl group, and n is an integer of 0 to 2, is more preferable, in which a group wherein at least one of R⁴ and R⁵ is a halogen atom is still more preferable.

As the halogen atom represented by R⁴ and R⁵, a fluorine atom or a chlorine atom is preferable.

As the halogenophenyl group, for example, a 2,3-difluorophenyl group, a 2,3-dichlorophenyl group, a 2,4-difluorophenyl group, a 2,4-dichlorophenyl group, a 2,5-difluorophenyl group, a 2,5-dichlorophenyl group, a 2,6-difluorophenyl group, a 2,6-dichlorophenyl group, a 3,4-difluorophenyl group, a 3,4-dichlorophenyl group, a 3,5-difluorophenyl group, a 3,5-dichlorophenyl group, a 2-fluorophenyl group, a 2-chlorophenyl group, a 3-fluorophenyl group, a 3-chlorophenyl group, a 4-fluorophenyl group, a 4-chlorophenyl group, a 4-chloro-2-fluorophenyl group, a 2-chloro-4-fluorophenyl group, a 4-bromo-2-fluorophenyl group, a 2,3,4-trifluorophenyl group, a 2,4,5-trifluorophenyl group, a 2,4,6-trifluorophenyl and the like are used.

As the lower (C₁₋₄) alkylphenyl group, for example, a 2-ethylphenyl group, a 2,6-diisopropylphenyl group and the like are preferably used, and as the lower (C₁₋₄) alkoxyphenyl group, for example, a 4-methoxyphenyl and the like are preferably used.

As the lower (C₁₋₄) alkoxy-carbonylphenyl group, for example, a 2-ethoxycarbonylphenyl group, a 2-methoxycarbonylphenyl group, a 4-methoxycarbonylphenyl group and the like are preferably used, and as the halogeno-lower (C₁₋₄) alkylphenyl group, for example, a 2-trifluoromethylphenyl group and the like are preferably used, and as the halogeno-lower (C₁₋₄) alkoxyphenyl group, for example, a 2-trifluoromethoxyphenyl group, a 4-(2,2,3,3,3-pentafluoropropoxy)phenyl group and the like are preferably used.

As the lower (C₁₋₄₎ alkanoylphenyl group, for example, a 2-acetylphenyl group and the like are preferably used, and as the 5-membered aromatic heterocycle-substituted phenyl group, for example, a 4-(2H-1,2,3-triazol-2-yl)phenyl group, a 4-(2H-tetrazol-2-yl)phenyl group, a 4-(1H-tetrazol-1-yl)phenyl group, a 4-(1H-1,2,3-triazol-1-yl)phenyl group and the like are preferably used, and as the lower (C₁₋₄) alkoxy-carbonyl-lower (C₁₋₄) alkyl-carbamoylphenyl group, for example, a 4-(N-ethoxycarbonylmethylcarbamoyl)phenyl group and the like are preferably used, and as the 1,3-diacylguanidino-lower (C₁-₄) alkylphenyl group, for example, a 4-(1,3-bis-tert-butoxycarbonylguanidinomethyl)phenyl group and the like are preferably used.

As the phenyl group substituted by halogen atom and lower (C₁₋₄) alkyl group, for example, a 2-fluoro-4-methylphenyl group, a 2-chloro-4-methylphenyl group, a 4-fluoro-2-methylphenyl group and the like are preferably used, and as the phenyl group substituted by halogen atom and lower (C₁₋₄) alkoxy-carbonyl group, for example, a 2-chloro-4-methoxycarbonylphenyl group and the like are preferably used, and the phenyl group substituted by halogen atom and cyano group, a 2-chloro-4-cyanophenyl group and the like are preferably used, and as the phenyl group substituted by halogen atom and 5-membered aromatic heterocyclic group, for example, a 2-fluoro-4-(1H-1,2,4-triazol-1-yl)phenyl group and the like are preferably used, and as the phenyl group substituted by halogen atom and lower (C₁₋₄) alkoxy-carbonyl-lower (C₁₋₄) alkyl-carbamoyl group, for example, a 2-chloro-4-(N-tert-butoxycarbonylmethylcarbamoyl)phenyl group, a 2-chloro-4-(N-ethoxycarbonylmethylcarbamoyl)phenyl group and the like are preferably used.

More specifically, as Ar, a phenyl group, a phenyl group substituted by 1 to 3 (particularly 1 or 2) halogen atoms (e.g., a 2,3-difluorophenyl group, a 2,3-dichlorophenyl group, a 2,4-difluorophenyl group, a 2,4-dichlorophenyl group, a 2,5-difluorophenyl group, a 2,5-dichlorophenyl group, a 2,6-difluorophenyl group, a 2,6-dichlorophenyl group, a 3,4-difluorophenyl group, a 3,4-dichlorophenyl group, a 3,5-difluorophenyl group, a 3,5-dichlorophenyl group, a 4-bromo-2-fluorophenyl group, a 2-fluorophenyl group, a 2-chlorophenyl group, a 3-fluorophenyl group, a 3-chlorophenyl group, a 4-fluorophenyl group, a 4-chlorophenyl group, a 2-fluoro-4-chlorophenyl group, a 2-chloro-4-fluorophenyl group, a 2,3,4-trifluorophenyl group, a 2,4,5-trifluorophenyl group etc.), a phenyl group substituted by halogen atom and lower (C₁₋₄) alkyl group (e.g., a 2-chloro-4-methylphenyl group, a 4-fluoro-2-methylphenyl group etc.), etc. are particularly preferable. Of these, a phenyl group substituted by 1 to 3 (particularly 1 or 2) halogen atoms (e.g., a 2,3-dichlorophenyl group, a 2,4-difluorophenyl group, a 2,4-dichlorophenyl group, a 2,6-dichlorophenyl group, a 2-fluorophenyl group, a 2-chlorophenyl group, a 3-chlorophenyl group, a 2-chloro-4-fluorophenyl group, a 2,4,5-trifluorophenyl group etc.), a phenyl group substituted by halogen atom and lower (C₁₋₄) alkyl group (e.g., a 2-chloro-4-methylphenyl group, a 4-fluoro-2-methylphenyl group etc.), etc. are preferable. Particularly, a 2,4-difluorophenyl group, a 2-chlorophenyl group, a 2-chloro-4-fluorophenyl group, a 2-chloro-4-methylphenyl group and the like are preferable, and a 2,4-difluorophenyl group, a 2-chloro-4-fluorophenyl group and the like are preferable.

In this specification, the ring A¹ represents a cycloalkene optionally substituted by 1 to 4 substituents selected from (i) an aliphatic hydrocarbon group optionally having substituents, (ii) an aromatic hydrocarbon group optionally having substituents, (iii) a group represented by the formula -OR¹¹ (wherein R¹¹ is a hydrogen atom or an aliphatic hydrocarbon group optionally having substituents) and (iv) a halogen atom, and a cycloalkene optionally substituted by 1 to 4 substituents selected from (i) an aliphatic hydrocarbon group optionally having substituents, (ii) an aromatic hydrocarbon group optionally having substituents and (iv) a halogen atom is preferable.

These substituents (i) - (iv) are substituted on substitutable carbon atoms in the ring A¹, and when the ring A¹ is substituted by two or more of such substituents, the substituents may be the same or different. A single carbon atom may be substituted by two substituents, and different carbon atoms may be substituted by two or more substituents.

As the "aliphatic hydrocarbon group optionally having substituents" as a substituent on the ring A¹,for example, the same substituents as those of the "aliphatic hydrocarbon group optionally having substituents" represented by R and the like described above may be used.

As the "aromatic hydrocarbon group optionally having substituents" as a substituent on the ring A¹, for example, the same substituents as those of the "aromatic hydrocarbon group optionally having substituents" represented by Ar described above may be used.

As the "heterocyclic group optionally having substituents" as a substituent on the ring A¹, for example, those similar to the "heterocyclic group" which is a "substituent" on the "aliphatic hydrocarbon group optionally having substituents" represented by R and the like described above may be used.

As the substituents for the ring A¹, 1 or 2 C₁₋₆ alkyl groups (e.g., a C₁₋₄ alkyl group such as a methyl group, a tert-butyl group, etc.), a phenyl group, a halogen atom (fluorine, chlorine, bromine, iodine atoms), etc. are preferably used.

As the integer of 1 to 4 represented by n, 1 to 3 is preferable, and 2 is particularly preferable.

As the compound represented by the formula (I), the compound represented by the formula (Ibb') is preferable, and the compound represented by the formula (Inn) is more preferable.

As the compound represented by the formula (Ibb') or the formula (Inn), a compound wherein R¹ is a lower alkyl group (more preferably R¹ is a C₁₋₆ alkyl group) optionally having substituents, R² is a hydrogen atom or a lower (C₁₋₆) alkyl group, Ar is a phenyl group optionally having substituents (more preferably Ar is a phenyl group substituted by 1 or 2 halogen atoms) and n is 1, 2 or 3 (more preferably n is 2) is preferable.

As the compound represented by the formula (I), the compound represented by the formula (Ia): 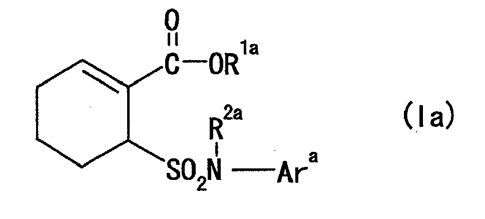 wherein R^{1a} represents a C₁₋₆ alkyl, R^{2a} represents a hydrogen atom or a C₁₋₆ alkyl and Ar^{a} represents a phenyl group substituted by 1 or 2 halogen atoms is preferable. Specifically, as the compound represented by the formula (I), a compound obtained in Reference Example B to be mentioned below and the like is used. Among others,
(i) d-ethyl 6-[N-(2,4-difluorophenyl)sulfamoyl]-1-cyclohexene-1-carboxylate,
(ii) ethyl 6-[N-(2-chlorophenyl)sulfamoyl]-l-cyclohexene-l-carboxylate,
(iii) ethyl 6-[N-(2-chloro-4-methylphenyl)sulfamoyl]-1-cyclohexene-1-carboxylate, and
(iiii) ethyl (6R)-6-[(2-chloro-4-fluoroanilino)sulfonyl]-1-cyclohexene-1-carboxylate and salts thereof and the like are preferable.

The compound of the formula (II) is explained in detail.

As the "aliphatic hydrocarbon group optionally having substituents" "aromatic hydrocarbon group optionally having substituents" and "heterocyclic group optionally having substituents" represented by R¹, those similar to these substituents for R can be used.

As the "aliphatic hydrocarbon group optionally having substituents" represented by R^{1a'} for example, those similar to the aforementioned "aliphatic hydrocarbon group optionally having substituents" represented by R can be used. As R^{1a'}, for example, lower alkyl group having 1 to 6 carbon atoms optionally having substituents (e.g., methyl group, ethyl group, n-propyl group, isopropyl group, n-butyl group, isobutyl group, tert-butoxycarbonylmethyl group, hydroxyethyl group etc.) and the like are preferably used. Of these, for example, methyl group, ethyl group, n-propyl group, isopropyl group, n-butyl group, isobutyl group and the like are preferably used. Particularly, for example, methyl group, ethyl group, n-propyl group and the like are preferable, and ethyl group and the like are specifically preferable.

As the "aliphatic hydrocarbon group optionally having substituents" represented by R^{1b'} and R^{1c'},for example, those similar to the aforementioned "aliphatic hydrocarbon group optionally having substituents" represented by R can be used. As R^{1b'} and R^{1c'} for example, a lower alkyl group having 1 to 6 carbon atoms optionally having substituents (e.g., methyl group, ethyl group, n-propyl group, isopropyl group, n-butyl group, isobutyl group, tert-butoxycarbonylmethyl group, hydroxyethyl group etc.) and the like are preferably used. Of these, for example, methyl group, ethyl group, n-propyl group, isopropyl group, n-butyl group, isobutyl group and the like are preferably used. Particularly, for example, methyl group, ethyl group, n-propyl group and the like are preferable, and ethyl group and the like are specifically preferable.

As R^{1'} for example, a lower alkyl group having 1 to 6 carbon atoms optionally having substituents (e.g., methyl group, ethyl group, n-propyl group, isopropyl group, n-butyl group, isobutyl group, tert-butoxycarbonylmethyl group, hydroxyethyl group etc.) and the like are preferably used. Of these, for example, methyl group, ethyl group, n-propyl group, isopropyl group, n-butyl group, isobutyl group and the like are preferably used. Particularly, for example, methyl group, ethyl group, n-propyl group and the like are preferable, and ethyl group and the like are specifically preferable.

As the "substituent" of the "methylene group optionally having substituents" represented by Y, for example, C₁₋₆ alkyl group (e.g., methyl group, ethyl group, n-propyl group, isopropyl group, n-butyl group, isobutyl group etc.), hydroxy-substituted-C₁₋₆ alkyl group (e.g., hydroxymethyl group, hydroxyethyl group etc.), C₁₋₄ alkoxy-carbonyl-C₁₋₄ alkyl group (e.g., methoxycarbonylmethyl group, ethoxycarbonylmethyl group, tert-butoxycarbonylmethyl group, methoxycarbonylethyl group, ethoxycarbonylethyl group, tert-butoxycarbonylethyl group etc.) and the like can be mentioned. Of these, methyl group is preferable. Unsubstituted methylene is particularly preferable.

As the "substituent" of the "NH optionally having substituents" represented by Y, C₁₋₆ alkyl group (e.g., methyl group, ethyl group, n-propyl group, isopropyl group, n-butyl group, isobutyl group etc.), hydroxy-substituted-C₁₋₆ alkyl group (e.g., hydroxymethyl group, hydroxyethyl group etc.), C₁₋₄ alkoxy-carbonyl-C₁₋₄ alkyl group (e.g., methoxycarbonylmethyl group, ethoxycarbonylmethyl group, tert-butoxycarbonylmethyl group, methoxycarbonylethyl group, ethoxycarbonylethyl group, tert-butoxycarbonylethyl group etc.) and the like can be mentioned. Of these, methyl group is preferable. Unsubstituted NH is particularly preferable.

In the "aromatic hydrocarbon group optionally having substituents" represented by Ar', those similar to the "aromatic hydrocarbon group optionally having substituents" for Ar can be used.

Particularly, as Ar', those similar to Ar are preferable. Among others, a group represented by the formula (c): 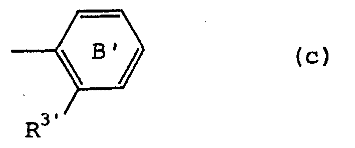 wherein R^{3'} represents a halogen atom or a lower alkyl group, and ring B' is optionally further substituted by 1 to 4 halogen atoms is preferable, and a group represented by the formula (c1): 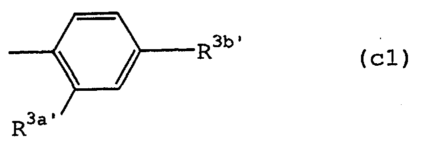 wherein R^{3a'} and R^{3b} are the same or different and each represents a halogen atom is more preferable.

As the halogen atom represented by R^{3'} in the formula (c), halogen atom which is a substituent of ring B' in the formula (c) and the halogen atom represented by R^{3a'} and R^{3b'} in the formula (cl), fluorine atom and chlorine atom are preferable. As the lower alkyl group represented by R^{3'} in the formula (c), for example, C₁₋₄ alkyl group such as methyl, ethyl, propyl and the like can be mentioned. Of the groups represented by the formula (c), a 2,4-difluorophenyl group, a 2-chloro-4-fluorophenyl group, a 2-methyl-4-chlorophenyl group and the like are preferable. Of the groups represented by the formula (c1), a 2,4-difluorophenyl group, a 2-chloro-4-fluorophenyl group and the like are preferable.

X represents a methylene group, NH, a sulfur atom or an oxygen atom, wherein a methylene group and an oxygen atom are preferable.

Ring A' is a 5 to 8-membered ring substituted by a group represented by the formula: -CO-R^{1'} wherein R^{1'} is as defined above and a group represented by the formula: -SO₂-Y-Ar' wherein Y and Ar' are as defined above, and optionally further substituted by 1 to 4 substituents selected from the group consisting of (i) an aliphatic hydrocarbon group optionally having substituents, (ii) an aromatic hydrocarbon group optionally having substituents, (iii) a group represented by the formula: -OR^{2'} wherein R^{2'} is as defined above and (iv) a halogen atom, with preference given to a 5 to 8-membered ring optionally substituted by 1 to 4 substituents selected from (i) an aliphatic hydrocarbon group optionally having substituents, (ii) an aromatic hydrocarbon group optionally having substituents and (iv) a halogen atom.

These substituents are substitutable at substitutable positions on the ring A'. When X constituting the ring is NH or a methylene group, they can substitute the NH and methylene group. When ring A' is substituted by plural substituents, the kinds of such substituents may be the same or different. In addition, two substituents may substitute on the same carbon atom.

As the "aliphatic hydrocarbon group optionally having substituents" and ^aromatic.hydrocarbon group optionally having substituents", which are substituents of ring A', for example, those similar to the aforementioned groups for R can be mentioned.

As the "aliphatic hydrocarbon group optionally having substituents" for R^{2'}, for example, those similar to the aforementioned groups for R can be mentioned.

As the substituent for ring A', 1 or 2 C₁₋₆ alkyl groups (e.g., C₁₋₄ alkyl group such as methyl group, tert-butyl group etc.), phenyl groups, halogen atoms (e.g., fluorine, chlorine, bromine, iodine etc.) and the like are preferably used.

The "s" is an integer of 0 to 2, '"t" is an integer of 1 to 3, and the total of "s" and "t" is not more than 4, with preference given to "s" being 1 and "t" being 1.

A group represented by the formula:

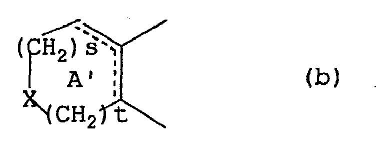

represents a group represented by the formula:

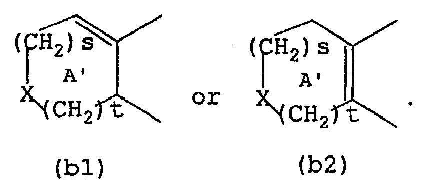

As the compound represented by the formula (II), for example, the following compounds and the like are preferable.
(1) Compound (II) wherein R^{1'} is a group represented by the formula: -OR^{1a'} (R^{1a'} represents a C_{I}-₆ alkyl group),
   the group represented by the formula: 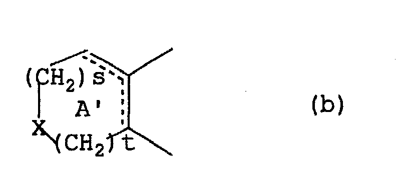 is a group represented by the formula: 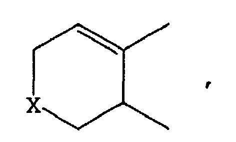 X is a methylene or an oxygen atom,
   Y is a methylene or -NH-, and
   Ar' is a phenyl group optionally having 1 or 2 substituents selected from a halogen atom and a C₁₋₆ alkoxy, thus, a compound represented by the formula (IIa): 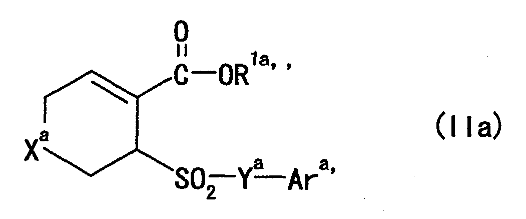 wherein R^{1a"} represents a C₁₋₆ alkyl, X^{a} represents a methylene group or an oxygen atom, Y^{a} represents a methylene group or - NH-, and Ar^{a'} represents a phenyl group optionally having 1 or 2 substituents selected from a halogen atom and a C₁₋₆ alkoxy.
(2) Compound (II) wherein R^{1'} is a group represented by the formula: -ORla' (R^{1a'} represents a C₁₋₆ alkyl group),
   the group represented by the formula: 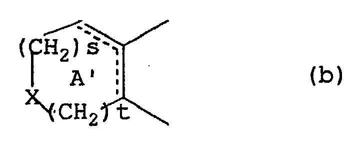 is a group represented by the formula: 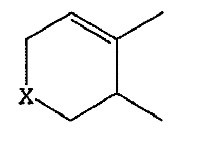 X and Y are each methylene, or X is an oxygen atom and Y is - NH-, and
   Ar' is a phenyl group optionally having two halogen atoms (e.g., 2-chloro-4-fluorophenyl group etc.).
(3) Ethyl 6-(benzylsulfonyl)-l-cyclohexene-l-carboxylate (compound 1),
   ethyl 6-[(4-methoxybenzyl)sulfonyl]-1-cyclohexene-1-carboxylate (compound 2),
   ethyl 6-[(2,4-difluorobenzyl)sulfonyl]-1-cyclohexene-1-carboxylate (compound 3),
   ethyl 6-[(2-chloro-4-fluorobenzyl)sulfonyl]-1-cyclohexene-1-carboxylate (compound 4),
   ethyl (-)-6-[(2-chloro-4-fluorobenzyl)sulfonyl]-1-cyclohexene-1-carboxylate (compound 5),
   ethyl (+)-6-[(2-chloro-4-fluorobenzyl)sulfonyl]-1-cyclohexene-1-carboxylate (compound 6),
   ethyl 3-[(2,4-difluorophenyl)sulfamoyl]-3,6-dihydro-2H-pyran-4-carboxylate (compound 7), and
   ethyl 3-[(2-chloro-4-fluorophenyl)sulfamoyl]-3,6-dihydro-2H-pyran-4-carboxylate (compound 8).
(4) Ethyl 6-[(2-chloro-4-fluorobenzyl)sulfonyl]-1-cyclohexene-1-carboxylate (compound 4),
   ethyl (+)-6-[(2-chloro-4-fluorobenzyl)sulfonyl]-1-cyclohexene-1-carboxylate (compound 6), and
   ethyl 3-[(2-chloro-4-fluorophenyl)sulfamoyl]-3,6-dihydro-2H-pyran-4-carboxylate (compound 8).

When the compounds represented by the formulas (I) and (II) have stereoisomers, each stereoisomer and a mixture of these stereoisomers are encompassed in the present invention.

Furthermore, when the compound represented by the formula (I) is a compound represented by the formula (Icc) or (Inn), and the formula (b) of the compound represented by the formula (II) is the formula (b1) and s and t are 1, each has an optical isomer based on the asymmetric carbon in cycloalkene or cyclohexene ring. Such optical isomer and a mixture of such optical isomers are both encompassed in the present invention.

The compounds (I) and (II) (hereinafter to be simply referred to as a Compound A), which are used for the agent for the prophylaxis or treatment of severe sepsis of the present invention, may be converted into a salt with an inorganic base, organic base, inorganic acid, organic acid, basic or acidic amino acid, and the like. The salt with an inorganic base may, for example, be used an alkaline metal salt such as sodium and potassium salts, etc.; an alkaline earth metal salt such as calcium and magnesium salts, etc.; aluminum salt; ammonium salt; and the like. The salt with an organic base may, for example, be used a salt with trimethylamine, triethylamine, pyridine, picoline, ethanolamine, diethanolamine, triethanolamine, dicyclohexylamine, N,N'-dibenzylethylenediamine, etc. The salt with an inorganic acid may, for example, be used a salt with hydrochloric acid, hydrobromic acid, nitric acid, sulfuric acid, phosphoric acid, etc. The salt with an organic acid may, for example, be used a salt with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, and the like. The salt with a basic amino acid may, for example, be used a salt with arginine, lysine, ornithine, etc. The salt with acidic amino acid may, for example, be used a salt with aspartic acid, glutamic acid, and the like.

A prodrug of Compound A or a salt thereof is a compound which is converted into Compound A as a result of a reaction with an enzyme, gastric acid etc. under physiological conditions in vivo. Thus, the compound is converted into Compound A by enzymatical oxidation, reduction, hydrolysis etc., by hydrolysis due to gastric acid etc. A prodrug of Compound A may be a compound obtained by subjecting an amino group of Compound A to an acylation, alkylation or phosphorylation (e.g., a compound obtained by subjecting an amino group of Compound A to an eicosanoylation, alanylation, pentylaminocarbonylation, 2-hydroxypropionylation, 2-acetoxypropionylation, (5-methyl-2-oxo-1,3-dioxolen-4-yl)methoxycarbonylation, tetrahydrofuranylation, pyrrolidylmethylation, pivaloyloxymethylation, tert-butylation, etc.); a compound obtained by subjecting a hydroxy group of Compound A to an acylation, alkylation, phosphorylation and boration (e.g., a compound obtained by subjecting a hydroxy group of Compound A to an acetylation, palmitoylation, propanoylation, pivaloylation, succinylation, fumarylation, alanylation, dimethylaminomethylcarbonylation, etc.); a compound obtained by subjecting a carboxyl group of Compound A to an esterification or amidation (e.g., a compound obtained by subjecting a carboxyl group of Compound A to an ethyl-esterification, phenyl-esterification, carboxymethyl-esterification, dimethylaminomethyl-esterification, pivaloyloxymethyl-esterification, ethoxycarbonyloxyethyl-esterification, phthalidyl-esterification, (5-methyl-2-oxo-1,3-dioxolen-4-yl)methyl-esterification, cyclohexyloxycarbonylethyl-esterification and methylamidation, etc.) and the like. Any of these compounds can be produced from Compound A by a method known *per se*.

A prodrug of Compound A may also be one which is converted into Compound A under a physiological condition, such as those described in "IYAKUHIN no KAIHATSU (Development of Pharmaceuticals)", Vol.7, Design of Molecules, p. 163-198, Published by HIROKAWA SHOTEN (1990).

The compound (I), a salt thereof and a prodrug thereof can be produced according to a method known per se, for example, a production method described in WO99/46242 or a method analogous thereto. The compound (II), a salt thereof and a prodrug thereof can be produced according to a production method described in WO01/10826 or a method analogous thereto.

When the optically active compound or a salt thereof contains an enantiomer, general separation means may be applied such as diastereomeric salt methods wherein a salt with an optically active acid (e.g., camphor sulfonic acid etc.) or optically active base (e.g., 1-methylbenzylamine etc.) is formed, inclusion compound methods using an optically active host molecule (e.g., 1,6-bis(2-chlorophenyl)-1,6-diphenylhexa-2,4-diyn-1,6-diol), various chromatographies (e.g., liquid chromatography using a chiral column etc.), fractional recrystallization and the like, whereby an optically pure compound can be obtained.

The Compound A, a salt thereof and a prodrug thereof (hereinafter to be comprehensively referred to as Compound A) may be a hydrate or non-hydrate.

The Compound A may be labeled with an isotope (e.g., ³H, ¹⁴C, ³⁵S, ¹²⁵I etc.) and the like.

The cycloalkene compound and Compound A in the present invention are highly safe for humans and can be used for mammals (e.g., rat, mouse, guinea pig, monkey, cattle, dog, pig, human etc.) as a pharmaceutical agent (e.g., an agent for the prophylaxis or treatment of various diseases), veterinary drugs and the like.

Since the cycloalkene compound and Compound A in the present invention have low toxicity, a nitric oxide (NO) production-inhibitory effect and an inhibitory effect on the production of inflammatory cytokines such as TNF-α, IL-1, IL-6, etc., the cycloalkene compound and Compound A are useful as an agent for the prophylaxis or treatment of a mammal (e.g., cat, cattle, dog, horse, goat, monkey, human etc.) against diseases such as cardiac disease, autoimmune disease, inflammatory disease, central nervous system diseases, infectious disease, septic shock, immune dysfunction and the like, including, for example, septicemia, endotoxin shock, exotoxin shock, systemic inflammatory response syndrome (SIRS), compensatory anti-inflammatory response syndrome (CARS), burn, trauma, post-operative complications, cardiac deficiency, shock, hypotension, rheumatoid arthritis, osteoarthritis, gastritis, ulcerative colitis, peptic ulcer, stress-induced gastric ulcer, Crohn's disease, autoimmune disease, post-transplant tissue failure and rejection, postischemic re-perfusion failure, acute coronary microvascular embolism, shock-induced vascular embolism (disseminated intravascular coagulation (DIC) etc.), ischemic cerebral disorder, arteriosclerosis, pernicious anemia, Fanconi's anemia, drepanocythemia, pancreatitis, nephrose syndrome, nephritis, renal failure, insulin-dependent diabetes, insulin-independent diabetes, hepatic porphyria, alcoholism, Parkinson's disease, chronic leukemia, acute leukemia, tumor, myeloma, infantile and adult respiratory distress syndrome, pulmonary emphysema, dementia, Alzheimer's disease, multiple sclerosis, vitamin E deficiency, aging, sunburn, muscular dystrophy, myocarditis, cardiomyopathy, myocardial infarction, myocardial post infarction syndrome, osteoporosis, pneumonia, hepatitis, psoriasis, pain, cataract, influenza infection, malaria, human immunodeficiency virus (HIV) infection, radiation hazard, burn, in vitro fertilization efficiency, hypercalcemia, tonic spondylitis, osteopenia, bone Paget's disease, osteomalacia, fracture, acute bacterial meningitis, *Helicobacter pylori* infection, invasive staphylococcal infection, tuberculosis, systemic mycosis, herpes simplex virus infection, varicella-zoster virus infection, human papilloma virus infection, acute viral encephalitis, encephalitis, meningitis, immune dysfunction due to infections, asthma, atopic dermatitis, allergic rhinitis, reflux esophargitis, fever, hyper cholesteremia, hyperglycemia, hyperlipidemia, diabetic complication, diabetic renal disease, diabetic neuropathy, diabetic retinopathy, gout, gastric atony, hemorrhoid, systemic lupus erythematosus, spinal damage, insomnia, schizophrenia, epilepsy, cirrhosis, hepatic failure, instable angina, valvular disease, dialysis-induced thrombocytopenia or hypotonia, acute ischemic cerebral apoplexy, acute cerebral thrombosis, cancer metastasis, urinary bladder cancer, mammary cancer, uterine cervical cancer, colon cancer, gastric cancer, ovarian cancer, prostatic cancer, parvicellular pulmonary cancer, non-parvicellular pulmonary cancer, malignant melanoma, Hodgkin's disease, non-Hodgkin lymphoma, side effects caused by administration of anticancer agents or immunosuppressants and the like. Accordingly, the agent for the prophylaxis or treatment of severe sepsis, which comprises the cycloalkene compound or Compound A, of the present invention is useful as an agent for the treatment of patients with severe sepsis, who have concurrently developed the above-mentioned diseases.

The cycloalkene compound or Compound A can be used concurrently with other drugs. As the combination drugs, for example, antibacterial agents, antifungal agents, non-steroidal antiinflammatory drugs, steroids, anticoagulants, antithrombotic drugs, thrombolytic drugs, immunomodulators, antiprotozoals, antitussive and expectorant drugs, sedatives, anesthetics, antinarcotics, antiulcer drugs, hyperlipidemia treating agents, therapeutic agents for arteriosclerosis, HDL increasing agents, unstable plaque stabilizing agents, myocardial protecting agent, hypothyroidism treating agent, nephrotic syndrome treating agent, chronic renal failure treating agent, diuretics, hypertension treating agents, cardiac failure treating agents, muscle relaxants, anticonvulsants, cardiacs, vasodilators, vasoconstrictors, antiarrhythmics, antidiabetic drugs, hypertensors, tranquilizers, antipsychotics, therapeutic agents for Alzheimer's diseases, anti-Parkinson drugs, therapeutic agents for amyotrophic spinal lateral sclerosis, neurotrophic factors, antidepressants, therapeutic agents for schizophrenia, antitumor drugs, vitamins, vitamin derivatives, therapeutic agents for arthritis, antirheumatics, antiallergic drugs, antiasthmatics, therapeutic agents for atopic dermatitis, therapeutic agents for allergic rhinitis, therapeutic agents for pollakisuria/anischuria, protease drugs, protease inhibitors, anti-SIDS drugs, anti-sepsis drugs, anti-septic shock drugs, endotoxin-antagonists or -antibodies, signal transduction inhibitors, inhibitors of inflammatory mediator activity, antibodies to inhibit inflammatory mediator activity, inhibitors of inflammatory mediator production, inhibitors of anti-inflammatory mediator activity, antibodies to inhibit anti-inflammatory mediator activity, inhibitors of anti-inflammatory mediator production, α1-adrenergic stimulating agents and the like can be mentioned. Of these, antibacterial agents, antifungal agents, non-steroidal antiinflammatory drugs, steroids, anticoagulants and the like are preferable. Specific examples thereof include the following.
(1) Antibacterial agents
   (A) Sulfa drugs sulfamethizole, sulfisoxazole, sulfamonomethoxine, sulfamethizole, salazosulfapyridine, silver sulfadiazine and the like.
   (B) Quinoline antibacterial agents nalidixic acid, pipemidic acid trihydrate, enoxacin, norfloxacin, ofloxacin, tosufloxacin tosilate, ciprofloxacin hydrochloride, lomefloxacin hydrochloride, sparfloxacin, fleroxacin and the like.
   (C) Antiphthisics isoniazid, ethambutol (ethambutol hydrochloride), p-aminosalicylic acid (calcium p-aminosalicylate), pyrazinamide, ethionamide, protionamide, rifampicin, streptomycin sulfate, kanamycin sulfate, cycloserine and the like.
   (D) Antiacidfast bacterium drugs diaphenylsulfone, rifampicin and the like.
   (E) Antiviral drugs idoxuridine, acyclovir, vidarabine, ganciclovir and the like.
   (F) Anti-HIV agents zidovudine, didanosine, zalcitabine, indinavir sulfate ethanolate, ritonavir and the like.
   (G) Antispirocheteles
   (H) Antibiotics
      tetracycline hydrochloride, ampicillin, piperacillin,
      gentamicin, dibekacin, kanendomycin, lividomycin, tobramycin, amikacin, fradiomycin, sisomycin, tetracycline, oxytetracycline, rolitetracycline, doxycycline, ampicillin, piperacillin, ticarcillin, cephalothin, cephapirin, cephaloridine, cefaclor, cephalexin, cefroxadine, cefadroxil, cefamandole, cefotoam, cefuroxime, cefotiam, cefotiam hexetil, cefuroxime axetil, cefdinir, cefditoren pivoxil, ceftazidime, cefpiramide, cefsulodin, cefmenoxime, cefpodoxime proxetil, cefpirome, cefozopran, cefepime, cefsulodin, cefmenoxime, cefmetazole, cefminox, cefoxitin, cefbuperazone, latamoxef, flomoxef, cefazolin, cefotaxime, cefoperazone, ceftizoxime, moxalactam, thienamycin, sulfazecin, aztreonam or a salt thereof, griseofulvin, lankacidin-group [Journal of Antibiotics, 38, 877-885(1985)] and the like.
(2) Antifungal agents
   (A) polyethylene antibiotics (e.g., amphotericin B, nystatin, trichomycin)
   (B) griseofulvin, pyrrolnitrin and the like.
   (C) cytosine metabolism antagonists (e.g., flucytosine)
   (D) imidazole derivatives (e.g., econazole, clotrimazole, miconazole nitrate, bifonazole, croconazole)
   (E) triazole derivatives (e.g. fluconazole, itraconazole, azole compound [2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone]
   (F) thiocarbamic acid derivatives (e.g. trinaphthol)
   (G) echinocandin derivatives (e.g., caspofungin, micafungin, anidulafungin) and the like.
(3) Non-steroidal antiinflammatory drugs acetaminophen, phenacetin, ethenzamide, sulpyrine, antipyrine, migrenin, aspirin, mefenamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, epirizole, tiaramide hydrochloride, zaltoprofen, gabexate mesilate, camostat mesilate, urinastatin, colchicine, probenecid, sulfinpyrazone, benzbromarone, allopurinol, gold sodium thiomalate, sodium hyaluronate, sodium salicylate, morphine hydrochloride, salicylic acid, atropine, scopolamine, morphine, pethidine, levorphanol, ketoprofen, naproxen, oxymorphone or a salt thereof, and the like.
(4) Steroids dexamethasone, hexestrol, methimazole, betamethasone, triamcinolone, triamcinolone acetonide, fluocinonide, fluocinolone acetonide, prednisolone, methylprednisolone, cortisone acetate, hydrocortisone, fluorometholone, beclometasone propionate, estriol and the like.
(5) Anticoagulants heparin sodium, sodium citrate, activated protein C, tissue factor pathway inhibitor, antithrombin III, dalteparin sodium, warfarin potassium, argatroban, gabexate, sodium citrate and the like.
(6) Antithrombotic drugs ozagrel sodium, ethyl icosapentate, beraprost sodium, alprostadil, ticlopidine hydrochloride, pentoxifylline, dipyridamole and the like.
(7) Thrombolytic drugs tisokinase, urokinase, streptokinase and the like.
(8) Immunomodulators cyclosporin, tacrolimus, gusperimus, azathioprine, antilymphocyte serum, dried sulfonated immunoglobulin, erythropoietin, colony-stimulating factor, interleukin, interferon and the like.
(9) Antiprotozoals metronidazole, tinidazole, diethylcarbamazine citrate, quinine hydrochloride, quinine sulfate and the like.
(10) Antitussive and expectorant drugs ephedrine hydrochloride, noscapine hydrochloride, codeine phosphate, dihydrocodeine phosphate, isoproterenol hydrochloride, ephedrine hydrochloride, methylephedrine hydrochloride, noscapine hydrochloride, alloclamide, chlophedianol, picoperidamine, chloperastine, protokylol, isoproterenol, salbutamol, terbutaline, oximetebanol, morphine hydrochloride, dextromethorphan hydrobromide, oxycodone hydrochloride, dimemorphan phosphate, tipepidine hibenzate, pentoxyverine citrate, clofedanol hydrochloride, benzonatate, guaifenesin, bromhexine hydrochloride, ambroxol hydrochloride, acetylcysteine, ethyl cysteine hydrochloride, carbocysteine and the like.
(11) Sedatives chlorpromazine hydrochloride, atropine sulfate, phenobarbital, barbital, amobarbital, pentobarbital, thiopental sodium, thiamylal sodium, nitrazepam, estazolam, flurazepam, haloxazolam, triazolam, flunitrazepam, bromovalerylurea, chloral hydrate, triclofos sodium and the like.
(12) Anesthetics
   (12-1) Local anesthetics cocaine hydrochloride, procaine hydrochloride, lidocaine, dibucaine hydrochloride, tetracaine hydrochloride, mepivacaine hydrochloride, bupivacaine hydrochloride, oxybuprocaine hydrochloride, ethyl aminobenzoate, oxethazaine) and the like.
   (12-2) General anesthetics
      (A) inhalation anesthetics (e.g., ether, halothane, nitrous oxide, isoflurane, enflurane),
      (B) intravenous anesthetics (e.g., ketamine hydrochloride, droperidol, thiopental sodium, thiamylal sodium, pentobarbital) and the like.
(13) Antinarcotics levallorphan, nalorphine, naloxone or a salt thereof and the like.
(14) Antiulcer drugs metoclopromide, histidine hydrochloride, lansoprazole, metoclopramide, pirenzepine, cimetidine, ranitidine, famotidine, urogastrone, oxethazaine, proglumide, omeprazole, sucralfate, sulpiride, cetraxate, gefarnate, aldioxa, teprenone, prostaglandin and the like.
(15) Hyperlipidemia treating agents HMG-CoA reductase inhibitors (e.g., fluvastatin, cerivastatin, atorvastatin etc.), fibrates (e.g., simfibrate, clofibrate aluminum, clinofibrate, fenofibrate etc.), adsorbents for bile acid (e.g., cholestyramide etc.), nicotinic acid formulations (e.g., nicomol, niceritrol, tocopherol nicotinate etc.), probucol and a derivative thereof, polyvalent unsaturated fatty acid derivatives (e.g., ethyl icosapentate, polyene phosphatidylcholine, melinamide etc.), vegetable sterols (e.g., γ-oryzanol, soysterol etc.), elastases, sodium dextran sulfate, squalene synthetase inhibitor, squalene epoxidase inhibitor, CETP inhibitor, ethyl 2-chloro-3-[4-(2-methyl-2-phenylpropoxy)phenyl]propionate [Chem. Pharm. Bull., 38, 2792, 2796 (1990)], LDL receptor enhancing drug, cholesterol absorption inhibitor (Ezetimibe etc.), MTP inhibitor, ileal bile acid transporter inhibitor, SCAP ligand, FXR ligand and the like.
(16) Therapeutic agents for arteriosclerosis MMP inhibitor, chymase inhibitor, ACAT inhibitor(Avasimibe, Eflucimibe etc.), apoAI Milano and an analogue thereof, scavenger receptor inhibitor, 15-lipoxygenase inhibitor, phospholipase A2 inhibitor, ABCA1 activator, LXR ligand, sphingomyelinase inhibitor, paraoxonase activator, estrogen receptor agonist and the like.
(17) HDL increasing agents squalene synthetase inhibitors, CETP inhibitors, LPL activators and the like.
(18) Unstable plaque stabilizing agents MMP inhibitors, kinase inhibitors, ACAT inhibitors, lipid-rich plaque regressing agents and the like.
(19) Myocardial protecting agents cardiac ATP-K oral formulation, endoserine antagonist, urotensin antagonist and the like.
(20) Hypothyroidism treating agents dried thyroid gland (thyreoid), levothyroxine sodium (thyradin S), liothyronidin sodium (thyronine, thyromin) and the like.
(21) Nephrotic syndrome treating agents prednisolone (Predonine), prednisolone succinate sodium (Predonine), methylprednisolone succinate sodium (Solu medrol), betamethasone (rinderon) and the like.
(22) Chronic renal failure treating agents diuretics [e.g., furosemide (lasix), bumetamide (lunetron), azosemide (diart)], hypotensive agent (e.g., ACE inhibitor, (enalapril maleate (renivase)) and Ca antagonist (manidipine), α-receptor blocker, AII antagonist (candesartan)] and the like.
(23) Diuretics thiazide diuretics (benzylhydro-chlorothiazide, cyclopenthiazide, ethiazide, hydrochlorothiazide, hydroflumethiazide, methyclothiazide, penfluthiazide, polythiazide, trichloromethiazide etc.), loop diuretics (clortharidone, clofenamide, indapamide, mefruside, meticrane, sotolazone, tripamide, quinethazone, metolazone, furosemide etc.), potassium retaining diuretics (spironolacton, triamterene etc.).
(24) Hypertension Treating Agents
   (i) sympathetic nerve suppressants α₂ stimulants (e.g., clonidine, guanabenz, guanfacine, methyldopa etc.), ganglionic blocking agents (e.g., hexamethonium, trimethaphan etc.), presynaptic blockers (e.g., alseroxylon, dimethylaminoreserpinate, rescinamine, reserpine, syrosingopine etc.), neuron blockers (e.g., betanidine, guanethidine etc.), α₁ blockers (e.g., bunazosin, doxazocin, prazosin, terazosin, urapidil etc.), β blockers (e.g., propranolol, nadolol, timolol, nipradilol, bunitrolol, indenolol, penbutolol, carteolol, carvedilol, pindolol, acebutolol, atenolol, bisoprolol, metoprolol, labetalol, amosulalol, arotinolol etc.).
   (ii) vasodilators calcium channel antagonists (e.g., manidipine, nicardipine, nilvadipine, nisoldipine, nitrendipine, benidipine, amlodipine, aranidipine etc.), phthalazine derivatives (e.g., budralazine, cadralazine, ecarazine, hydralazine, todralazine etc.) and the like.
   (iii) ACE inhibitors alacepril, captopril, cilazapril, delapril, enalapril, lisinopril, temocapril, trandolapril, quinapril, imidapril, benazepril, perindopril and the like.
   (iv) AII antagonists losartan, candesartan, valsartan, termisartan, irbesartan, forasartan and the like.
   (v) diuretics (for example, diuretics described above)
(25) Cardiac failure treating agents cardiotonic agents (e.g., digitoxin, digoxin, methyldigoxin, lanatoside C, proscillaridine etc.), α,β-stimulants (e.g., epinephrine, norepinephrine, isoproterenol, dopamine, docarpamine, dobutamine, denopamine etc.), phosphodiesterase inhibitors (e.g., aminone, milrinone, olprinone hydrochloride etc.), calcium channel sensitivity promoters (e.g., pimobendan etc.), nitrate agents (e.g., nitroglycerin, isosorbide nitrate etc.), ACE inhibitors (for example, ACE inhibitors described above), diuretics (for example, diuretics described above), carperitide, ubidecarenone, vesnarinone, aminophylline and the like.
(26) Muscle relaxants pridinol, tubocurarine, pancuronium, tolperisone hydrochloride, chlorphenesin carbamate, baclofen, chlormezanone, mephenesin, chlorzoxazone, eperisone, tizanidine and the like.
(27) Anticonvulsants phenytoin, ethosuximide, acetazolamide, chlordiazepoxide, trimethadione, carbamazepine, phenobarbital, primidone, sulthiame, sodium valproate, clonazepam, diazepam, nitrazepam and the like.
(28) Cardiacs trans-pi-oxocamphor, terephyllol, aminophyllin, etilefrine, dopamine, dobutamine, denopamine, aminophyllin, bencirin, amrinone, pimobendan, ubidecarenone, digitoxin, digoxin, methyldigoxin, lanatoside C, G-strophanthin and the like.
(29) Vasodilators oxyfedrine, diltiazem, tolazoline, hexobendine, bamethan, clonidine, methyldopa, guanabenz and the like.
(30) Vasoconstrictors dopamine, dobutamine, denopamine and the like.
(31) Antiarrhythmics
   (A) Na channel blockers (e.g., quinidine, procainamide, disopyramide, ajmaline, cibenzoline, lidocaine, diphenylhydantoin, mexiletine, propafenone, flecainide, pilsicainide, phenitoin etc.),
   (B) β-blockers (e.g., propranolol, alprenolol, bufetolol, oxprenolol, atenolol, acebutolol, metoprolol, bisoprolol, pindolol, carteolol, arotinolol etc.),
   (C) K channel blockers (e.g., amiodarone etc.),
   (D) Ca channel blockers (e.g., verapamil, diltiazem etc.) and the like.
(32) Hypertensors dopamine, dobutamine, denopamine, digitoxin, digoxin, methyldigoxin, lanatoside C, G-strophanthin and the like.
(33) Antidiabetic drugs sulfonylureas (e.g., tolbutamide, chlorpropamide, glyclopyramide, acetohexamide, tolazamide, glibenclamide, glybuzole etc.), biguanides (e.g., metformin hydrochloride, buformin hydrochloride etc.), α-glucosidase inhibitors (e.g., voglibose, acarbose etc.), insulin resistance improving agents (e.g., pioglitazone, rosiglitazone, troglitazone etc.), insulin, glucagon, agents for treating diabetic complications (e.g., epalrestat etc.) and the like.
(34) Tranquilizers diazepam, lorazepam, oxazepam, chlordiazepoxide, medazepam, oxazolam, cloxazolam, clotiazepam, bromazepam, etizolam, fludiazepam, hydroxyzine and the like.
(35) Antipsychotics
   chlorpromazine hydrochloride, prochlorperazine, trifluoperazine, thioridazine hydrochloride, perphenazine maleate, fluphenazine enanthate, prochlorperazine maleate, levomepromazine maleate, promethazine hydrochloride, haloperidol, bromperidol, spiperone, reserpine, clocapramine hydrochloride, sulpiride, zotepine and the like.
(36) Therapeutic agents for Alzheimer's diseases
   (i) choline esterase inhibitors such as donepezil, rivastigmine, galanthamine, TAK-147 and the like.
   (ii) cerebral function activators such as Idebenone, Memantine, vinpocetine and the like.
(37) Anti-Parkinson drugs
   L-dopa, Deprenyl, carbidopa+levodopa, Pergolide, Ropinirole, cabergoline, Pramipexol, Entacapone, Lazabemide and the like.
(38) Therapeutic agents for amyotrophic spinal lateral sclerosis riluzole, mecasermin, Gabapentin and the like.
(39) Antidepressants imipramine, clomipramine, noxiptiline, phenelzine, amitriptyline hydrochloride, nortriptyline hydrochloride, amoxapine, mianserin hydrochloride, maprotiline hydrochloride, sulpiride, fluvoxamine maleate, trazodone hydrochloride and the like.
(40) Therapeutic agents for schizophrenia Olanzapine, risperidone, Quetiapine, Iloperidone and the like.
(41) Antitumor drugs 6-O-(N-chloroacetylcarbamoyl) fumagillol, bleomycin, methotrexate, actinomycin D, mitomycin C, daunorubicin, adriamycin, neocarzinostatin, cytosine arabinoside, fluorouracil, tetrahydrofuryl-5-fluorouracil, picibanil, lentinan, levamisole, bestatin, azimexon, glycyrrhizin, doxorubicin hydrochloride, aclarubicin hydrochloride, bleomycin hydrochloride, peplomycin sulfate, vincristine sulfate, vinblastine sulfate, irinotecan hydrochloride, cyclophosphamide, melphalan, busulphan, thiotepa, procarbazine hydrochloride, cisplatin, azathioprine, mercaptopurine, tegafur, carmofur, cytarabine, methyltestosterone, testosterone propionate, testosterone enanthate, mepitiostane, fosfestol, chlormadinone acetate, leuprorelin acetate, buserelin acetate and the like.
(42) Vitamins
   (A) vitamin A: vitamin A₁, vitamin A₂ and retinol palmitate
   (B) vitamin D: vitamin D₁, D₂, D₃, D₄ and D₅
   (C) vitamin E: α-tocopherol, β-tocopherol, γ-tocopherol, δ-tocopherol, dl-α-tocopherol nicotinate
   (D) vitamin K: vitamin K₁, K₂, K₃ and K₄
   (E) folic acid (vitamin M)
   (F) vitamin B: vitamin B₁, vitamin B₂, vitamin B₃, vitamin B₅, vitamin B₆ and vitamin B₁₂
   (G) biotin (vitamin H) and the like.
(43) Vitamin derivatives various derivatives of vitamins, for example, vitamin D₃ derivatives such as 5,6-trans-cholecalciferol, 2,5-hydroxycholecalciferol, 1-α-hydroxycholecalciferol and the like, vitamin D₂ derivatives such as 5,6-trans-ergocalciferol and the like.
(44) Antiallergic drugs diphenhydramine, chlorpheniramine, tripelennamine, metodilamine, clemizole, diphenylpyraline, methoxyphenamine, sodium cromoglycate, tranilast, repirinast, amlexanox, ibudilast, ketotifen, terfenadine, mequitazine, azelastine, epinastine, ozagrel hydrochloride, pranlkast hydrate, seratrodast and the like.
(45) Antiasthmatics isoprenaline hydrochloride, salbutamol sulfate, procaterol hydrochloride, terbutaline sulfate, trimetoquinol hydrochloride, tulobuterol hydrochloride, orciprenaline sulfate, fenoterol hydrobromide, ephedrine hydrochloride, iprotropium bromide, oxitropium bromide, flutropium bromide, theophyline, aminophyllin, sodium cromoglycate, tranilast, repirinast, anrexanone, ibudilast, ketotifen, terfenadine, mequitazine, azelastine, epinastine, ozagrel hydrochloride, pranlkast hydrate, seratrodast, dexamethasone, prednisolone, hydrocortisone, beclometasone dipropionate and the like.
(46) Therapeutic agents for atopic dermatitis sodium cromoglycate and the like.
(47) Therapeutic agents for allergic rhinitis sodium cromoglycate, chlorpheniramine maleate, alimemazine tartrate, clemastine fumarate, homochlorcyclizine hydrochloride, terfenadine, mequitazine and the like.
(48) Therapeutic agents for pollakisuria/anischuria flavoxate hydrochloride and the like.
(49) Anti-sepsis drugs peptidic compounds such as rBPI-21 (bactericidal permeability increasing protein), BI-51017 (antithrombin III), SC-59735 (rTFPI), r-PAF acetylhydrase, LY-203638 (r-activated protein C), anti-TNF-α antibody and the like, and non-peptidic compounds such as JTE-607, E-5531, E-5564, S-5920, FR-167653, ONO-1714, ONO-5046 (sivelestat), GW-273629, RWJ-67657 and the like.
(50) Others hydroxycam, diaserine, megestrol acetate, nicerogolin, prostaglandins and the like.

A combined use of the cycloalkene compound or Compound A and other drugs provides the following effects.
(1) The dose of the above-mentioned cycloalkene compound or Compound A and a combination drug can be lower than in the case of a sole administration of these compounds.
(2) A synergistic therapeutic effect can be achieved against the above-mentioned sepsis, particularly severe sepsis, septic shock, inflammatory diseases, infectious diseases and the like.
(3) A broad range of therapeutic effects can be achieved against various diseases developed in association with viral infection and the like.

With regard to the combined use, the cycloalkene compound or Compound A and a combination drug are free of any limitation on the timing of the administration. The cycloalkene compound or Compound A or a pharmaceutical composition thereof and the combination drug or a pharmaceutical composition thereof may be simultaneously administered to the administration object, or may be administered with time difference. The dose of the combination drug follows a clinical dose and can be appropriately determined depending on the administration object, administration route, disease, combination and the like.

The mode of administration of the combination agent is not particularly limited, as long as the cycloalkene compound or Compound A and the combination drug are combined for administration. As the mode of such administration, for example, (1) administration of a single preparation obtained by simultaneous addition of the cycloalkene compound or Compound A or a pharmaceutical composition thereof and the combination drug, (2) simultaneous administration of two kinds of preparations obtained by separate preparation of the cycloalkene compound or Compound A or a pharmaceutical composition thereof and the combination drug or a pharmaceutical composition thereof, by a single administration route, (3) time staggered administration of two kinds of preparations obtained by separate preparation of the cycloalkene compound or Compound A or a pharmaceutical composition thereof and the combination drug or a pharmaceutical composition thereof, by the same administration route, (4) simultaneous administration of two kinds of preparations obtained by separate preparation of the cycloalkene compound or Compound A or a pharmaceutical composition thereof and the combination drug or a pharmaceutical composition thereof, by different administration routes, (5) time staggered administration of two kinds of preparations obtained by separate preparation of the cycloalkene compound or Compound A or a pharmaceutical composition thereof and the combination drug or a pharmaceutical composition thereof, by different administration routes, such as administration in the order of the cycloalkene compound or Compound A or a pharmaceutical composition thereof and then the combination drug or a pharmaceutical composition thereof, or in a reversed order, and the like are exemplified.

The combination ratio of the cycloalkene compound or Compound A and a combination drug in the combination agent of the present invention can be appropriately determined depending on the administration object, administration route, disease and the like.

The content of the cycloalkene compound or Compound A in the combination agent of the present invention varies depending on the form of the preparation. It is generally about 0.01 - 100 wt%, preferably about 0.1 - 50 wt%, more preferably about 0.5 - 20 wt%, based on the preparation in total.

The content of the combination drug in the combination agent of the present invention varies depending on the form of the preparation. It is generally about 0.01 - 100 wt%, preferably about 0.1 - 50 wt%, more preferably about 0.5 - 20 wt%, based on the preparation in total.

The content of the additive, such as a carrier, in the combination drug of the present invention varies depending on the form of the preparation. It is generally about 1 - 99.99 wt%, preferably about 10 - 90 wt%, based on the preparation in total.

When the cycloalkene compound or Compound A and the combination drug are prepared into separate pharmaceutical preparations, the contents as mentioned above can be employed.

When the cycloalkene compound or compound A is administered to a human, it can be safely administered orally or parenterally as it is or in a mixture with an appropriate pharmacologically acceptable carrier, excipient and diluent, in a pharmaceutical composition such as an oral formulation (e.g., powder, granule, tablet, capsule etc.), a parenteral formulation (e.g., injection, external formulation (e.g., nasal formulation, percutaneous formulation etc.) and suppository (e.g., rectal suppository and vaginal suppository etc.) .

Any of these formulations may be produced by any method known per se which is employed ordinarily for producing a pharmaceutical formulation. The amount of the cycloalkene compound or compound A to be incorporated into a formulation may vary depending on the dosage forms, and is preferably about 10 to 95% by weight in an oral formulation described above and about 0.001 to about 95% by weight in a parenteral formulation described above.

For example, a cycloalkene compound or Compound A can be prepared into an aqueous injection together with a solubilizer (e.g., β-cyclodextrins etc.), a dispersant (e.g., Tween 80 (manufactured by ATLASPOWDER USA), HCO 60 (manufactured by NIKKO CHEMICALS), carboxymethylcellulose, sodium arginate etc.), a preservative (e.g., methyl paraben, propyl paraben, benzyl alcohol chlorobutanol etc.), an isotonic agent (e.g., sodium chloride, glycerine, sorbitol, glucose etc.) and the like, or into an oil-based injection by dissolving, suspending or emulsifying using a vegetable oil (e.g., olive oil, sesame oil, peanut oil, cottonseed oil, corn oil etc.) and propylene glycol and the like.

An oral formulation can be produced by a method known per se by, for example, compressing the cycloalkene compound or Compound A together with an excipient (e.g., lactose, sucrose, starch etc.), a disintegrant (e.g., starch, calcium carbonate etc.), a binder (e.g., starch, gum arabic, carboxymethyl cellulose, polyvinyl pyrrolidone, hydroxypropyl cellulose etc.), a lubricant (e.g., talc, magnesium stearate, polyethylene glycol 6000 etc.), and the like, followed by, where necessary, a coating process known per se for the purpose of masking a taste, forming an enteric coat, or achieving a sustained release. For such coating may be used, for example, hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (manufactured by ROHM, Germany, a copolymer of methacrylic acid and acrylic acid), a dye (e.g., colcothar, titanium dioxide etc.) and the like.

The cycloalkene compound or compound A can also be employed as an external formulation in the form of a solid or semi-solid or a liquid.

For example, a solid external formulation may be the cycloalkene compound or compound A as it is or in a mixture with an excipient (e.g., glycol, mannitol, starch, microcrystalline cellulose etc.), a thickening agent (e.g., natural gums, cellulose derivatives, acrylic acid polymers etc.) which is then converted into a powder composition. A semi-solid external formulation may be produced by a standard method and preferably used in the form of an aqueous or oil-based gel or ointment. A liquid external formulation may be produced by a method employed for producing an injection formulation or an analogous method in the form of an oil-based or aqueous suspension.

The solid, semi-solid or liquid external formulation may be supplemented also with a pH modifier (e.g., carbonic acid, phosphoric acid, citric acid, hydrochloric acid, sodium hydroxide etc.), an antiseptic (e.g., p-oxybenzoates, chlorobutanol, benzalkonium chloride etc.) and the like, as appropriate. Typically, a vaseline or a lanolin is used as a formulation base, per 1 g of which about 0.1 to 100 mg of the cycloalkene compound or compound A is contained to form an ointment.

The cycloalkene compound or Compound A may be also formulated as an oil or aqueous, solid or semi-solid or liquid suppository by a method known per se. As an oil base, for example, a high fatty acid glyceride (e.g., cocoa butter, WITEPSOL (manufactured by DYNAMIT NOBEL, Germany) etc.), a middle fatty acid (e.g., MYGLYOL (manufactured by DYNAMIT NOBEL, Germany) etc.), a vegetable oil (e.g., sesame oil, soybean oil, cottonseed oil etc.) and the like are used as appropriate. An aqueous base may be, for example, polyethylene glycol or propylene glycol, and an aqueous gel base may be, for example, a natural gum, a cellulose derivative, a vinyl polymer, an acrylic polymer and the like.

While the dose of the cycloalkene compound or Compound A varies depending on the patient's age, body weight and condition, the dosage form, the mode and the period of the treatment, the dose of the cycloalkene compound or Compound A may be, for example, generally about 0.01 to about 1000 mg/kg, preferably about 0.01 to about 100 mg/kg, more preferably about 0.1 to about 100 mg/kg, most preferably about 0.1 to about 50 mg/kg, and particularly about 1.5 to about 30 mg/kg, as the amount of the Compound A, per day for a patient with severe sepsis (adult weighing about 60 kg), and said daily dose is given orally or parenterally all at once or in several portions a day. It is a matter of course that a lower daily dose may be sufficient or an excessive dose may be required since the dose may vary depending on various factors as discussed above.

While the dose of the combination agent of the present invention varies depending on the kind of the compound, the patient's age, body weight and condition, the dosage form, the mode and the period of the treatment, the dose of the combination agent may be, for example, generally about 0.01 to about 1000 mg/kg, preferably about 0.01 to about 100 mg/kg, more preferably about 0.1 to about 100 mg/kg, most preferably about 0.1 to about 50 mg/kg, and particularly about 1.5 to about 30 mg/kg, as the amount of the cycloalkene compound or Compound A and the combination drug, per day for a patient with severe sepsis (adult weighing about 60 kg), said daily dose being given intravenously all at once or in several portions during a day. It is a matter of course that a lower daily dose may be sufficient or an excessive dose may be required since the dose may vary depending on various factors as discussed above.

The combination drug may be contained in any amount as long as a side effect does not pose a problem. While the daily dose of the combination drug may vary depending on the disease state, the age, sex, body weight and difference in sensitivity of the administration object, timing and interval of administration, characteristics, dispensing and kind of the pharmaceutical preparation, the kind of active ingredient and the like and is not particularly limited, the amount of the drug is generally about 0.001 - 2000 mg, preferably about 0.01 - 500 mg, more preferably about 0.1 - 100 mg, per 1 kg body weight of mammal by oral administration, which is generally administered all at once or in 2 to 4 portions during a day.

When the combination agent of the present invention is administered, a cycloalkene compound or Compound A and a combination drug may be administered at the same time, or a combination drug may be administered first, and then the cycloalkene compound or Compound A may be administered. Alternatively, the cycloalkene compound or Compound A may be administered first, and then the combination drug may be administered. For time staggered administration, the time difference varies depending on the active ingredient to be administered, dosage form and administration route. For example, when a combination drug is to be administered first, the cycloalkene compound or Compound A is administered within 1 min - 3 days, preferably 10 min - 1 day, more preferably 15 min - 1 hour, after the administration of the combination drug. When the cycloalkene compound or Compound A is to be administered first, the combination drug is administered within 1 min - 1 day, preferably 10 min - 6 hours, more preferably 15 min - 1 hour, after the administration of the combination drug.

The present invention also provides a TLR signal inhibitor comprising a non-peptide compound. By the "TLR signal" is meant a signal transduction, with which any Toll-like receptor recognizes bacterial component and the like of microorganism and induces biological defense response, and, for example, signal transduction via known TLR1-TLR10 can be mentioned.

The non-peptide compound which is an active ingredient of the TLR signal inhibitor of the present invention (hereinafter sometimes to be referred to as the inhibitor of the present invention) is free of any particular limitation, as long as it can inhibit signal transduction via any of the above-mentioned TLRs, and can suppress the production of inflammatory mediators such as NO and/or cytokine. While one capable of specifically inhibiting signal transduction via TLR4 is preferable, one that specifically inhibits other TLR signals and one capable of inhibiting plural kinds of TLRs are also preferable. For example, a low molecular non-peptide compound having a molecular weight of not more than about 1000, preferably about not more than 500, is used, and particularly, the cycloalkene compound or the above-mentioned compound A is preferably used.

These non-peptide compounds are highly safe for humans and can be used for mammals (e.g., rat, mouse, guinea pig, monkey, cattle, dog, pig, human etc.) as a pharmaceutical agent (e.g., an agent for the prophylaxis or treatment of various diseases), veterinary drugs and the like.

Since the non-peptide compound in the present invention has low toxicity, a TLR signal inhibitory action and an inhibitory effect on inflammatory mediator such as the production of NO and/or cytokine, the non-peptide compound is useful for the prophylaxis and treatment of diseases caused by changes in the signals, such as organ dysfunction and the like. As the organs here, various organs of central nervous system, circulatory system, respiratory system, bone and joint system, digestive system or renal and urinary system can be mentioned. The TLR signal inhibitor comprising the non-peptide compound of the present invention is specifically useful for the prophylaxis and/or treatment of diseases caused by changes in TLR signals, such as
(1) central nervous system diseases [(i) neurodegenerative disease (e.g., senile dementia, Alzheimer's disease, Down's syndrome, Parkinson's syndrome, Creutzfeldt-Jakob disease, amyotrophic spinal lateral sclerosis, diabetic neuropathy etc.), (ii) neuropathy in cerebrovascular diseases (e.g. impairment of cerebral blood flow based on cerebral infarction, cerebral hemorrhage, cerebral sclerosis, etc.), brain trauma, spiral cord injury, cerebritis sequela and cerebral palsy, (iii) dysmnesia (e.g. senile dementia, amnesia, etc.) and the like], particularly Alzheimer's disease,
(2) circulatory system diseases [(i) coronary artery syndrome such as acute cardiac infarction and unstable angina pectoris, (ii) peripheral obstruction, (iii) restenosis after coronary intervention (percutaneous transluminal coronary angioplasty (PTCA), atherectomy (DCA), stenting etc.), (iv) restenosis after coronary bypass surgery, (v) restenosis after other peripheral arterial interventions (angioplasty, atherectomy, stenting etc.) and bypass surgery, (vi) ischemic heart disease such as cardiac infarction and angina pectoris, (vii) intermittent claudication, (viii) stroke (cerebral infarction, cerebral embolus, cerebral hemorrhage etc.), (ix) lacunar infarct, (x) cerebrovascular dementia, (xi) arteriosclerosis (e.g., atherosclerosis etc.) and diseases caused thereby (e.g., ischemic heart disease such as cardiac infarction, cerebrovascular disorder such as cerebral infarction·stroke, etc.), (xii) cardiac failure, (xiii) arrhythmia, (xiv) progression of focus of arteriosclerosis, (xv) thrombogenesis, (xvi) hypotension, (xvii) shock, (xviii) shock-induced vascular embolism (disseminated intravascular coagulation (DIC) etc.)], particularly arteriosclerosis,
(3) respiratory system diseases [respiratory distress syndrome, respiratory failure, emphysema, pneumonia, bronchitis, bronchiolitis and the like],
(4) diseases of bone and joint system [arthritis rheumatoides, osteoporosis, osteomalacia, osteopenia, Paget's disease of bone, osteomalacia and the like], particularly arthritis rheumatoides,
(5) diseases of digestive·liver, biliary tract and pancreas system [ulcerative colitis, gastritis, digestive ulcer, cirrhosis, hepatic failure, hepatitis, cholecystitis, pancreatitis and the like], particularly ulcerative colitis,
(6) diseases of renal and urinary system [nephritis, kidney failure, cystitis and the like]
or a combination of these diseases (multiple organ failure etc.) and the like. Moreover, the TLR signal inhibitor comprising a non-peptide compound of the present invention is useful for the prophylaxis and/or treatment of infectious diseases caused by changes in TLR signals, particularly sepsis (severe sepsis) accompanying organ dysfunction.

Accordingly, the present invention also provides an agent for the prophylaxis or treatment of a disease caused by changes in TLR signals (e.g., the above-mentioned diseases), comprising a TLR signal inhibitor comprising a non-peptide compound (preferably having a molecular weight of not more than about 1000), preferably a cycloalkene compound or the above-mentioned compound A, as an active ingredient.

The non-peptide compound in the present invention can be formulated by a method similar to the method for the above-mentioned cycloalkene compound or compound A, as a TLR signal inhibitor or an agent for the prophylaxis or treatment of a disease caused by changes in TLR signals, which contains the compound, and can be administered to mammals by way of the same administration route, dose and the like as those mentioned above.

The non-peptide compound in the present invention can be used in combination with the above-mentioned combination drug for cycloalkene compound or compound A, for the prophylaxis and/or treatment of the above-mentioned diseases. Particularly, for the prophylaxis and/or treatment of severe sepsis, the compound can be used in combination with at least one kind of a drug selected from antibacterial agent, antifungal agent, non-steroidal antiinflammatory drug, steroid and anticoagulant. In addition, for the prophylaxis and/or treatment of central nervous system diseases such as Alzheimer's disease, the compound can be used in combination with at least one kind of a drug selected from therapeutic agents for Alzheimer's diseases, antiparkinson agent, therapeutic agents for amyotrophic spinal lateral sclerosis, neurotrophic factor, antidepressant and therapeutic agents for schizophrenia. For the prophylaxis and/or treatment of circulatory system diseases such as arteriosclerosis, the compound can be used in combination with at least one kind of drug selected from hyperlipidemia treating agents, therapeutic agent for arteriosclerosis, diuretics, hypertension treating agents, cardiac failure treating agents, antiarrhythmic, anticoagulant, antithrombotic drug, antidiabetic drug, HDL increasing agents and unstable plaque stabilizing agents.

According to the present invention, a non-peptide compound of the present invention, particularly the cycloalkene compound, among others compound A, have been found as TLR signal selective inhibitory substances. It has been found, moreover, that the aforementioned various organ dysfunctions, infectious diseases such as severe sepsis, central nervous system diseases such as Alzheimer's disease, circulatory system diseases such as arteriosclerosis, bone and joint diseases such as arthritis rheumatoides, digestive system diseases such as ulcerative colitis, and the like can be improved by the action of these TLR signal inhibitory substances. Therefore, the present invention also provides an agent for the prophylaxis or treatment of organ dysfunction, infectious diseases such as severe sepsis, central nervous system diseases such as Alzheimer's disease, circulatory system diseases such as arteriosclerosis, bone and joint diseases such as arthritis rheumatoides, digestive system diseases such as ulcerative colitis, comprising a TLR signal inhibitory substance.

As the TLR signal inhibitory substance, for example, a peptide compound (e.g., anti TLR antibody, TLR inhibitory peptide, MIF (migration inhibitory factor) etc.) and the above-mentioned non-peptide compound can be mentioned. Of these, the cycloalkene compound or compound A is preferable. Accordingly, the TLR signal inhibitory substance of the present invention has low toxicity and preferably used for the prophylaxis and treatment of organ dysfunction and the like in mammal (e.g., rat, mouse, guinea pig, monkey, cattle, dog, pig, human etc.).

The TLR signal inhibitory substance can be processed by a method similar to the method for the above-mentioned non-peptide compound to give a preparation, which can be administered to mammals by the same administration route, dose and the like as mentioned above.

The agent for the prophylaxis or treatment of organ dysfunction and the like can be used in combination with a combination drug similar to any of the above-mentioned combination agents, and can be administered to mammals by the same administration route, dose and the like as mentioned above.

The present invention further provides a method for the prophylaxis or treatment of severe sepsis or the above-mentioned organ dysfunction by inhibiting TLR signals by inhibition with a TLR signal inhibitory substance, or other method (e.g., hypothermia therapy by placing in a low temperature chamber, or hypnotherapy by hypnotism or hypnotic administration, etc.).

### Examples

The present invention is further described in the following by referring to Reference Examples, Examples and Experimental Examples, which are not intended to restrict the invention.

The ¹H-NMR spectrum was determined by a VARIAN GEMINI 200 (200 MHz) spectrometer using tetramethylsilane as an internal standard and represented as the entire 8 values in ppm. The number in a bracket when a solvent mixture was employed is the volume ratio of each solvent, wherein % means % by weight unless otherwise specified. The ratio of the solvents in silica gel chromatography shows the volume ratio of the solvents to be admixed.

A more polar diastereomer means a diastereomer having a smaller Rf value, and a less polar diastereomer means a diastereomer having a larger Rf value, when determined by normal phase thin layer chromatography under the same conditions (e.g., using ethyl acetate/hexane etc. as an eluent) .

The melting point was measured using melting point measuring apparatus (manufactured by Yanako). The data of the powder X-ray diffraction was measured using RINT2500 (Rigaku Industrial Corporation) using Cu-K_{α1} ray as a ray source.

Each symbol in examples mean the following:
s: singlet d: doublet: t: triplet q: quartet
dd: double doublet tt: triple triplet m: multiplet
br: broad J: coupling constant

The following Reference Examples A can be produced according to Reference Examples of WO99/46242, Reference Example B can be produced according to Examples of WO99/46242, Reference Examples C can be produced according to Reference Examples of WO01/10826, and Reference Examples D can be produced according to Examples of WO01/10826.

### [Reference Examples A]

Reference Example A1 ethyl 2-sulfo-1-cyclohexene-1-carboxylate
Reference Example A2 ethyl 2-chlorosulfonyl-1-cyclohexene-1-carboxylate
Reference Example A3 ethyl 2-chlorosulfonyl-1-cyclopentene-1-carboxylate
Reference Example A4 ethyl 2-chlorosulfonyl-1-cycloheptene-1-carboxylate
Reference Example A5 sodium 6-[N-(4-chloro-2-fluorophenyl)-sulfamoyl]-1-cyclohexene-1-carboxylate
Reference Example A6 1-(3-fluoro-4-nitrophenyl)-1H-1,2,4-triazole
Reference Example A7 1-(4-amino-3-fluorophenyl)-1H-1,2,4-triazole
Reference Example A8 methyl 4-(benzyloxycarbonylamino)-3-chlorobenzoate
Reference Example A9 4-(benzyloxycarbonylamino)-3-chlorobenzoic acid
Reference Example A10 tert-butyl N-(4-benzyloxycarbonylamino-3-chlorobenzoyl)glycinate
Reference Example All tert-butyl N-(4-amino-3-chlorobenzoyl)-glycinate
Reference Example A12 6-[N-(2,4-difluorophenyl)sulfamoyl]-1-cyclohexene-1-carboxylic acid
Reference Example A13 ethyl 2-mercapto-5-phenyl-1-cyclohexene-1-carboxylate
Reference Example A14 ethyl 2-chlorosulfonyl-5-phenyl-1-cyclohexene-1-carboxylate
Reference Example A15 ethyl 5-tert-butyl-2-mercapto-1-cyclohexene-1-carboxylate
Reference Example A16 ethyl 5-tert-butyl-2-chlorosulfonyl-1-cyclohexene-1-carboxylate
Reference Example A17 ethyl 5,5-dimethyl-2-mercapto-1-cyclohexene-1-carboxylate
Reference Example A18 ethyl 2-chlorosulfonyl-5,5-dimethyl-1-cyclohexene-1-carboxylate

### [Reference Examples B]

Reference Example B1 ethyl 6-[N-(4-chloro-2-fluorophenyl)-sulfamoyl]-1-cyclohexene-1-carboxylate (compound 1)
Reference Example B2 ethyl 6-[N-(4-chloro-2-fluorophenyl)-N-methylsulfamoyl]-1-cyclohexene-1-carboxylate (compound 2)
Reference Example B3 ethyl 6-[N-(2,4-difluorophenyl)sulfamoyl]-1-cyclohexene-1-carboxylate (compound 3)
Reference Example B4 ethyl 6-[N-(2,6-diisopropylphenyl)-sulfamoyl]-1-cyclohexene-1-carboxylate (compound 4)
Reference Example B5 ethyl 6-[N-(4-nitrophenyl)sulfamoyl]-1-cyclohexene-1-carboxylate (compound 5)
Reference Example B6 ethyl 6-(N-phenylsulfamoyl)-1-cyclohexene-1-carboxylate (compound 6)
ethyl 2-(N-phenylsulfamoyl)-1-cyclohexene-1-carboxylate (compound 7)
Reference Example B7 ethyl 2-[N-(4-chloro-2-fluorophenyl)-sulfamoyl]-1-cyclohexene-1-carboxylate (compound 9)
Reference Example B8 2-(4-methoxyphenyl)-4,5,6,7-tetrahydro-1,2-benzisothiazol-3(2H)-one 1,1-dioxide (compound 67)
ethyl 2-[N-(4-methoxyphenyl)sulfamoyl]-1-cyclohexene-1-carboxylate (compound 8)
Reference Example B9 ethyl 6-[N-(2-fluorophenyl)sulfamoyl]-1-cyclohexene-1-carboxylate (compound 10)
Reference Example B10 ethyl 6-[N-(3-fluorophenyl)sulfamoyl]-1-cyclohexene-1-carboxylate (compound 11)
Reference Example B11 2-(4-fluorophenyl)-4,5,6,7-tetrahydro-1,2-benzisothiazol-3(2H)-one 1,1-dioxide (compound 68)
ethyl 6-[N-(4-fluorophenyl)sulfamoyl]-1-cyclohexene-1-carboxylate (compound 12)
ethyl 2-[N-(4-fluorophenyl)sulfamoyl]-1-cyclohexene-1-carboxylate (compound 18)
Reference Example B12 ethyl 6-[N-(2,6-difluorophenyl)-sulfamoyl]-1-cyclohexene-1-carboxylate (compound 13)
Reference Example B13 ethyl 6-[N-(2,3-difluorophenyl)-sulfamoyl]-1-cyclohexene-1-carboxylate (compound 14)
Reference Example B14 ethyl 6-[N-(2,5-difluorophenyl)-sulfamoyl]-1-cyclohexene-1-carboxylate (compound 15)
Reference Example B15 ethyl 6-[N-(3,4-difluorophenyl)-sulfamoyl]-1-cyclohexene-1-carboxylate (compound 16)
Reference Example B16 ethyl 6-[N-(3,5-difluorophenyl)-sulfamoyl]-1-cyclohexene-1-carboxylate (compound 17)
Reference Example B17 1-ethyl 6-[N-(2,4-difluorophenyl)-sulfamoyl]-1-cyclohexene-1-carboxylate (compound 19)
d-ethyl 6-[N-(2,4-difluorophenyl)sulfamoyl]-1-cyclohexene-1-carboxylate (compound 20)
Reference Example B18 ethyl 6-[N-(2-ethoxycarbonylphenyl)-sulfamoyl]-1-cyclohexene-1-carboxylate (compound 21)
Reference Example B19 methyl 6-[N-(2,4-difluorophenyl)-sulfamoyl]-1-cyclohexene-1-carboxylate (compound 22)
Reference Example B20 propyl 6-[N-(2,4-difluorophenyl)-sulfamoyl]-1-cyclohexene-1-carboxylate (compound 23)
Reference Example B21 methyl 6-[N-(4-chloro-2-fluorophenyl)-sulfamoyl]-1-cyclohexene-1-carboxylate (compound 24)
Reference Example B22 isopropyl 6-[N-(2,4-difluorophenyl)-sulfamoyl]-1-cyclohexene-1-carboxylate (compound 25)
Reference Example B23 ethyl 6-[N-(2-methoxycarbonylphenyl)-sulfamoyl]-1-cyclohexene-1-carboxylate (compound 26)
Reference Example B24 ethyl 6-[N-(2-fluoro-4-methylphenyl)-sulfamoyl]-1-cyclohexene-1-carboxylate (compound 27)
Reference Example B25 ethyl 6-[N-(2-chlorophenyl)sulfamoyl]-1-cyclohexene-1-carboxylate (compound 28)
Reference Example B26 ethyl 6-[N-(2-chloro-4-fluorophenyl)-sulfamoyl]-1-cyclohexene-1-carboxylate (compound 29)
Reference Example B27 ethyl 6-[N-(4-chlorophenyl)sulfamoyl]-1-cyclohexene-1-carboxylate (compound 30)
Reference Example B28 ethyl 6-[N-(2,3,4-trifluorophenyl)-sulfamoyl]-1-cyclohexene-1-carboxylate (compound 31)
Reference Example B29 isobutyl 6-[N-(2,4-difluorophenyl)-sulfamoyl]-1-cyclohexene-1-carboxylate (compound 32)
Reference Example B30 butyl 6-[N-(2,4-difluorophenyl)-sulfamoyl]-1-cyclohexene-1-carboxylate (compound 33)
Reference Example B31 ethyl 6-[N-(4-bromo-2-fluorophenyl)-sulfamoyl]-1-cyclohexene-1-carboxylate (compound 34)
Reference Example B32 ethyl 6-[N-(2,4-dichlorophenyl)-sulfamoyl]-1-cyclohexene-1-carboxylate (compound 35)
Reference Example B33 ethyl 6-[N-(2-acetoxyphenyl)sulfamoyl]-1-cyclohexene-1-carboxylate (compound 36)
Reference Example B34 ethyl 6-[N-(3-chlorophenyl)sulfamoyl]-1-cyclohexene-1-carboxylate (compound 37)
Reference Example B35 ethyl 6-[N-(2,3-dichlorophenyl)-sulfamoyl]-1-cyclohexene-1-carboxylate (compound 38)
Reference Example B36 ethyl 6-[N-(2-ethylphenyl)sulfamoyl]-1-cyclohexene-1-carboxylate (compound 39)
Reference Example B37 ethyl 6-[N-[4-(2H-1,2,3-triazol-2-yl)phenyl]sulfamoyl]-1-cyclohexene-1-carboxylate (compound 40)
Reference Example B38 ethyl 6-[N-(2,5-dichlorophenyl)-sulfamoyl]-1-cyclohexene-1-carboxylate (compound 41)
Reference Example B39 ethyl 6-[N-(2-trifluoromethoxyphenyl)-sulfamoyl]-1-cyclohexene-1-carboxylate (compound 42)
Reference Example B40 ethyl 6-[N-(2,4,5-trifluorophenyl)-sulfamoyl]-1-cyclohexene-1-carboxylate (compound 43)
Reference Example B41 ethyl 6-[N-[4-(2H-tetrazol-2-yl)phenyl]sulfamoyl]-1-cyclohexene-1-carboxylate (compound 44)
Reference Example B42 ethyl 6-[N-(2-chloro-4-methylphenyl)-sulfamoyl]-1-cyclohexene-1-carboxylate (compound 45)
Reference Example B43 ethyl 6-[N-(4-fluoro-2-methylphenyl)-sulfamoyl]-1-cyclohexene-1-carboxylate (compound 46)
Reference Example B44 ethyl 6-[N-(2,6-dichlorophenyl)-sulfamoyl]-1-cyclohexene-1-carboxylate (compound 47)
Reference Example B45 ethyl 6-[N-[4-(1H-tetrazol-1-yl)phenyl]sulfamoyl]-1-cyclohexene-1-carboxylate (compound 48)
Reference Example B46 ethyl 6-[N-(4-(1H-1,2,3-triazol-1-yl)phenyl]sulfamoyl]-1-cyclohexene-1-carboxylate (compound 49)
Reference Example B47 ethyl 6-[N-(2-trifluoromethylphenyl)-sulfamoyl]-1-cyclohexene-1-carboxylate (compound 50)
Reference Example B48 ethyl 6-[N-(4-methoxycarbonylphenyl)-sulfamoyl]-1-cyclohexene-1-carboxylate (compound 51)
Reference Example B49 benzyl 6-[N-(2,4-difluorophenyl)-sulfamoyl]-1-cyclohexene-1-carboxylate(compound 52)
Reference Example B50 ethyl 6-[N-[4-[2,3-bis(tert-butoxycarbonyl)guanidinomethyl]phenyl]sulfamoyl]-1-cyclohexene-1-carboxylate (compound 53)
Reference Example B51 ethyl 6-[N-(2-chloro-4-methoxycarbonylphenyl)sulfamoyl]-1-cyclohexene-1-carboxylate (compound 54)
Reference Example B52 ethyl 6-[N-(2-chloro-4-cyanophenyl)-sulfamoyl]-1-cyclohexene-1-carboxylate (compound 55)
Reference Example B53 2-hydroxyethyl 6-[N-(2,4-difluorophenyl)sulfamoyl]-1-cyclohexene-1-carboxylate (compound 56)
Reference Example B54 ethyl 6-[N-[2-fluoro-4-(1H-1,2,4-triazol-1-yl)phenyl]sulfamoyl]-1-cyclohexene-1-carboxylate (compound 57)
Reference Example B55 ethyl 2-[N-(2,4-difluorophenyl)-sulfamoyl]-1-cyclopentene-1-carboxylate (compound 66)
ethyl 5-[N-(2,4-difluorophenyl)sulfamoyl]-1-cyclopentene-1-carboxylate (compound 58)
Reference Example B56 tert-butyl [6-[N-(2,4-difluorophenyl)-sulfamoyl]-1-cyclohexen-1-yl]carbonyloxyacetate (compound 59)
Reference Example B57 [6-(N-(2,4-difluorophenyl)sulfamoyl]-1-cyclohexen-1-yl]carbonyloxyacetic acid (compound 60)
Reference Example B58 ethyl 7-[N-(2,4-difluorophenyl)-sulfamoyl]-1-cycloheptene-1-carboxylate (compound 61)
Reference Example B59 ethyl 6-[N-[2-chloro-4-(N-tert-butoxycarbonylmethylcarbamoyl)phenyl]sulfamoyl]-1-cyclohexene-1-carboxylate (compound 62)
Reference Example B60 ethyl 6-[N-[2-chloro-4-(N-ethoxycarbonylmethylcarbamoyl)phenyl]sulfamoyl]-1-cyclohexene-1-carboxylate (compound 63)
Reference Example B61 ethyl 5-[N-(2-chloro-4-fluorophenyl)-sulfamoyl]-1-cyclopentene-1-carboxylate (compound 64)
Reference Example B62 2-[4-(2,2,3,3,3-pentafluoropropoxy)-phenyl]-4,5,6,7-tetrahydro-1,2-benzisothiazol-3(2H)-one 1,1-dioxide (compound 69)
Reference Example B63 ethyl 7-[N-(2-chloro-4-fluoraphenyl)-sulfamoyl]-1-cycloheptene-1-carboxylate (compound 65)
Reference Example B64 2-(2,4-difluorophenyl)-5,6,7,7a-tetrahydro-1,2-benzisothiazol-3(2H)-one 1,1-dioxide (compound 70)
Reference Example B65 ethyl 6-[N-(2-chloro-4-fluorophenyl)-sulfamoyl]-1-cyclohexene-1-carboxylate (compound 29)
Reference Example B66 ethyl (6S)-6-[(2-chloro-4-fluoroanilino)sulfonyl]-1-cyclohexene-1-carboxylate (1-ethyl 6-[N-(2-chloro-4-fluorophenyl)sulfamoyl]-1-cyclohexene-1-carboxylate) (compound 71)
ethyl (6R)-6-[(2-chloro-4-fluoroanilino)sulfonyl]-1-cyclohexene-1-carboxylate (d-ethyl 6-[N-(2-chloro-4-fluorophenyl)sulfamoyl]-1-cyclohexene-1-carboxylate) (compound 72)
Reference Example B67 ethyl 6-[N-(2-bromo-4-fluorophenyl)-sulfamoyl]-1-cyclohexene-1-carboxylate (compound 73)
Reference Example B68 ethyl 6-[N-(4-bromo-2-chlorophenyl)-sulfamoyl]-1-cyclohexene-1-carboxylate (compound 74)
Reference Example B69 more polar diastereomer (compound 75) and less polar diastereomer (compound 76) of ethyl 6-[N-(2,4-difluorophenyl)sulfamoyl]-3-phenyl-1-cyclohexene-1-carboxylate
Reference Example B70 more polar diastereomer (compound 77) and less polar diastereomer (compound 78) of ethyl 6-[N-(2-chloro-4-fluorophenyl)sulfamoyl]-3-phenyl-1-cyclohexene-1-carboxylate
Reference Example B71 more polar diastereomer (compound 79) and less polar diastereomer (compound 80) of ethyl 6-[N-(2,4-difluorophenyl)sulfamoyl]-3-tert-butyl-1-cyclohexene-1-carboxylate
Reference Example B72 more polar diastereomer (compound 81) and less polar diastereomer (compound 82) of ethyl 6-[N-(2-chloro-4-fluorophenyl)sulfamoyl]-3-tert-butyl-1-cyclohexene-1-carboxylate
Reference Example B73 ethyl 6-[N-(2,4-difluorophenyl)-sulfamoyl]-3,3-dimethyl-1-cyclohexene-1-carboxylate (compound 83)
Reference Example B74 ethyl 6-[N-(2-chloro-4-fluorophenyl)-sulfamoyl]-3,3-dimethyl-1-cyclohexene-1-carboxylate (compound 84)
Reference Example B75 ethyl 3-bromo-6-[N-(2,4-difluorophenyl)-sulfamoyl]-1-cyclohexene-1-carboxylate (compound 85)

The chemical structures of compounds 1 - 85 are shown in Tables 1 - 12.

**Table 1**

| | | | | |
|---|---|---|---|---|
| 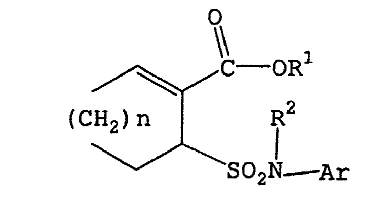 | | | | |

| Compound No. | R¹ | R² | Ar | n |
|---|---|---|---|---|
| 1 | C₂H₅ | H | 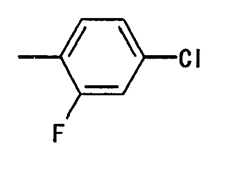 | 2 |
| 2 | C₂H₅ | CH₃ | 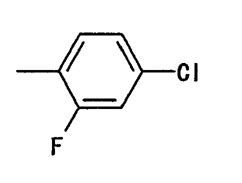 | 2 |
| 3 | C₂H₅ | H | 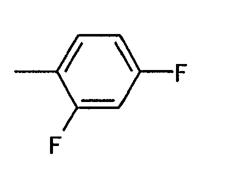 | 2 |
| 4 | C₂H₅ | H | 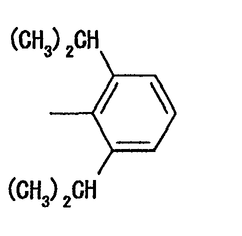 | 2 |
| 5 | C₂H₅ | H | 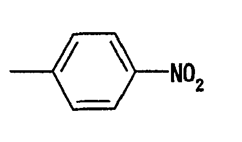 | 2 |
| 6 | C₂H₅ | H | 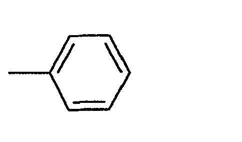 | 2 |
| 10 | C₂H₅ | H | 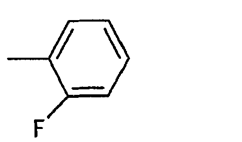 | 2 |

**Table 2**

| | | | | |
|---|---|---|---|---|
| 11 | C₂H₅ | H | 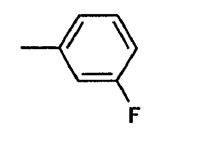 | 2 |
| 12 | C₂H₅ | H | 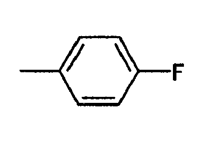 | 2 |
| 13 | C₂H₅ | H | 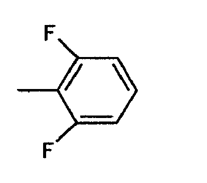 | 2 |
| 14 | C₂H₅ | H | 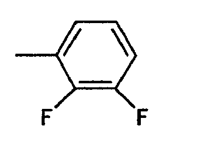 | 2 |
| 15 | C₂H₅ | H | 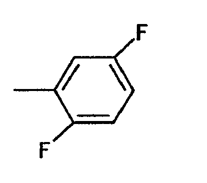 | 2 |
| 16 | C₂H₅ | H | 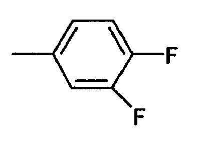 | 2 |
| 17 | C₂H₅ | H | 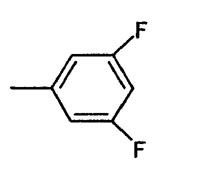 | 2 |
| 19 (1-form) | C₂H₅ | H | 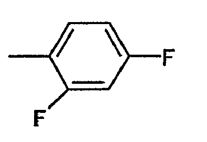 | 2 |
| 20 (d-form) | C₂H₅ | H | 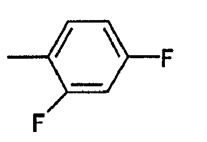 | 2 |

**Table 3**

| | | | | |
|---|---|---|---|---|
| 21 | C₂H₅ | H | 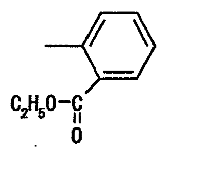 | 2 |
| 22 | CH₃ | H | 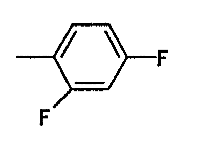 | 2 |
| 23 | (CH₂)₂CH₃ | H | 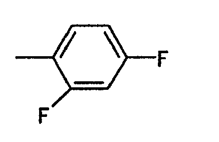 | 2 |
| 24 | CH₃ | H | 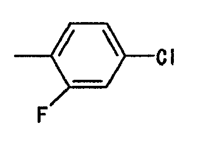 | 2 |
| 25 | CH(CH₃)₂ | H | 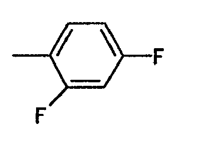 | 2 |
| 26 | C₂H₅ | H | 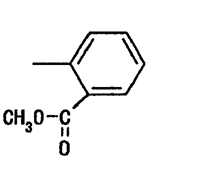 | 2 |
| 27 | C₂H₅ | H | 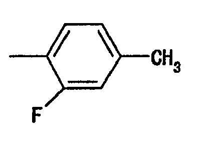 | 2 |
| 28 | C₂H₅ | H | 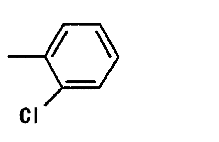 | 2 |
| 29 | C₂H₅ | H | 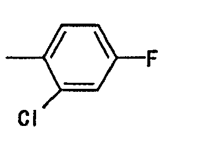 | 2 |
| 30 | C₂H₅ | H | 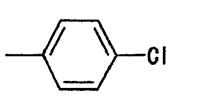 | 2 |

**Table 4**

| | | | | |
|---|---|---|---|---|
| 31 | C₂H₅ | H | 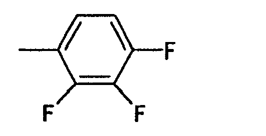 | 2 |
| 32 | CH₂CH (CH₃)₂ | H | 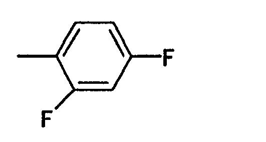 | 2 |
| 33 | (CH₂)₃CH₃ | H | 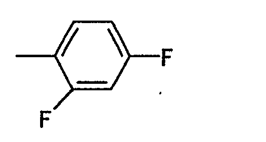 | 2 |
| 34 | C₂H₅ | H | 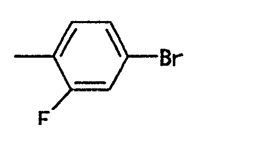 | 2 |
| 35 | C₂H₅ | H | 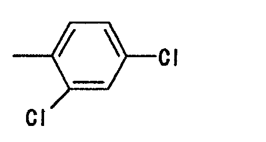 | 2 |
| 36 | C₂H₅ | H | 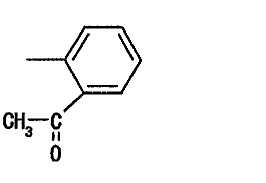 | 2 |
| 37 | C₂H₅ | H | 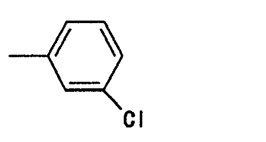 | 2 |
| 38 | C₂H₅ | H | 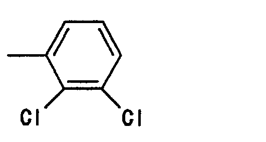 | 2 |
| 39 | C₂H₅ | H | 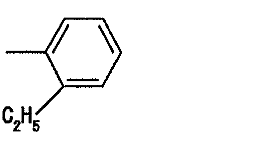 | 2 |
| 40 | C₂H₅ | H | 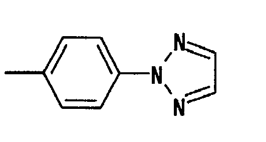 | 2 |

**Table 5**

| | | | | |
|---|---|---|---|---|
| 41 | C₂H₅ | H | 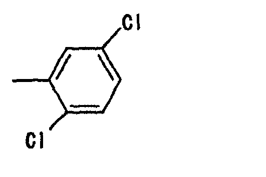 | 2 |
| 42 | C₂H₅ | H | 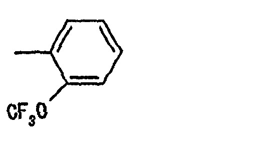 | 2 |
| 43 | C₂H₅ | H | 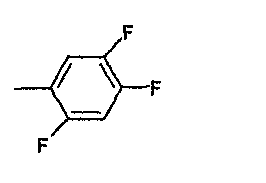 | 2 |
| 44 | C₂H₅ | H | 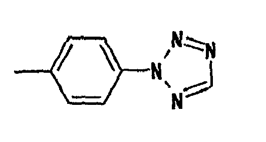 | 2 |
| 45 | C₂H₅ | H | 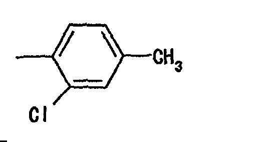 | 2 |
| 46 | C₂H₅ | H | 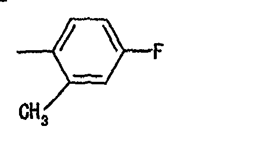 | 2 |
| 47 | C₂H₅ | H | 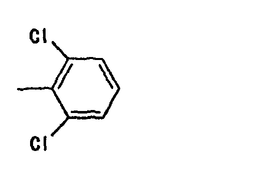 | 2 |
| 48 | C₂H₅ | H | 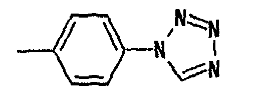 | 2 |
| 49 | C₂H₅ | H | 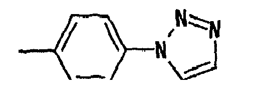 | 2 |
| 50 | C₂H₅ | H | 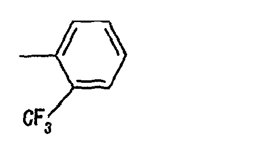 | 2 |

**Table 6**

| | | | | |
|---|---|---|---|---|
| 51 | C₂H₅ | H | 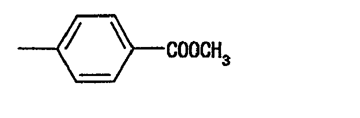 | 2 |
| 52 | 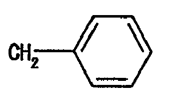 | H | 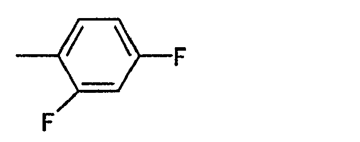 | 2 |
| 53 | C₂H₅ | H | 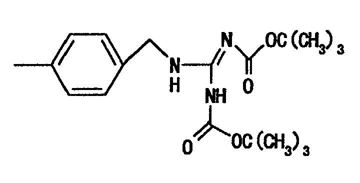 | 2 |
| 54 | C₂H₅ | H | 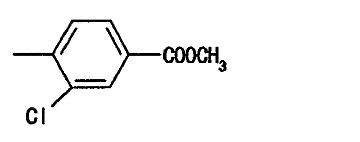 | 2 |
| 55 | C₂H₅ | H | 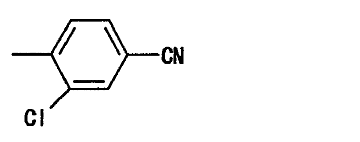 | 2 |
| 56 | (CH₂) ₂OH | H | 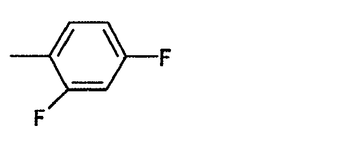 | 2 |
| 57 | C₂H₅ | H | 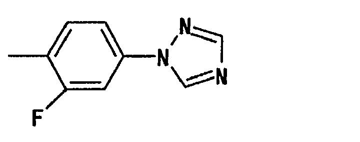 | 2 |
| 58 | C₂H₅ | H | 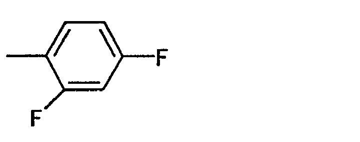 | 1 |
| 59 | CH₂COOC (CH₃)₃ | H | 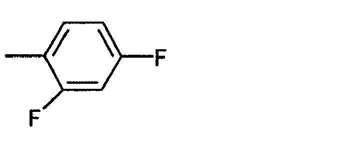 | 2 |
| 60 | CH₂COOH | H | 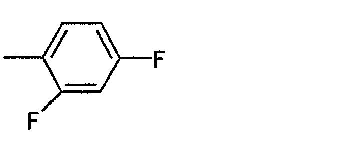 | 2 |

**Table 7**

| | | | | |
|---|---|---|---|---|
| 61 | C₂H₅ | H | 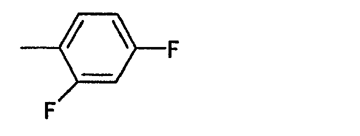 | 3 |
| 62 | C₂H₅ | H | 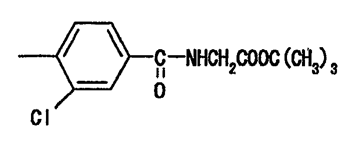 | 2 |
| 63 | C₂H₅ | H | 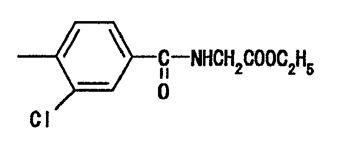 | 2 |
| 64 | C₂H₅ | H | 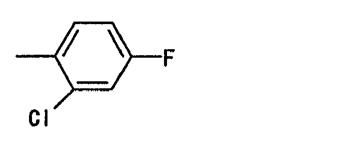 | 1 |
| 65 | C₂H₅ | H | 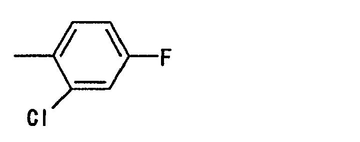 | 3 |
| 71 (S-form) | C₂H₅ | H | 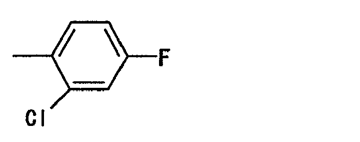 | 2 |
| 72 (R-form) | C₂H₅ | H | 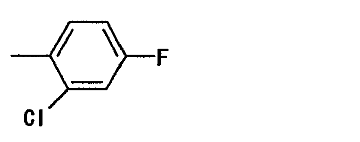 | 2 |
| 73 | C₂H₅ | H | 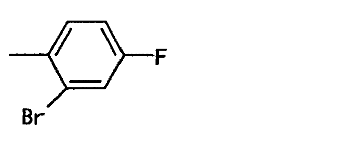 | 2 |
| 74 | C₂H₅ | H | 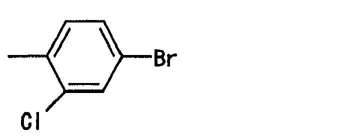 | 2 |

**Table 8**

| | | | |
|---|---|---|---|
| 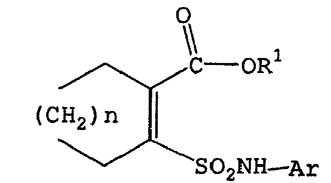 | | | |

| Compound No. | R¹ | Ar | n |
|---|---|---|---|
| 7 | C₂H₅ | 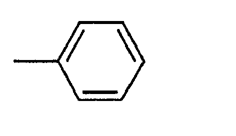 | 2 |
| 8 | C₂H₅ | 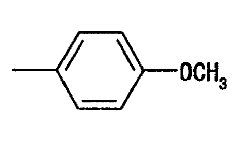 | 2 |
| 9 | C₂H₅ | 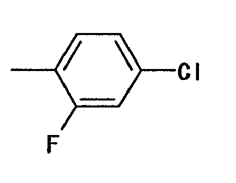 | 2 |
| 18 | C₂H₅ | 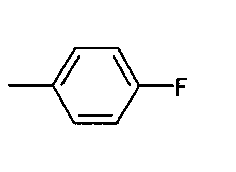 | 2 |
| 66 | C₂H₅ | 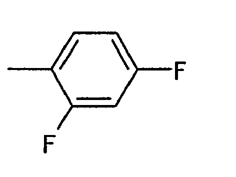 | 1 |

**Table 9**

| | | |
|---|---|---|
| 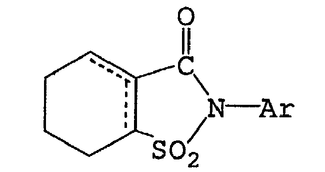 | | |

| Compound No. | 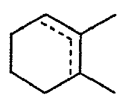 | Ar |
|---|---|---|
| 67 | 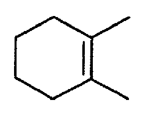 | 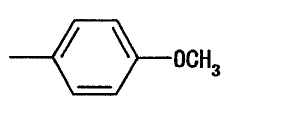 |
| 68 | 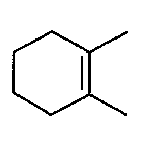 | 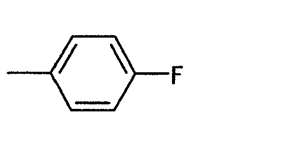 |
| 69 | 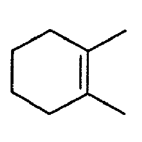 | 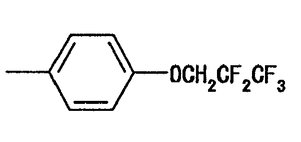 |
| 70 | 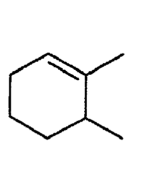 | 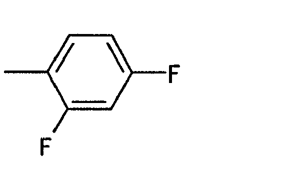 |

**Table 10**

| | | | | |
|---|---|---|---|---|
| 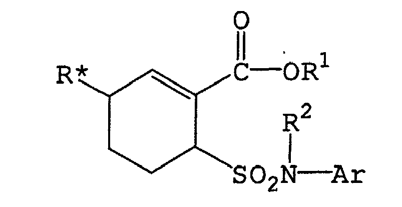 | | | | |

| Compound No. | R¹ | R² | R* | Ar |
|---|---|---|---|---|
| 75 (more polar diastereomer) | C₂H₅ | H | 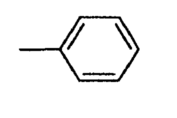 | 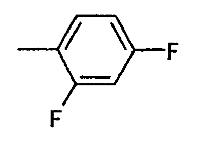 |
| 76 (less polar diastereomer) | C₂H₅ | H | 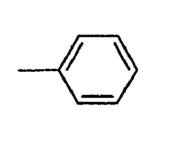 | 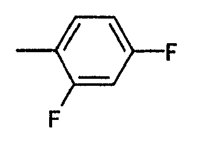 |
| 77 (more polar diastereomer) | C₂H₅ | H | 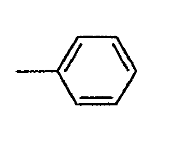 | 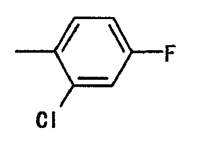 |
| 78 (less polar diastereomer) | C₂H₅ | H | 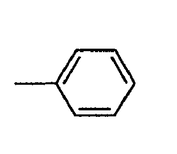 | 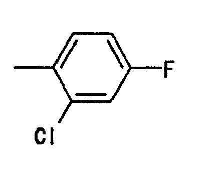 |
| 79 (more polar diastereomer) | C₂H₅ | H | C(CH₃)₃ | 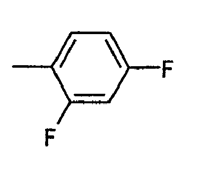 |
| 80 (less polar diastereomer) | C₂H₅ | H | C (CH₃)₃ | 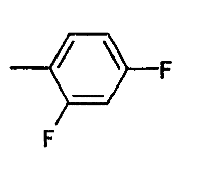 |
| 81 (more polar diastereomer) | C₂H₅ | H | C (CH₃)₃ | 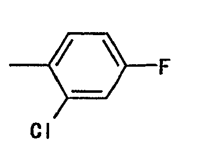 |

**Table 11**

| | | | | |
|---|---|---|---|---|
| 82 (less polar diastereomer) | C₂H₅ | H | C(CH₃)₃ | 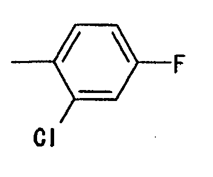 |
| 85 | C₂H₅ | H | Br | 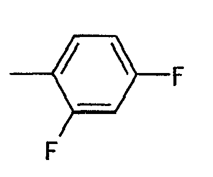 |

**Table 12**

| | |
|---|---|
| 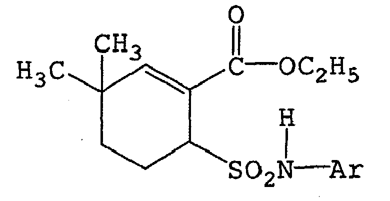 | |

| Compound No. | Ar |
|---|---|
| 83 | 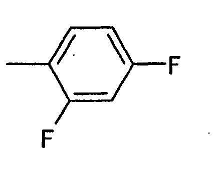 |
| 84 | 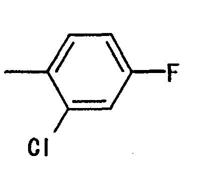 |

### [Reference Examples C]

Reference Example C1 ethyl 6-(benzylsulfanyl)-1-cyclohexene-1-carboxylate
Reference Example C2 ethyl 6-[(4-methoxybenzyl)sulfanyl]-1-cyclohexene-1-carboxylate
Reference Example C3 ethyl 6-[(2,4-difluorobenzyl)sulfanyl]-1-cyclohexene-1-carboxylate
Reference Example C4 ethyl 6-[(2-chloro-4-fluorobenzyl)sulfanyl]-1-cyclohexene-1-carboxylate
Reference Example C5 ethyl 5-hydroxy-3,6-dihydro-2H-pyran-4-carboxylate
Reference Example C6 ethyl 5-sulfanyl-3,6-dihydro-2H-pyran-4-carboxylate
Reference Example C7 4-(ethoxycarbonyl)-5,6-dihydro-2H-pyran-3-sulfonic acid
Reference Example C8 ethyl 5-(chlorosulfonyl)-3,6-dihydro-2H-pyran-4-carboxylate

### [Reference Examples D]

Reference Example D1 ethyl 6-(benzylsulfonyl)-1-cyclohexene-1-carboxylate (compound 1')
Reference Example D2 ethyl 6-[(4-methoxybenzyl)sulfonyl]-1-cyclohexene-1-carboxylate (compound 2')
Reference Example D3 ethyl 6-[(2,4-difluorobenzyl)sulfonyl]-1-cyclohexene-1-carboxylate (compound 3')
Reference Example D4 ethyl 6-[(2-chloro-4-fluorobenzyl)sulfonyl]-1-cyclohexene-1-carboxylate (compound 4')
Reference Example D5 ethyl (-)-6-[(2-chloro-4-fluorobenzyl)sulfonyl]-1-cyclohexene-1-carboxylate (compound 5')
ethyl (+)-6-[(2-chloro-4-fluorobenzyl)sulfonyl]-1-cyclohexene-1-carboxylate (compound 6')
Reference Example D6 ethyl 3-[(2,4-difluorophenyl)sulfamoyl]-3,6-dihydro-2H-pyran-4-carboxylate (compound 7')
Reference Example D7 ethyl 3-[(2-chloro-4-fluorophenyl)sulfamoyl]-3,6-dihydro-2H-pyran-4-carboxylate (compound 8')

The chemical structures of the compounds 1' - 8' are shown in Table 13 and Table 14.

**Table 13**

| | |
|---|---|
| 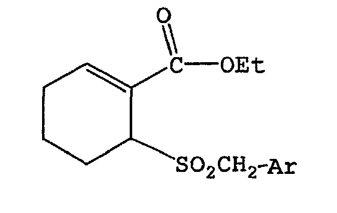 | |

| Compound No. | Ar |
|---|---|
| 1' | 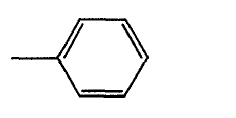 |
| 2' | 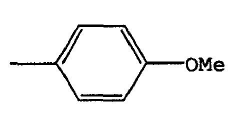 |
| 3' | 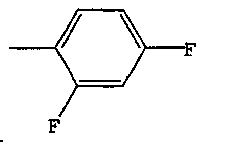 |
| 4' | 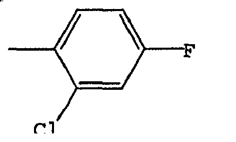 |
| 5' (-)-form | 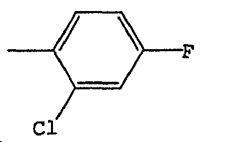 |
| 6' (+) -form | 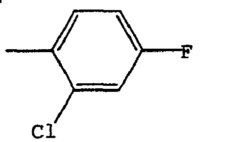 |

**Table 14**

| | |
|---|---|
| 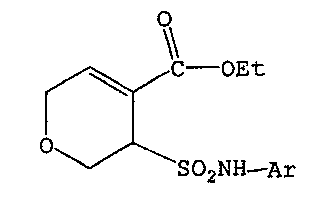 | |

| Compound No. | Ar |
|---|---|
| 7' | 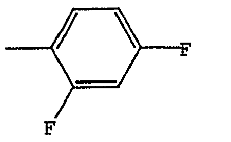 |
| 8' | 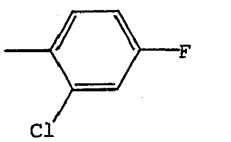 |

### Example (Formulation Example) 1

| | | | |
|---|---|---|---|
| (1) | compound 72 of Reference Example B66 | 10 | mg |
| (2) | lactose | 60 | mg |
| (3) | cornstarch | 35 | mg |
| (4) | gelatin | 3 | mg |
| (5) | magnesium stearate | 2 | mg |

A mixture of compound 72 (10 mg) of Reference Example B66, lactose (60 mg) and cornstarch (35 mg) is granulated by the use of a 10% aqueous gelatin solution (0.03 ml) (3 mg as gelatin) and passing through a 1 mm mesh sieve, dried at 40°C and again passed through a sieve. The granules thus obtained are mixed with magnesium stearate (2 mg) and compressed. The obtained core tablets are sugar coated by applying an aqueous suspension of sucrose, titanium dioxide, talc and gum arabic and glazed with beeswax to give coated tablets.

### Example (Formulation Example) 2

| | | | |
|---|---|---|---|
| (1) | compound 72 of Reference Example B66 | 10 | mg |
| (2) | lactose | 70 97 | mg |
| (3) | cornstarch | 50 | mg |
| (4) | soluble starch | 7 | mg |
| (5) | magnesium stearate | 3 | mg |

The compound 72 (10 mg) of Reference Example B66 and magnesium stearate (3 mg) are granulated using an aqueous solution (0.07 ml) of soluble starch (7 mg as soluble starch), dried and mixed with lactose (70 mg) and cornstarch (50 mg). The mixture is compressed to give tablets.

### Example (Formulation Example) 3

| | | | |
|---|---|---|---|
| (1) | compound 29 of Reference Example B65 | 10 | mg |
| (2) | lactose | 60 | mg |
| (3) | cornstarch | 35 | mg |
| (4) | gelatin | 3 | mg |
| (5) | magnesium stearate | 2 | mg |

A mixture of compound 29 (10 mg) of Reference Example B65, lactose (60 mg) and cornstarch (35 mg) is granulated by the use of a 10% aqueous gelatin solution (0.03 ml) (3 mg as gelatin) and passing through a 1 mm mesh sieve, dried at 40°C and again passed through a sieve. The granules thus obtained are mixed with magnesium stearate (2 mg) and compressed. The obtained core tablets are sugar coated by applying an aqueous suspension of sucrose, titanium dioxide, talc and gum arabic and glazed with beeswax to give coated tablets.

### Example (Formulation Example) 4

| | | | |
|---|---|---|---|
| (1) | compound 29 of Reference Example B65 | 10 | mg |
| (2) | lactose | 70 | mg |
| (3) | cornstarch | 50 | mg |
| (4) | soluble starch | 7 | mg |
| (5) | magnesium stearate | 3 | mg |

The compound 29 (10 mg) of Reference Example B65 and magnesium stearate (3 mg) are granulated using an aqueous solution (0.07 ml) of soluble starch (7 mg as soluble starch), dried and mixed with lactose (70 mg) and cornstarch (50 mg). The mixture is compressed to give tablets.

### Experimental Example 1: Effect by administration after LPS inoculation in endotoxin shock model (1)

To investigate the treatment effect during the process toward an advanced degree after the onset of sepsis, the effect of a test substance (Reference Example B1) by administration after LPS inoculation in an endotoxin shock model was investigated. To be specific, LPS (10 mg/kg) was intraperitoneally inoculated to female BALB/c mice (7 weeks old) (n=7) and survival of the mice was observed for one week. The test substance was suspended in 0.5% aqueous methyl cellulose solution, and intraperitoneally inoculated (30 mg/kg) at 1 hr before, immediately after, 30 min after and 1 hr after LPS inoculation. The solvent was given to the control group at 1 hr before LPS inoculation. The results are shown in Fig. 1.

The test substance was found to show a clear life-saving effect even by the administration at 1 hr after LPS inoculation. In other words, the test substance can be expected to show clear life-saving effect by the administration in the state of severe sepsis (see Reference Experimental Example 1).

### Reference Experimental Example 1: Measurement of white blood count and platelet count

LPS (10 mg/kg) was intraperitoneally administered to mice and blood was drawn after a given time. The blood (about 45 µL) taken with a calibrated pipette and 1.5% EDTA-2Na (4.5 µL) were mixed, white blood count and platelet count were measured with an automatic cell counter (F-800, Sysmex). The results are shown in Figs. 2 and 3. The white blood count (Fig. 2) and platelet count (Fig. 3) decreased in 30 min to 1 hr after the LPS inoculation, thereby suggesting the possibility of induction of a disease state of severe sepsis patients in 30 - 1 hr from the LPS inoculation.

### Experimental Example 2: Effect by administration after LPS inoculation in endotoxin shock model (2)

To investigate the treatment effect during the process toward an advanced degree after the onset of sepsis, the effect of a test substance (Reference Example B66) by administration after LPS inoculation in an endotoxin shock model was investigated. To be specific, LPS (4 mg/kg) was intraperitoneally inoculated to female BALB/c mice (7 weeks old) (n=10) and survival of the mice was observed for 5 days. The test substance was prepared into an emulsified liquid of a soybean oil, and intravenously administered (10 mg/kg) immediately after, 1, 2, 4 and 6 hr after LPS inoculation. An emulsified liquid without a drug was intravenously administered to the control group immediately after LPS inoculation. The results are shown in Fig. 4.

The test substance was found to show a clear life-saving effect even by the administration at 4 hr after LPS inoculation. In other words, the test substance can be expected to show a clear life-saving effect by the administration in the state of severe sepsis (see Reference Experimental Example 1).

### Experimental Example 3: Galactosamine loaded mouse Escherichia coli inoculated lethal model

Since sepsis is a systemic inflammatory response caused by infection, the effect of a test substance (Reference Example B26) by administration after bacterial inoculation in infection model was studied. That is, *E*. *coli* 0111 (5.9 x 10⁵ CFU) was intraperitoneally inoculated to female BALB/c mice (7 weeks old) (n=8) together with galactosamine (1 g/kg), which is a hepatopathy inducing substance, and survival of the mice was observed for 6 days. The test substance was suspended in 0.5% aqueous methyl cellulose solution, and orally administered (30 mg/kg) immediately, 30 min and 1, 2 and 4 hr after the bacterial inoculation. The solvent was orally administered to the control group immediately after the bacterial inoculation. The results are shown in Fig. 5.

The test substance was found to show a clear life-saving effect even by the administration at 1 hr after bacterial inoculation in mice having hepatopathy induced by inoculation of galactosamine. In other words, the test substance can be expected to show effect of improving the state of severe sepsis complicated with organ dysfunction.

### Experimental Example 4: Effect of TLR selective agonist on NO production

Using mouse macrophage cell line RAW264.7, the suppressive effect of the test substance on cytokine production by a TLR selective agonist was investigated. On the previous day of the experiment, the cells were suspended in RPMI-1640 medium supplemented with 10% inactivated fetal calf serum to 5 × 10⁵ cells/mL and inoculated to a 96-well microplate at 0.2 mL/well. After incubation at 37°C under an atmosphere of 5% CO₂/95% air overnight, the medium was changed to an RPMI-1640 medium supplemented with 1% inactivated fetal calf serum, and a test substance, various TLR agonists and interferon-γ (final concentration 0.1 ng/mL) were added. After further incubation overnight, the concentration of nitrite ion (stable metabolite of NO) in the culture supernatant was quantified by fluorescence method using 2,3-dimaminonaphthalene. As the TLR4 selective agonist, LPS was added, as the TLR2 selective agonist, peptide glycan (PGN) was added, and as the TLR9 selective agonist, CpG oligoDNA (CPG) was added, to the final concentrations of 5 ng/mL, 1 µg/mL and 75 nmol/L, respectively. In addition, the test substance was dissolved at 10 mM in N,N-dimethylformamide, diluted with an RPMI-1640 medium supplemented with 1% inactivated fetal calf serum to the concentration of 0.1 mM, then diluted with a medium to a 10-fold concentration of the final concentration and 1/10 was added. The results are shown in Table 15.

**[Table 15]**

| Reference Example No. | IC₅₀ (nmol/L) | | |
|---|---|---|---|
| | LPS | PGN | CPG |
| B66 | 10 | >1000 | >1000 |
| D4 | 11 | >1000 | >1000 |
| D7 | 18 | 530 | >1000 |

The test substance suppressed NO production due to LPS, which is a TLR4 agonist, at IC₅₀ in the order of 10⁻⁸ mol/L, but scarcely suppressed NO production due to PGN and CPG, that are TLR2 and TLR9 agonists, respectively. Therefore, the test substance can be said a TLR4 signal selective inhibitor.

### Experimental Example 5: Effect on cytokine production by β-amyloid peptide

Using mouse macrophage cell line RAW264.7, a suppressive effect of test substances (Reference Example B1, Reference Example B26 and Reference Example B3) on cytokine production by β-amyloid peptide (1-40) considered to be involved in the onset of Alzheimer's disease was investigated. On the previous day of the experiment, the cells were suspended in RPMI-1640 medium supplemented with 10% inactivated fetal calf serum to 5 × 10⁵ cells/mL and inoculated to a 96-well microplate at 0.2 mL/well. After incubation at 37°C under 5% CO₂/95% air overnight, the medium was changed to an RPMI-1640 medium supplemented with 1% inactivated fetal calf serum, a test substance and β-amyloid peptide (1-40) (final concentration 10 µM) and interferon-γ (final concentration 10 U/mL) were added. After further incubation overnight, the concentrations of tumor necrosis factor-α (TNF-α) and IL-6 in culture supernatants were measured using an enzyme immunoassay kit manufactured by Amersham. The test substance was dissolved at 10 mM in N,N-dimethylformamide and diluted with an RPMI-1640 medium supplemented with 1% inactivated fetal calf serum to the concentration of 0.1 mM, then diluted with a medium to a 10-fold concentration of the final concentration and 1/10 was added. The suppressive rate is shown in Table 16.

**[Table 16]**

| Reference Example No. | Concentration (µM) | Suppressive rate (%) | |
|---|---|---|---|
| | | TNF-α | IL-6 |
| B1 | 1 | 77 | 80 |
| B1 | 10 | 99 | 100 |
| B26 | 1 | 88 | 93 |
| B26 | 10 | 100 | 96 |
| B3 | 1 | 87 | 100 |
| B3 | 10 | 97 | 100 |

The test substance markedly suppressed cytokine production from macrophage cells due to β-amyloid peptide stimulation.

### Experimental Example 6: Effect in rat balloon injury model

Using male Sprague-Dawley rats, the neck was incised under sodium pentobarbital (45 mg/kg ip) anesthesia to expose left carotid artery. A Forgaty catheter (2F, manufactured by Baxtar) was inserted from the femoral artery to a bifurcation area of internal and external carotid arteries of left carotid artery, a balloon was blown and abraded to the aortic arch bifurcation. This operation was repeated three times to injure the common carotid artery. After the injury, the catheter was extracted, the incision was sutured, and the rats were individually bred in ordinary breeding cages. After 2 weeks from the injury, the rats were exsanguinated under anesthesia, and the left (injured) side and the right (non-injured) side of carotid arteries were removed by 5 mm on the heart side, at 5 mm from the internal and external carotid artery bifurcation area, and the DNA content was measured. The drug (Reference Example B3) was suspended in 0.5% methyl cellulose, and intraperitoneally administered once a day from the day of balloon injury operation to carotid artery enucleation. When balloon injury operation was performed, the drug was administered 30 min before anesthesia and the balloon injury was produced.

As shown in Fig. 6, the test substance suppressed vascular thickening due to balloon injury in a dose-dependent manner.

### Experimental Example 7: Effect on dextran sulfate induced colitis model

A 5% dextran sulfate (DSS) solution was placed in a water feeder to allow free drinking by female CBA/J mice (8 w) for 5 days, the solution was changed to 1% DSS solution on day 6 to allow free drinking for 7 days. The drug (Reference Example B3) (10 - 30 mg/kg) was suspended in 0.5% methyl cellulose (MC), and orally administered from day 6 under 1% DSS solution drinking once a day for 7 days. After 12 days of drinking DSS, the body weight was measured and stool was observed (grossly bloody stool: GBS). Blood was drawn and white blood count (WBC), red blood count (RBC), hemoglobin concentration (Hb) and hematocrit value (Ht) were measured by using an automatic cell counter (Sysmex). The results are shown in Table 17.

**[Table 17]**

| | WBC (x10²/µL) | RBC (x10⁴/µL) | Hb (g/dL) |
|---|---|---|---|
| Normal | 39.6±4.2 | 941.4±55.4 | 16.2±0.8 |
| Vehicle | 95.7±13.0** | 475.8±46.5** | 10.9±1.0** |
| Reference Example B3 | 41.0±4.1⁺ | 607.5±45.3 | 13.2±0.8 |
| 10 mg/kg | | | |
| Reference Example B3 30 mg/kg | 53.5±7.8⁺ | 616.7±51.1 | 12.9±0.5 |

| | Ht (%) | BW (%) | GBS (%) |
|---|---|---|---|
| Normal | 53.6±3.4 | 108.0±2.4 | 0 |
| Vehicle | 29.2±2.4** | 93.3±5.4* | 100** |
| Reference Example B3 | 35.4±2.7 | 102.0±7.1 | 83 |
| 10 mg/kg | | | |
| Reference Example B3 | 35.1±2.7 | 102.8±7.9 | 67 |
| 30 mg/kg | | | |

| | | | |
|---|---|---|---|
| Data are expressed as means±SE of six mice. *, p≤0.05; **, p≤0.01 (Student's test) vs. normal. ⁺, p≤0.05 (Student's test) vs. vehicle. | | | |

Since the test substance remarkably suppressed changes in WBC, RBC, Hb and Ht induced by DSS, it was found to be useful for the treatment of ulcerative colitis and the like.

### Experimental Example 8: Effect on collagen induced arthritis model

Bovine-derived type II collagen was dissolved in 0.05% acetic acid solution, and an equivalent amount of Freund's complete adjuvant was mixed therewith to give an emulsion. The collagen emulsion was administered intracutaneously into the tail head of male DBA/1 mice (6W) (n=10-12) at 100 µg/0.1 mL to induce arthritis. The levels of flare and edema of four limbs were visually observed during the progress thereof. The drug (Reference Example B3) (30 mg/kg) was suspended in 0.5% MC, and orally administered once a day from collagen immunization for 8 weeks (except Sundays). The onset of arthritis was evaluated every week, and the rate of onset was expressed by (number of limbs having arthritis/number of limbs of mice in one group × 100). As indices of arthritis, for each limb, no change was 0, swelling of one finger or plural fingers was 1, flare and swelling seen in the entirety was 2, strong swelling seen in the entirety was 3, crampus tonicus of joint was 4, and the total of four limbs was 16, and arthritis was expressed in (total of all scores of onset mice/number of limbs of mice in one group), wherein 4 was the highest value for one limb. The results are shown in Table 18.

**[Table 18]**

| | N | Arthritis incidence (%) | | | Arthritis index | | |
|---|---|---|---|---|---|---|---|
| | | 6W | 7W | 8W | 6W | 7W | 8W |
| Vehicle | 12 | 58.3 | 65.0 | 70.0 | 1.20 | 1.23 | 1.40 |
| Reference Example B3 | 10 | 37.5 | 40.0 | 60.0 | 0.65 | 0.80 | 1.15 |

Since the test substance decreased the incidence and index of arthritis, it was found to be useful for the treatment of arthritis rheumatoides and the like.

### Industrial Applicability

The pharmaceutical agent of the present invention, which comprises a cycloalkene compound, is useful as a prophylaxis· therapeutic agent for sepsis, particularly severe sepsis. In addition, the TLR signal inhibitor of the present invention, which comprises a non-peptide compound, is useful as an agent for the prophylaxis or treatment of various organ dysfunctions, severe sepsis, Alzheimer's disease, arteriosclerosis, ulcerative colitis, arthritis rheumatoides and the like.

## Claims

1. An agent for the prophylaxis or treatment of severe sepsis, which comprises a cycloalkene compound as an active ingredient.

2. An agent for the prophylaxis or treatment of severe sepsis, which comprises a compound represented by the formula (I): 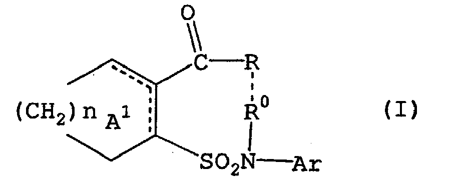 wherein
R represents an aliphatic hydrocarbon group optionally
having substituents, an aromatic hydrocarbon group optionally having substituents, a heterocyclic group optionally having substituents, a group represented by the formula: -OR¹ wherein R¹ represents a hydrogen atom or an aliphatic hydrocarbon group optionally having substituents, or a group represented by the formula: 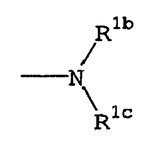 wherein
R^{1b} and R^{1c}
are the same or different and each represents a hydrogen atom or an aliphatic hydrocarbon group optionally having substituents,
R⁰ represents a hydrogen atom or an aliphatic hydrocarbon
group, or R and R⁰ in combination form a bond,
ring A¹ represents a cycloalkene optionally substituted by 1
to 4 substituents selected from the group consisting of
(1) an aliphatic hydrocarbon group optionally having substituents,
(2) an aromatic hydrocarbon group optionally having substituents,
(3) a group represented by the formula: -OR¹¹ wherein R¹¹ represents a hydrogen atom or an aliphatic hydrocarbon group optionally having substituents and
(4) a halogen atom,
Ar represents an aromatic hydrocarbon group optionally
having substituents,
a group represented by the formula: 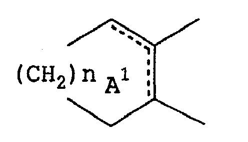 represents a group represented by the formula: 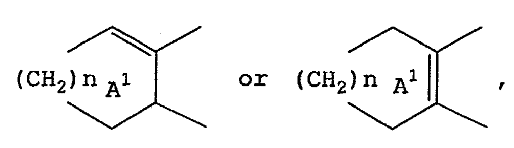 and
n represents an integer of 1 to 4,
or a salt thereof or a prodrug thereof, or a compound represented by the formula (II): 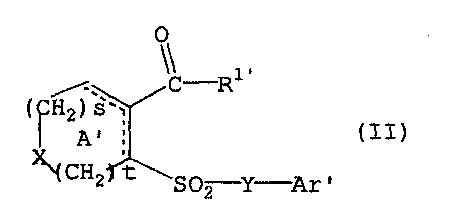 wherein R^{1'} represents an aliphatic hydrocarbon group optionally having substituents, an aromatic hydrocarbon group optionally having substituents, a heterocyclic group optionally having substituents, a group represented by the formula: -OR^{1a'} wherein R^{1a'} represents a hydrogen atom or an aliphatic hydrocarbon group optionally having substituents, or a group represented by the formula: 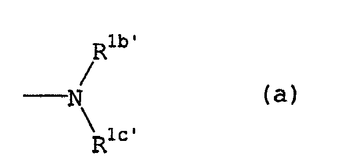 wherein R^{1b'} and R^{1c'} are the same or different and each represents a hydrogen atom or an aliphatic hydrocarbon group optionally having substituents,
X represents a methylene group, NH, a sulfur atom or an oxygen atom,
Y represents a methylene group optionally having substituents or NH optionally having substituents,
ring A' represents a 5- to 8-membered ring optionally having 1 to 4 substituents selected from the group consisting of (1) an aliphatic hydrocarbon group optionally having substituents, (2) an aromatic hydrocarbon group optionally having substituents, (3) a group represented by the formula: -OR^{2'} wherein R^{2'} represents a hydrogen atom or an aliphatic hydrocarbon group optionally having substituents and (4) a halogen atom,
Ar' represents an aromatic hydrocarbon group optionally having substituents,
a group represented by the formula: 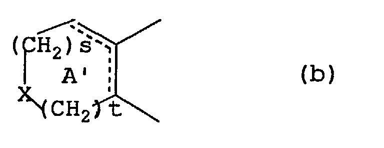 represents a group represented by the formula: 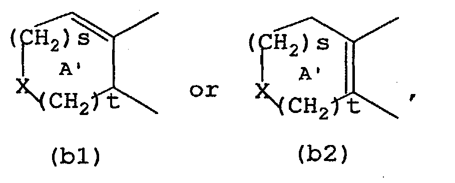 s represents an integer of 0 to 2,
t represents an integer of 1 to 3, and
the total of s and t is not more than 4;
provided that when X is a methylene group, Y represents a
methylene group optionally having substituents, or a salt thereof or a prodrug thereof.

3. The agent of claim 2, wherein the formula (I) is the formula (Ia) : 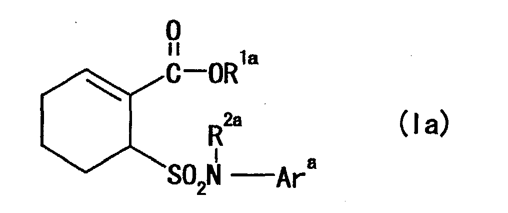 wherein R^{1a} represents a C₁₋₆ alkyl, R^{2a} represents a hydrogen atom or a C₁₋₆ alkyl and Ar^{a} represents a phenyl group substituted by 1 or 2 halogen atoms, and the formula (II) is the formula (IIa): 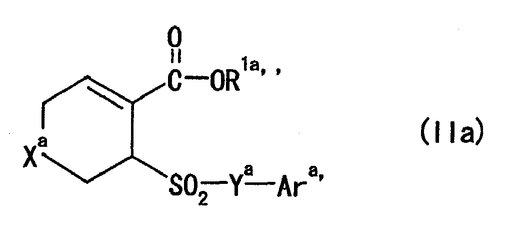 wherein R^{1a"} represents a C₁₋₆ alkyl, X^{a} represents a methylene group or an oxygen atom, Y^{a} represents a methylene group or - NH- and Ar^{a'} represents a phenyl group optionally having 1 or 2 substituents selected from a halogen atom and a C₁₋₆ alkoxy group.

4. The agent of claim 2, further comprising at least one kind of drug selected from the group consisting of antibacterial agent, antifungal agent, non-steroidal antiinflammatory drug, steroid and anticoagulant.

5. A method for the prophylaxis or treatment of severe sepsis, which comprises administration of an effective amount of a compound represented by the formula (I) or the formula (II) or a salt thereof or a prodrug thereof described in claim 2 to a mammal.

6. Use of a compound represented by the formula (I) or the formula (II) or a salt thereof or a prodrug thereof described in claim 2 for the production of an agent for the prophylaxis or treatment of severe sepsis.

7. A TLR signal inhibitor comprising a non-peptide compound as an active ingredient.

8. The agent of claim 7, wherein the non-peptide compound is a non-peptide compound having a molecular weight of not more than about 1000.

9. The agent of claim 8, wherein the non-peptide compound is a compound represented by the formula (I): 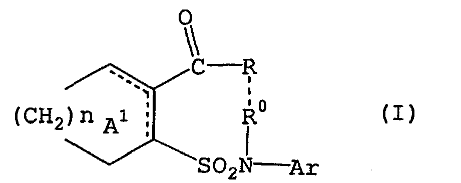 wherein R represents an aliphatic hydrocarbon group optionally having substituents, an aromatic hydrocarbon group optionally having substituents, a heterocyclic group optionally having substituents, a group represented by the formula: -OR¹ wherein R¹ represents a hydrogen atom or an aliphatic hydrocarbon group optionally having substituents, or a group represented by the formula: 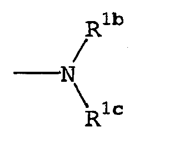 wherein R^{1b} and R^{1c} are the same or different and each represents a hydrogen atom or an aliphatic hydrocarbon group
optionally having substituents,
R⁰ represents a hydrogen atom or an aliphatic hydrocarbon group, or R and R⁰ in combination form a bond,
ring A¹ represents a cycloalkene optionally substituted by 1 to 4 substituents selected from the group consisting of (1) an aliphatic hydrocarbon group optionally having substituents, (2) an aromatic hydrocarbon group optionally having substituents, (3) a group represented by the formula: -OR¹¹ wherein R¹¹ represents a hydrogen atom or an aliphatic hydrocarbon group optionally having substituents and (4) a halogen atom,
Ar represents an aromatic hydrocarbon group optionally having substituents,
a group represented by the formula: 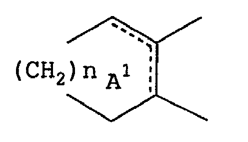 represents a group represented by the formula: 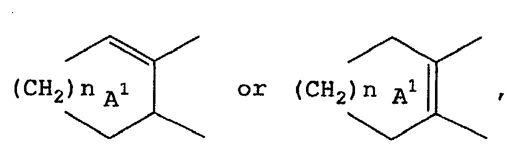 and
n represents an integer of 1 to 4, or a salt thereof or a prodrug thereof, or, a compound represented by the formula (II): 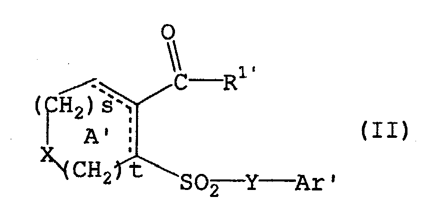 wherein R^{1'} represents an aliphatic hydrocarbon group optionally having substituents, an aromatic hydrocarbon group optionally having substituents, a heterocyclic group optionally having substituents, a group represented by the
formula: -OR^{1a'} wherein R^{1a'} represents a hydrogen atom or an aliphatic hydrocarbon group optionally having substituents, or a group represented by the formula: 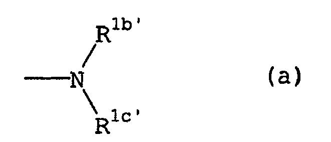 wherein R^{1b'} and R^{1c'} are the same or different and each represents a hydrogen atom or an aliphatic hydrocarbon group optionally having substituents,
X represents a methylene group, NH, sulfur atom or oxygen atom,
Y represents a methylene group optionally having substituents or NH optionally having substituents,
ring A' represents a 5 to 8-membered ring optionally having 1 to 4 substituents selected from the group consisting of (1) an aliphatic hydrocarbon group optionally having substituents, (2) an aromatic hydrocarbon group optionally having substituents, (3) a group represented by the formula: -OR^{2'} wherein R^{2'} represents a hydrogen atom or an aliphatic hydrocarbon group optionally having substituents and (4) a halogen atom,
Ar' represents an aromatic hydrocarbon group optionally having substituents,
a group represented by the formula: 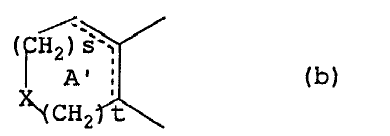 represents a group represented by the formula: 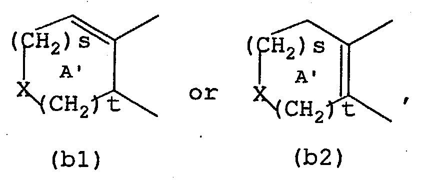 s represents an integer of 0 to 2,
t represents an integer of 1 to 3,
the total of s and t is not more than 4;
provided that when X is a methylene group, Y represents a methylene group optionally having substituents, or a salt thereof or a prodrug thereof.

10. The agent of claim 7, wherein TLR is TLR4.

11. An agent for the prophylaxis or treatment of a disease caused by a change in a TLR signal, which comprises the agent of claim 7.

12. The agent of claim 11, wherein the disease caused by the changes in the TLR signal is organ dysfunction.

13. The agent of claim 12, wherein the organ is an organ of central nervous system, circulatory system, respiratory system, bone and joint system, digestive system or renal and urinary system.

14. A method for the inhibition of TLR signal, which comprises administration of an effective amount of a non-peptide compound to a mammal.

15. A method for the prophylaxis or treatment of a disease caused by a change in a TLR signal, which comprises administration of an effective amount of a non-peptide compound to a mammal.

16. Use of a non-peptide compound for the production of a TLR signal inhibitor.

17. Use of a non-peptide compound for the production of an agent for the prophylaxis or treatment of a disease caused by a change in a TLR signal.

18. An agent for the prophylaxis or treatment of organ dysfunction, which comprises a TLR signal inhibitory substance.

19. The agent of claim 18, wherein the organ is an organ of central nervous system, circulatory system, respiratory system, bone and joint system, digestive system or renal and urinary system.

20. A method for the prophylaxis or treatment of severe sepsis or organ dysfunction, which comprises inhibition of TLR signal.
